# EUROPEAN PATENT APPLICATION

(11) **EP 4 813 985 A1**
(43) Date of publication of application: **30.09.2026**
(21) Application number: 24893560.3
(22) Date of filing: 22.11.2024
(51) Int. Cl.: C07D 471/04, C07D 487/00, C07D 487/04, A61K 31/513, A61K 31/517, A61K 31/519, A61P 35/00

(54) **PREPARATION OF QUINAZOLINONE DERIVATIVE AS KINASE INHIBITOR, AND USE THEREOF**

(30) Priority: 22.11.2023 CN 202311563261; 26.12.2023 CN 202311807306; 05.02.2024 CN 202410163788; 24.04.2024 CN 202410497576; 18.06.2024 CN 202410785177
(71) Applicant: Xizang Haisco Pharmaceutical Co., Ltd., Shannan, Xizang 856099 (CN)
(72) Inventor: LI, Yao, Shannan, Xizang 856099 (CN); SHI, Zongjun, Shannan, Xizang 856099 (CN); SHI, Shaohui, Shannan, Xizang 856099 (CN); WANG, Pengcheng, Shannan, Xizang 856099 (CN); SHU, Tianbo, Shannan, Xizang 856099 (CN); PEI, Yunpeng, Shannan, Xizang 856099 (CN); TANG, Pingming, Shannan, Xizang 856099 (CN); ZHANG, Chen, Shannan, Xizang 856099 (CN); YAN, Pangke, Shannan, Xizang 856099 (CN)
(74) Representative: AWA Benelux
(86) International application number: PCT/CN2024/133731
(87) International publication number: WO 2025/108405

(57) **Abstract**

Provided are a compound represented by formula I, a stereoisomer, a deuterated substance, or a pharmaceutically acceptable salt thereof, or a pharmaceutical composition containing same, as well as the use thereof as a BRAF regulator in the preparation of a drug for treating related diseases. Each group in formula (I) is as defined in the description.

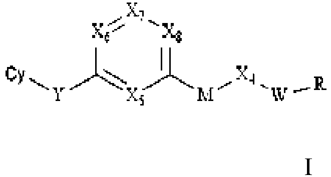

## Description

### Cross Reference to Related Applications

The present application claims priority to Chinese Patent Application No. 202311563261.X filed on November 22, 2023, Chinese Patent Application No. 202311807306.3 filed on December 26, 2023, Chinese Patent Application No. 202410163788.1 filed on February 05, 2024, Chinese Patent Application No. 202410497576.7 filed on April 24, 2024, and Chinese Patent Application No. 202410785177.0 filed on June 18, 2024, the contents of which are incorporated herein by reference.

### Technical Field

The present invention provides a quinazolinone derivative, a pharmaceutical composition comprising such a compound and a method of using such a compound or composition, e.g., for treating a proliferative disease, cancer or tumour, or in some embodiments, a disease or condition related to kinase (such as but not limited to B-Raf kinase) dysregulation.

### Background Art

Kinases are enzymes that catalyse the transfer of phosphate groups from high-energy, phosphate-donating molecules to specific substrates. This process is called phosphorylation, in which the substrate gains a phosphate group and the high-energy ATP molecule donates a phosphate group. Kinases are divided into the following broad categories based on the substrates on which they act: protein kinases, lipid kinases, and carbohydrate kinases. Kinases are found in a variety of species, from bacteria to moulds, to worms, and to mammals. More than five hundred different kinases have been identified in humans.

MAP kinases (MAPKs) are a family of serine/threonine kinases that respond to a variety of extracellular growth signals. For example, growth hormone, epidermal growth factor, platelet-derived growth factor, and insulin are all thought to participate in mitogenic stimulation of the MAPK pathway. Activation of this pathway at the receptor level initiates a signalling cascade whereby Ras GTPase exchanges GDP for GTP. Next, Ras activates Raf kinase (also known as MAPKKK), which activates MEK (MAPKK).

BRAF protein is a member of the RAF family of serine/threonine kinases that participates in cascades of the Ras-Raf-MEK-extracellular signal-regulated kinase (ERK) pathway or the mitogen-activated protein kinase (MAPK)/ERK signalling pathway that affect cell division and differentiation. Mutations in the BRAF gene can lead to uncontrolled growth and subsequent tumour formation. BRAF is mutated and/or overactivated in common human cancers, such as melanoma, colorectal cancer, thyroid cancer, non-small cell lung cancer, and ovarian cancer and their metastatic cancers, and primary brain tumours. Although some BRAF inhibitors produce excellent extracranial responses, cancers may still develop brain metastases during or subsequent to BRAF inhibitor therapy. An estimated 20% of subjects with cancer will develop brain metastases, with the majority of brain metastases occurring in those with melanoma, colorectal cancer, lung cancer, and renal cell carcinoma. Brain metastases remain a substantial contributor to overall cancer mortality in subjects with advanced cancers, and despite multimodality treatments and advances in systemic therapies, including combinations of surgery, radiotherapy, chemotherapy, immunotherapy, and/or targeted therapies, the prognosis remains poor.

Multimodality treatments and advances, including combinations of surgery, radiotherapy, chemotherapy, immunotherapy, and/or targeted therapies, the prognosis remains poor.

Additionally, BRAF has been identified as a potential target for the treatment of primary brain tumours. The prevalence of BRAF-V600E mutations in primary brain tumours has been reported. Schindler et al. analysed 1,320 central nervous system (CNS) tumours and Behling et al. analysed 969 CNS tumours in paediatric and adult populations. These studies, combined with others, have reported the presence of BRAF-V600E mutations in various cancers, including papillary craniopharyngioma, pleomorphic xanthoastrocytoma (PXA), ganglioglioma, astroblastoma, etc.

The blood-brain barrier (BBB) is a highly selective physical transport and metabolic barrier that separates the CNS from the blood. The BBB prevents certain drugs from entering brain tissue and is a limiting factor in the delivery of many peripherally-administered agents to the CNS. Many drugs commonly used to treat cancer cannot penetrate the blood-brain barrier. This means that these drugs cannot penetrate the brain and therefore cannot effectively kill cancer cells in the brain. Current treatments for subjects with brain tumours include surgical resection, radiotherapy, and/or chemotherapy with agents such as temozolomide and/or bevacizumab. However, surgical treatment of brain cancer is not always possible; for example, the tumour may not be accessible or the subject may not be able to withstand the trauma of neurosurgery. Additionally, radiotherapy and treatments with cytotoxic agents are known to have undesirable side effects. For example, there is growing evidence that the use of temozolomide itself can induce mutations and worsen prognosis in a significant proportion of subjects, and the bevacizumab label has a boxed warning for gastrointestinal perforation, surgical and wound healing complications, and bleeding. Kinase inhibitors are used to treat many peripheral cancers. However, many kinase inhibitors such as BRAF inhibitors (e.g., vemurafenib and dabrafenib), due to their structural properties, are substrates of active transporters such as P-glycoprotein (P-gp) or breast cancer resistance protein (BCRP). For example, dabrafenib was reported to have an MDR1 efflux ratio of 11.4, a BCRP efflux ratio of 21.0, and a total brain-to-plasma ratio of 0.023.

Given that both P-gp and BCRP are expressed in the endothelial cells lining blood-brain capillaries, the activity of both P-gp and BCRP in the BBB plays a critical role in preventing the distribution of most kinase inhibitors in the brain parenchyma. Therefore, kinase inhibitors are generally not suitable for the treatment of tumours or cancers in the brain (which is protected by the BBB). Therefore, treatments against tumours harbouring BRAF mutations are still needed. Additionally, there remains an unmet need for the treatment of CNS tumours, including CNS tumours harbouring BRAF mutations.

### Summary of the Invention

The present invention provides a small molecule compound with BRAF-regulating activity, or a stereoisomer, a deuterated substance or a pharmaceutically acceptable salt thereof. The compound can better penetrate the blood-brain barrier, has a higher brain-blood ratio, and has good activity, few side effects, excellent pharmacokinetics and high bioavailability.

The present invention provides a compound represented by formula I, formula II, formula III, or formula IV, or a stereoisomer, a deuterated substance or a pharmaceutically acceptable salt thereof, 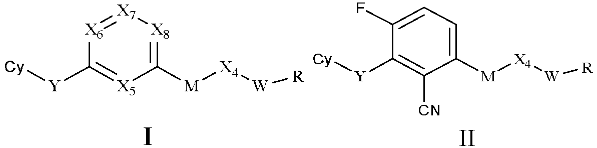 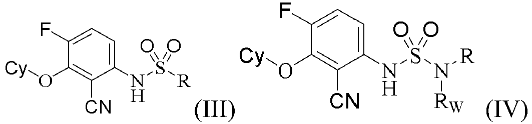
wherein Cy is selected from 8- to 15-membered fused heterocyclyl, which is optionally substituted with 1-5 groups selected from halogen, CN, OH, =O, NH₂,-SF₅, -COOH, C₁₋₄ alkyl, C₂₋₄ alkenyl, C₂₋₄ alkynyl, C₁₋₄ alkoxy, -NHC₁₋₄ alkyl,-N(C₁₋₄ alkyl)₂, halo C₁₋₄ alkoxy, halo C₁₋₄ alkyl, C₃₋₆ cycloalkyl, -C₁₋₄ alkyl-C₃₋₆ cycloalkyl, 4- to 12-membered heterocycloalkyl containing 1-3 heteroatoms selected from N, S, or O, 6- to 9-membered aryl, or 5- to 9-membered heteroaryl containing 1-3 heteroatoms selected from N, S, or O, wherein the alkyl, alkenyl, alkynyl, alkoxy, cycloalkyl, heterocyclyl, aryl and heteroaryl are optionally further substituted with 1-3 groups selected from halogen, =O, 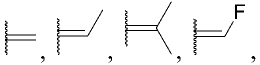 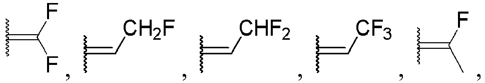 C₁₋₄ **alkyl,** C₁₋₄ alkoxy, halo C₁₋₄ alkoxy, or halo C₁₋₄ alkyl;
in some embodiments, Cy is selected from 8- to 15-membered fused heterocyclyl, which is optionally substituted with 1-5 groups selected from halogen, CN, OH, =O, NH₂, -SF₅, -COOH, C₁₋₄ alkyl, C₂₋₄ alkenyl, C₂₋₄ alkynyl, C₁₋₄ alkoxy, -NHC₁₋₄ alkyl, -N(C₁₋₄ alkyl)₂, halo C₁₋₄ alkoxy, halo C₁₋₄ alkyl, C₃₋₆ cycloalkyl, 4-to 12-membered heterocycloalkyl containing 1-3 heteroatoms selected from N, S, or O, 6- to 9-membered aryl, or 5- to 9-membered heteroaryl containing 1-3 heteroatoms selected from N, S, or O, wherein the alkyl, alkenyl, alkynyl, alkoxy, cycloalkyl, heterocyclyl, aryl and heteroaryl are optionally further substituted with 1-3 groups selected from halogen, =O, 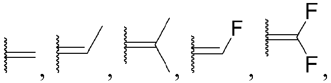 C₁₋₄ alkyl, C₁₋₄ alkoxy, halo C₁₋₄ alkoxy, or halo C₁₋₄ alkyl;
in some embodiments, Cy is selected from 8- to 12-membered bicyclic fused heterocyclyl or 10- to 15-membered tricyclic fused heterocyclyl, which are optionally substituted with 1-5 groups selected from halogen, CN, OH, =O, NH₂,-SF₅, C₁₋₄ alkyl, C₁₋₄ alkoxy, halo C₁₋₄ alkoxy, halo C₁₋₄ alkyl, C₃₋₆ cycloalkyl, 4- to 12-membered heterocycloalkyl containing 1-3 heteroatoms selected from N, S, or O, 6- to 9-membered aryl, or 5- to 9-membered heteroaryl containing 1-3 heteroatoms selected from N, S, or O, wherein the alkyl, alkenyl, alkynyl, alkoxy, cycloalkyl, heterocyclyl, aryl and heteroaryl are optionally further substituted with 1-3 groups selected from halogen, =O, 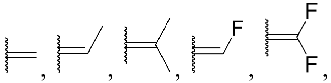 C₁₋₄ alkyl, C₁₋₄ alkoxy, halo C₁₋₄ alkoxy, or halo C₁₋₄ alkyl;
in some embodiments, Cy is selected from P1, P2, P3, or P4; 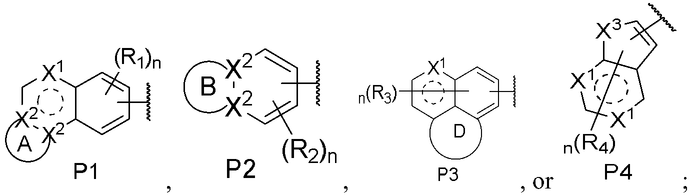
ring A is 5- to 6-membered heterocycloalkyl or 5- to 6-membered heteroaryl containing 1-3 heteroatoms selected from N, S, or O, wherein the heterocycloalkyl or heteroaryl is optionally substituted with 1-3 groups selected from halogen, C₁₋₄ alkoxy, -NHC₁₋₄ alkyl, -N(C₁₋₄ alkyl)₂, CN, C₁₋₄ alkyl, halo C₁₋₄ alkoxy, and halo C₁₋₄ alkyl;
in some embodiments, ring A is 5- to 6-membered heteroaryl containing 1-3 heteroatoms selected from N, S, or O, wherein the heteroaryl is optionally substituted with 1-3 groups selected from halogen, C₁₋₄ alkoxy, -NHC₁₋₄ alkyl, - N(C₁₋₄ alkyl)₂, CN, C₁₋₄ alkyl, halo C₁₋₄ alkoxy, and halo C₁₋₄ alkyl;
in some embodiments, ring A is 5-membered or 6-membered heterocycloalkyl containing 1-3 heteroatoms selected from N, S, or O, or 5-membered or 6-membered heteroaryl containing 1-3 heteroatoms selected from N, S, or O, wherein the heterocycloalkyl and heteroaryl are optionally substituted with 1-3 groups selected from halogen, C₁₋₄ alkoxy, -NHC₁₋₄ alkyl, -N(C₁₋₄ alkyl)₂, CN, C₁₋₄ alkyl, halo C₁₋₄ alkoxy, and halo C₁₋₄ alkyl; in some embodiments, ring A is 5-membered heterocycloalkyl or 5-membered heteroaryl, which are optionally substituted with 1-2 groups selected from halogen, C₁₋₄ alkoxy, -NHC₁₋₄ alkyl, -N(C₁₋₄ alkyl)₂, CN, C₁₋₄ alkyl, halo C₁₋₄ alkoxy, and halo C₁₋₄ alkyl; in some embodiments, the heterocycloalkyl is selected from pyrrolidyl, imidazolidinyl, tetrahydrofuryl, tetrahydrothienyl, tetrahydropyranyl, dihydroimidazolyl, dihydrofuryl, dihydropyrazolyl, etc.; in some embodiments, the heteroaryl is selected from pyrazolyl, oxazolyl, imidazolyl, triazole, thiazolyl, isoxazolyl, isothiazolyl, pyrrolyl, pyridyl, pyrimidyl, etc., and the heterocycloalkyl or heteroaryl is optionally substituted with 1-2 groups selected from halogen, C₁₋₄ alkoxy, -NHC₁₋₄ alkyl, - N(C₁₋₄ alkyl)₂, CN, C₁₋₄ alkyl, halo C₁₋₄ alkoxy, and halo C₁₋₄ alkyl;
in some embodiments, ring A is 5-membered or 6-membered heteroaryl containing 1-3 heteroatoms selected from N, S, or O, wherein the heteroaryl is optionally substituted with 1-3 groups selected from halogen, C₁₋₄ alkoxy, -NHC₁₋₄ alkyl, -N(C₁₋₄ alkyl)₂, CN, C₁₋₄ alkyl, halo C₁₋₄ alkoxy, and halo C₁₋₄ alkyl; in some embodiments, ring A is 5-membered heteroaryl, which is optionally substituted with 1-2 groups selected from halogen, C₁₋₄ alkoxy, -NHC₁₋₄ alkyl, -N(C₁₋₄ alkyl)₂, CN, C₁₋₄ alkyl, halo C₁₋₄ alkoxy, and halo C₁₋₄ alkyl; in some embodiments, the heteroaryl is selected from pyrazolyl, oxazolyl, imidazolyl, triazole, thiazolyl, isoxazolyl, isothiazolyl, pyrrolyl, pyridyl, pyrimidyl, etc., and is optionally substituted with 1-2 groups selected from halogen, C₁₋₄ alkoxy, -NHC₁₋₄ alkyl, -N(C₁₋₄ alkyl)₂, CN, C₁₋₄ alkyl, halo C₁₋₄ alkoxy, and halo C₁₋₄ alkyl;
ring B is 5- to 6-membered heteroaryl containing 1-3 heteroatoms selected from N, S, or O, wherein the heteroaryl is optionally substituted with 1-3 groups selected from =O, halogen, C₁₋₄ alkyl, halo C₁₋₄ alkyl, C₁₋₄ alkoxy, OH, halo C₁₋₄ alkoxy, 5-to 6-membered heterocyclyl containing 1-3 heteroatoms selected from N, S, or O, or -C₁₋₄ alkyl-C₃₋₆ cycloalkyl, wherein the heterocyclyl and cycloalkyl are optionally further substituted with groups 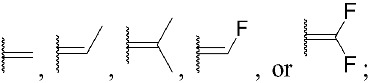 ring B is 6-membered heteroaryl containing 1-3 heteroatoms selected from N, S, or O, wherein the heteroaryl is optionally substituted with 1-2 groups selected from =O, halogen, C₁₋₄ alkyl, C₁₋₄ alkoxy, halo C₁₋₄ alkyl, C₃₋₆ cycloalkyl, -C₁₋₂ alkyl-C₃₋₆ cycloalkyl, or 5- to 6-membered heterocycloalkyl containing 1-3 heteroatoms selected from N, S, or O, wherein the heterocyclyl and cycloalkyl are optionally further substituted with groups 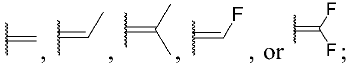 in some embodiments, ring B is 5-to 6-membered heteroaryl containing 1-3 heteroatoms selected from N, S, or O, wherein the heteroaryl is optionally substituted with 1-3 groups selected from =O, halogen, C₁₋₄ alkyl, halo C₁₋₄ alkyl, C₁₋₄ alkoxy, OH, halo C₁₋₄ alkoxy, C₃₋₆ cycloalkyl, and 5- to 6-membered heterocyclyl containing 1-3 heteroatoms selected from N, S, or O, wherein the heterocyclyl is optionally further substituted with groups 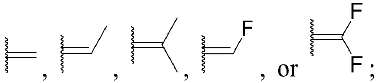 in some embodiments, ring B is 6-membered heteroaryl containing 1-3 heteroatoms selected from N, S, or O, wherein the heteroaryl is optionally substituted with 1-2 groups selected from =O, halogen, C₁₋₄ alkyl, halo C₁₋₄ alkyl, and 5- to 6-membered heterocycloalkyl containing 1-3 heteroatoms selected from N, S, or O, wherein the heterocyclyl is optionally further substituted with groups 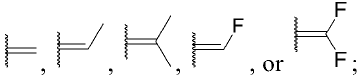 in some embodiments, ring B is 6-membered heteroaryl containing 1-3 heteroatoms selected from N, S, or O, wherein the heteroaryl is optionally substituted with 1-2 groups selected from =O, halogen, C₁₋₄ alkyl, halo C₁₋₄ alkyl, C₃₋₆ cycloalkyl, and 5- to 6-membered heterocycloalkyl containing 1-3 heteroatoms selected from N, S, or O, wherein the heterocyclyl is optionally further substituted with groups 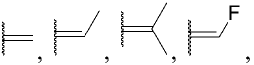 or 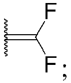
ring D is 5- to 6-membered heterocyclyl containing 1-3 heteroatoms selected from N, S, or O, wherein the heterocyclyl is optionally substituted with 1-3 groups selected from halogen, C₁₋₄ alkoxy, -NHC₁₋₄ alkyl, -N(C₁₋₄ alkyl)₂, CN, C₁₋₄ alkyl, halo C₁₋₄ alkoxy, and halo C₁₋₄ alkyl;
in some embodiments, ring D is 5- to 6-membered heterocyclyl containing 1-3 heteroatoms selected from N, S, or O, wherein the heterocyclyl is optionally substituted with 1-2 groups selected from halogen, CN, C₁₋₄ alkyl, and halo C₁₋₄ alkyl; in some embodiments, ring D is 5-membered or 6-membered heterocyclyl containing 1-3 heteroatoms selected from N, S, or O, wherein the heterocyclyl is optionally substituted with 1-2 groups selected from halogen, CN, C₁₋₄ alkyl, and halo C₁₋₄ alkyl;
each n is independently selected from 0, 1, 2, 3, 4, or 5; in some embodiments, each n is independently selected from 0, 1, 2, or 3; in some embodiments, each n is independently selected from 0, 1, or 2; in some embodiments, each n is independently selected from 1 or 2;
each X₁ is independently selected from N, NR₁₁, CR₁₁, or CR₁₁R₁₂; in some embodiments, each X₁ is independently selected from N or CR₁₁; in some embodiments, each X₁ is independently N; in some embodiments, each X₁ is independently CR₁₁;
each X₂ is independently selected from N, C, or CR₂₁; in some embodiments, each X₂ is independently selected from N, C, or CR₂₁; in some embodiments, each X₂ is independently selected from N or C; in some embodiments, each X₂ is independently N; in some embodiments, each X₂ is independently C;
X₃ is selected from NR₃₁, O, or CR₃₁R₃₂; in some embodiments, X₃ is selected from NR₃₁ or O; in some embodiments, X₃ is NR₃₁; in some embodiments, X₃ is O;
in some embodiments, Cy is selected from 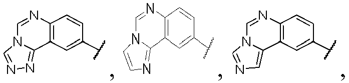 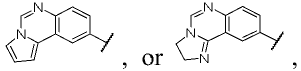 which are optionally substituted with 1-3 groups selected from F, Cl, Br, -CH₃, -CH₂CH₃, -CH₂F, -CHF₂, -CF₃, -CH₂CH₂F,-CH₂CHF₂, or -CH₂CF₃; or is selected from 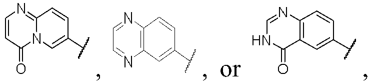 which are optionally substituted with 1-3 groups selected from F, Cl, Br, -CH₃,-CH₂CH₃, -CH₂F, -CHF₂, -CF₃, -CH₂CH₂F, -CH₂CHF₂, -CH₂CF₃, -OCH₃, cyclopropyl, 6-membered heterocycloalkyl containing 1-3 heteroatoms selected from N, S, or O, 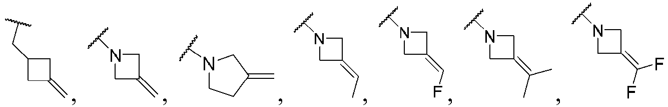 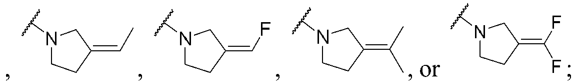 or is selected from 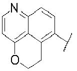 or 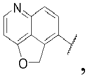 which are optionally substituted with 1-3 groups selected from F, Cl, Br, =O, -CH₃, -CH₂CH₃, -CH₂F, -CHF₂, -CF₃, -CH₂CH₂F, -CH₂CHF₂, or -CH₂CF₃; or is selected from 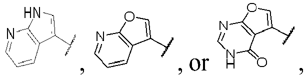 which are optionally substituted with 1-3 groups selected from F, Cl, Br, =O, -CH₃, -CH₂CH₃, -CH₂F, -CHF₂, -CF₃,-CH₂CH₂F, -CH₂CHF₂, -CH₂CF₃, or 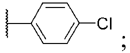 in some embodiments, Cy is selected from 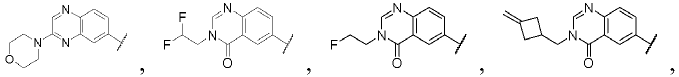 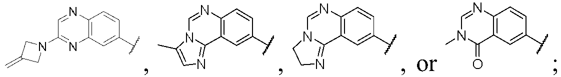 in some embodiments, Cy is selected from 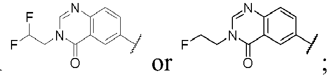
Y is C₁₋₂ alkyl, O, C=O, or NR_{y};
in some embodiments, Y is O, C=O, or NH;
in some embodiments, Y is O or C=O;
in some embodiments, Y is O or NH; in some embodiments, Y is O;
R_{y} is H or C₁₋₄ alkyl; in some embodiments, R_{y} is H; in some embodiments, R_{y} is methyl or ethyl;
M is C₁₋₂ alkyl, O, or NRₘ; in some embodiments, M is NRₘ;
W is a bond, O, or NR_{w}; in some embodiments, W is a bond; in some embodiments, W is NR_{w};
Rₘ and R_{w} are independently H or C₁₋₄ alkyl; in some embodiments, Rₘ and R_{w} are independently H; in some embodiments, Rₘ is H; in some embodiments, R_{w} is H or CH₃; in some embodiments, R_{w} is CH₃;
X₄ is selected from C(O), S(O), or S(O)₂; in some embodiments, X₄ is S(O)₂;
X₅ is selected from N or CRₓ₅; in some embodiments, X₅ is CRₓ₅;
X₆ is selected from N or CRₓ₆; in some embodiments, X₆ is CRₓ₆;
X₇ is selected from N or CRₓ₇; in some embodiments, X₇ is CRₓ₇;
X₈ is selected from N or CRₓ₈; in some embodiments, X₈ is CRₓ₈;
R₁, R₂, R₃, and R₄ are each independently selected from H, halogen, OH, =O,-NHC₁₋₄ alkyl, -N(C₁₋₄ alkyl)₂, CN, C₁₋₄ alkyl, C₁₋₄ alkoxy, halo C₁₋₄ alkoxy, halo C₁₋₄ alkyl, C₂₋₄ alkenyl, C₂₋₄ alkynyl, 5- to 8-membered heterocycloalkyl containing 1-3 heteroatoms selected from N, S, or O, or 6- to 9-membered aryl, wherein the alkyl, heterocycloalkyl, and aryl are optionally substituted with 1-3 groups selected from halogen, OH, NH₂, CN, and C₁₋₄ alkyl; in some embodiments, R₁, R₂, R₃, and R₄ are each independently selected from H, halogen, =O, CN, C₁₋₄ alkyl, C₁₋₄ alkoxy, halo C₁₋₄ alkoxy, halo C₁₋₄ alkyl, 5- to 8-membered heterocycloalkyl containing 1-3 heteroatoms selected from N, S, or O, or 6- to 9-membered aryl, wherein the alkyl, heterocycloalkyl, and aryl are optionally substituted with 1-3 groups selected from halogen, CN, and C₁₋₄ alkyl; in some embodiments, R₁, R₂, R₃, and R₄ are each independently selected from H, F, Cl, Br, =O, -CH₃, -CH₂CH₃, -CH₂F, -CHF₂, -CF₃, -CH₂CH₂F, -CH₂CHF₂, -CH₂CF₃, 6-membered heterocycloalkyl containing 1-3 heteroatoms selected from N, S, or O, or 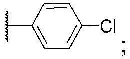
Rₓ₅, Rₓ₆, Rₓ₇, and Rₓ₈ are each independently selected from H, halogen, CN, C₁₋₄ alkyl, halo C₁₋₄ alkyl, C₂₋₄ alkenyl, C₂₋₄ alkynyl, C₁₋₄ alkoxy, halo C₁₋₄ alkoxy, - NHC₁₋₄ alkyl, -N(C₁₋₄ alkyl)₂, C₃₋₆ cycloalkyl, a 5- to 10-membered heterocyclic ring containing 1-3 heteroatoms selected from N, S, or O, and 6- to 9-membered aryl, wherein the alkyl, alkenyl, alkynyl, alkoxy, cycloalkyl, heterocyclic ring, or aryl is optionally substituted with 1-3 groups selected from halogen, C₁₋₄ alkyl, halo C₁₋₄ alkyl, C₁₋₄ alkoxy, halo C₁₋₄ alkoxy, OH, NH₂, and CN;
R₁₁, R₁₂, R₂₁, R₃₁, and R₃₂ are each independently selected from H, halogen, CN, C₁₋₄ alkyl, halo C₁₋₄ alkyl, C₂₋₄ alkenyl, C₂₋₄ alkynyl, C₁₋₄ alkoxy, halo C₁₋₄ alkoxy, -NHC₁₋₄ alkyl, -N(C₁₋₄ alkyl)₂, C₃₋₆ cycloalkyl, a 5- to 10-membered heterocyclic ring containing 1-3 heteroatoms selected from N, S, or O, and 6- to 9-membered aryl, wherein the alkyl, alkenyl, alkynyl, alkoxy, cycloalkyl, heterocyclic ring, or aryl is optionally substituted with 1-3 groups selected from halogen, C₁₋₄ alkyl, halo C₁₋₄ alkyl, C₁₋₄ alkoxy, halo C₁₋₄ alkoxy, OH, NH₂, and CN;
in some embodiments, R₁₁, R₂₁, R₃₁, Rₓ₅, Rₓ₆, Rₓ₇, and Rₓ₈ are each independently selected from H, halogen, CN, C₁₋₄ alkyl, halo C₁₋₄ alkyl, C₁₋₄ alkoxy, halo C₁₋₄ alkoxy, a 5- to 6-membered heterocyclic ring containing 1-3 heteroatoms selected from N, S, or O, and 6-membered aryl, wherein the alkyl, alkoxy, heterocyclic ring, or aryl is optionally substituted with 1-3 groups selected from halogen, C₁₋₄ alkyl, or halo C₁₋₄ alkyl;
in some embodiments, Rₓ₅, Rₓ₆, Rₓ₇, and Rₓ₈ are each independently selected from H, F, Cl, Br, CN, -CH₃, -CH₂CH₃, -CH₂F, -CHF₂, -CF₃, -CH₂CH₂F, - CH₂CHF₂, or -CH₂CF₃; in some embodiments, Rₓ₅, Rₓ₆, Rₓ₇, and Rₓ₈ are each independently selected from H, F, Cl, Br, CN, -CH₃, or -CH₂CH₃; in some embodiments, Rₓ₅, Rₓ₆, Rₓ₇, and Rₓ₈ are each independently selected from H, F, or CN; in some embodiments, Rₓ₅ is CN; in some embodiments, Rₓ₆ is F or Cl; in some embodiments, Rₓ₆ is F; in some embodiments, Rₓ₇ and Rₓ₈ are each independently H;
alternatively, R₁₁ and R₃₁, together with the atom(s) to which they are attached, form a C₃₋₆ carbocyclic ring, or a 5- to 6-membered heterocyclic ring containing 1-3 heteroatoms selected from N, S, or O, wherein the carbocyclic ring or heterocyclic ring is optionally substituted with 1-3 groups selected from halogen, C₁₋₄ alkyl, halo C₁₋₄ alkyl, C₁₋₄ alkoxy, OH, NH₂, and CN;
R is selected from C₃₋₁₀ cycloalkyl or 4- to 10-membered heterocyclyl containing 1-3 heteroatoms selected from N, S, or O, wherein the cycloalkyl and heterocyclyl are optionally substituted with 1-3 groups selected from halogen, C₁₋₄ alkyl, C₁₋₄ alkoxy, halo C₁₋₄ alkoxy, OH, NH₂, 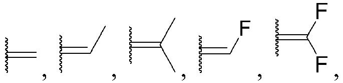 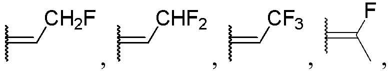 -NHC₁₋₄ alkyl, -N(C₁₋₄ alkyl)₂, and halo C₁₋₄ alkyl;
in some embodiments, R is selected from C₅₋₁₀ bicyclic cycloalkyl, C₃₋₆ monocyclic cycloalkyl, 5- to 10-membered bicyclic heterocycloalkyl containing 1-3 heteroatoms selected from N, S, or O, or 4- to 6-membered monocyclic heterocycloalkyl containing 1-2 heteroatoms selected from N, S, or O, wherein the bicyclic cycloalkyl and bicyclic heterocycloalkyl are optionally substituted with 1-3 groups selected from halogen, C₁₋₄ alkyl, C₁₋₄ alkoxy, halo C₁₋₄ alkoxy, OH, NH₂, - NHC₁₋₄ alkyl, -N(C₁₋₄ alkyl)₂, and halo C₁₋₄ alkyl, and the monocyclic cycloalkyl and monocyclic heterocycloalkyl are optionally further substituted with 1-3 groups selected from halogen, =O, 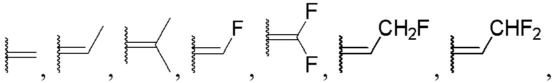 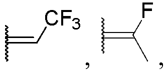 C₁₋₄ alkyl, C₁₋₄ alkoxy, halo C₁₋₄ alkoxy, or halo C₁₋₄ alkyl, provided that the monocyclic cycloalkyl and monocyclic heterocycloalkyl are substituted with at least one group selected from 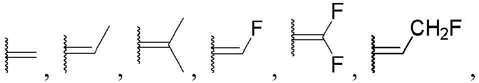 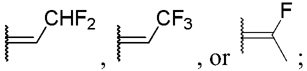
in some embodiments, R is selected from 6- to 10-membered spirocyclic heterocycloalkyl containing 1-3 heteroatoms selected from N, S, or O, or 4- to 5-membered monocyclic heterocycloalkyl containing 1-2 heteroatoms selected from N, S, or O, wherein the spirocyclic heterocycloalkyl is optionally substituted with 1-3 groups selected from halogen, C₁₋₄ alkyl, C₁₋₄ alkoxy, halo C₁₋₄ alkoxy, OH, NH₂, -NHC₁₋₄ alkyl, -N(C₁₋₄ alkyl)₂, and halo C₁₋₄ alkyl, and the monocyclic heterocycloalkyl is optionally further substituted with 1 group selected from F, Cl, Br, =O, 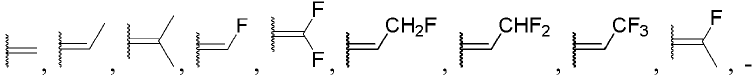 CH₃, -CH₂CH₃, -CH₂F, -CHF₂, -CF₃, -CH₂CH₂F, -CH₂CHF₂, or -CH₂CF₃, provided that the monocyclic heterocycloalkyl is substituted with at least one group selected from 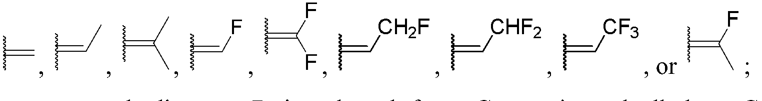
in some embodiments, R is selected from C₆₋₁₀ spirocycloalkyl or C₄₋₅ monocyclic cycloalkyl, wherein the spirocycloalkyl is optionally substituted with 1-3 groups selected from halogen, C₁₋₄ alkyl, C₁₋₄ alkoxy, halo C₁₋₄ alkoxy, OH, NH₂, -NHC₁₋₄ alkyl, -N(C₁₋₄ alkyl)₂, and halo C₁₋₄ alkyl, and the monocyclic cycloalkyl is optionally further substituted with 1-3 groups selected from F, Cl, Br, =O, 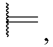 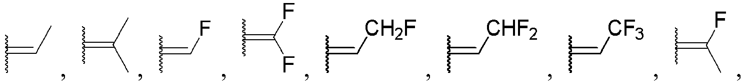 -CH₃,-CH₂CH₃, -CH₂F, -CHF₂, -CF₃, -CH₂CH₂F, -CH₂CHF₂, or -CH₂CF₃, provided that the monocyclic cycloalkyl is substituted with at least one group selected from , 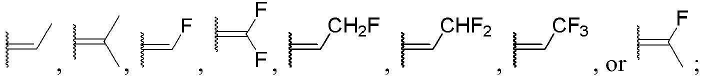
in some embodiments, R is selected from 6- to 10-membered spirocyclic heterocycloalkyl containing 1-3 heteroatoms selected from N, S, or O, 4- to 5-membered monocyclic heterocycloalkyl containing 1-2 heteroatoms selected from N, S, or O, C₆₋₁₀ spirocycloalkyl, or C₄₋₅ monocyclic cycloalkyl, wherein the spirocyclic heterocycloalkyl and spirocycloalkyl are optionally substituted with 1-3 groups selected from halogen, C₁₋₄ alkyl, C₁₋₄ alkoxy, halo C₁₋₄ alkoxy, OH, NH₂,-NHC₁₋₄ alkyl, -N(C₁₋₄ alkyl)₂, and halo C₁₋₄ alkyl, and the monocyclic heterocycloalkyl and monocyclic cycloalkyl are optionally further substituted with 1-3 groups selected from F, Cl, Br, =O, 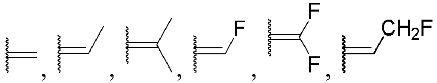 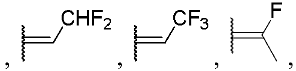 -CH₃, -CH₂CH₃, -CH₂F, -CHF₂, -CF₃, -CH₂CH₂F,-CH₂CHF₂, or -CH₂CF₃, provided that the monocyclic heterocycloalkyl and monocyclic cycloalkyl are substituted with at least one group selected from 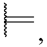 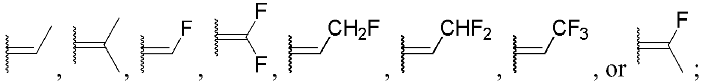
in some embodiments, R is selected from 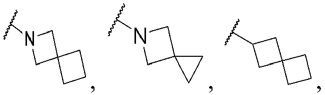 or 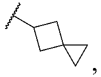 which are optionally substituted with 1-3 groups selected from F, Cl, Br, -CH₃, -CH₂CH₃, -CH₂F, -CHF₂, -CF₃, -CH₂CH₂F, -CH₂CHF₂, or -CH₂CF₃;
in some embodiments, R is selected from 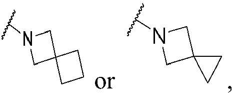 which are optionally substituted with 1-3 groups selected from F, Cl, Br, -CH₃, -CH₂CH₃, - CH₂F, -CHF₂, -CF₃, -CH₂CH₂F, -CH₂CHF₂, or -CH₂CF₃;
in some embodiments, R is selected from 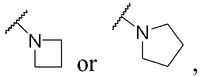 which are optionally substituted with 1-2 groups selected from F, Cl, Br, 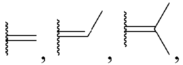 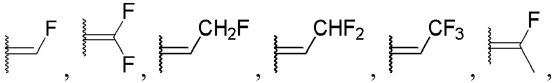 -CH₃, -CH₂CH₃, -CH₂F, - CHF₂, -CF₃, -CH₂CH₂F, -CH₂CHF₂, or -CH₂CF₃, and is substituted with at least one group selected from 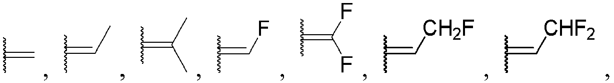 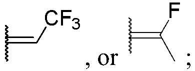
in some embodiments, R is selected from 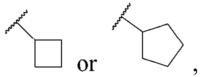 which are optionally substituted with 1-2 groups selected from F, Cl, Br, 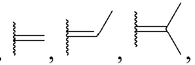 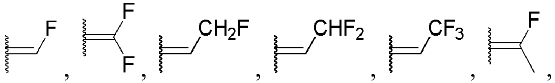 -CH₃, -CH₂CH₃, -CH₂F, - CHF₂, -CF₃, -CH₂CH₂F, -CH₂CHF₂, or -CH₂CF₃, and is substituted with at least one group selected from 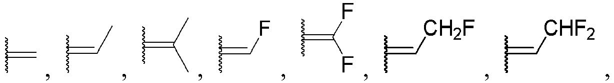 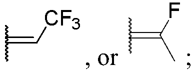
in some embodiments, R is selected from C₃₋₁₀ cycloalkyl or 4- to 10-membered heterocyclyl containing 1-3 heteroatoms selected from N, S, or O, wherein the cycloalkyl and heterocyclyl are optionally substituted with 1-3 groups selected from **halogen,** C₁₋₄ alkyl, C₁₋₄ alkoxy, halo C₁₋₄ alkoxy, OH, NH₂, 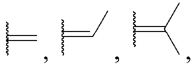 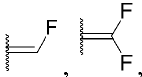 -NHC₁₋₄ alkyl, -N(C₁₋₄ alkyl)₂, and halo C₁₋₄ alkyl;
in some embodiments, R is selected from 4- to 10-membered heterocyclyl containing 1-3 heteroatoms selected from N, S, or O, wherein the heterocyclyl is optionally substituted with 1-3 groups selected from halogen, C₁₋₄ alkyl, C₁₋₄ alkoxy, halo C₁₋₄ alkoxy, OH, NH₂, 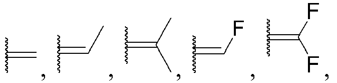 -NHC₁₋₄ alkyl, -N(C₁₋₄ alkyl)₂, and halo C₁₋₄ alkyl;
in some embodiments, R is selected from C₅₋₁₀ bicyclic cycloalkyl, C₃₋₆ monocyclic cycloalkyl, 5- to 10-membered bicyclic heterocycloalkyl containing 1-3 heteroatoms selected from N, S, or O, or 4- to 6-membered monocyclic heterocycloalkyl containing 1-2 heteroatoms selected from N, S, or O, wherein the bicyclic cycloalkyl and bicyclic heterocycloalkyl are optionally substituted with 1-3 groups selected from halogen, C₁₋₄ alkyl, C₁₋₄ alkoxy, halo C₁₋₄ alkoxy, OH, NH₂, - NHC₁₋₄ alkyl, -N(C₁₋₄ alkyl)₂, and halo C₁₋₄ alkyl, and the monocyclic cycloalkyl and monocyclic heterocycloalkyl are further substituted with groups 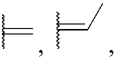 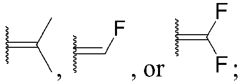
in some embodiments, R is selected from 5- to 10-membered bicyclic heterocycloalkyl containing 1-3 heteroatoms selected from N, S, or O, or 4- to 6-membered monocyclic heterocycloalkyl containing 1-2 heteroatoms selected from N, S, or O, wherein the bicyclic heterocycloalkyl is optionally substituted with 1-3 groups selected from halogen, C₁₋₄ alkyl, C₁₋₄ alkoxy, halo C₁₋₄ alkoxy, OH, NH₂,-NHC₁₋₄ alkyl, -N(C₁₋₄ alkyl)₂, and halo C₁₋₄ alkyl, and the monocyclic heterocycloalkyl is further substituted with groups 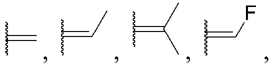 or 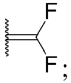
in some embodiments, R is selected from 6- to 10-membered spirocyclic heterocycloalkyl containing 1-3 heteroatoms selected from N, S, or O, or 4- to 6-membered monocyclic heterocycloalkyl containing 1-2 heteroatoms selected from N, S, or O, wherein the spirocyclic heterocycloalkyl is optionally substituted with 1-3 groups selected from halogen, C₁₋₄ alkyl, C₁₋₄ alkoxy, halo C₁₋₄ alkoxy, OH, NH₂, -NHC₁₋₄ alkyl, -N(C₁₋₄ alkyl)₂, and halo C₁₋₄ alkyl, and the monocyclic heterocycloalkyl is further substituted with groups 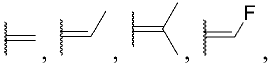 or 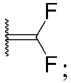
in some embodiments, R is selected from 6- to 10-membered spirocyclic heterocycloalkyl containing 1-3 heteroatoms selected from N, S, or O, or 4- to 5-membered monocyclic heterocycloalkyl containing 1-2 heteroatoms selected from N, S, or O, wherein the spirocyclic heterocycloalkyl is optionally substituted with 1-3 groups selected from halogen, C₁₋₄ alkyl, C₁₋₄ alkoxy, halo C₁₋₄ alkoxy, OH, NH₂, -NHC₁₋₄ alkyl, -N(C₁₋₄ alkyl)₂, and halo C₁₋₄ alkyl, and the monocyclic heterocycloalkyl is further substituted with groups 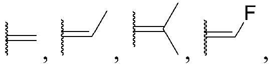 or 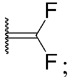
in some embodiments, R is selected from C₆₋₁₀ spirocycloalkyl or C₄₋₆ monocyclic cycloalkyl, wherein the spirocycloalkyl is optionally substituted with 1-3 groups selected from halogen, C₁₋₄ alkyl, C₁₋₄ alkoxy, halo C₁₋₄ alkoxy, OH, NH₂, -NHC₁₋₄ alkyl, -N(C₁₋₄ alkyl)₂, and halo C₁₋₄ alkyl, and the monocyclic cycloalkyl is further substituted with groups 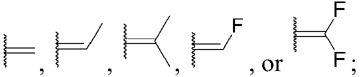
in some embodiments, R is selected from C₆₋₁₀ spirocycloalkyl or C₄₋₅ monocyclic cycloalkyl, wherein the spirocycloalkyl is optionally substituted with 1-3 groups selected from halogen, C₁₋₄ alkyl, C₁₋₄ alkoxy, halo C₁₋₄ alkoxy, OH, NH₂, -NHC₁₋₄ alkyl, -N(C₁₋₄ alkyl)₂, and halo C₁₋₄ alkyl, and the monocyclic cycloalkyl is further substituted with groups 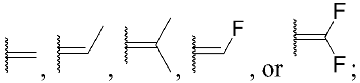
in some embodiments, R is selected from 6- to 8-membered spirocyclic heterocycloalkyl containing 1-3 heteroatoms selected from N, S, or O, or 4- to 6-membered monocyclic heterocycloalkyl containing 1-2 heteroatoms selected from N, S, or O, wherein the spirocyclic heterocycloalkyl is optionally substituted with 1-3 groups selected from halogen, C₁₋₄ alkyl, C₁₋₄ alkoxy, halo C₁₋₄ alkoxy, OH, NH₂, -NHC₁₋₄ alkyl, -N(C₁₋₄ alkyl)₂, and halo C₁₋₄ alkyl, and the monocyclic heterocycloalkyl is further substituted with groups 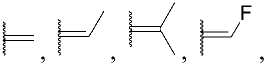 or 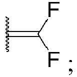 in some embodiments, R is selected from 6-membered or 7-membered spirocyclic heterocycloalkyl containing 1-3 heteroatoms selected from N, S, or O, or 4-, 5-, or 6-membered monocyclic heterocycloalkyl containing 1 heteroatom selected from N, S, or O, wherein the spirocyclic heterocycloalkyl is optionally substituted with 1-3 groups selected from F, Cl, Br, -CH₃, -CH₂CH₃, methoxy, ethoxy, -CH₂F, -CHF₂, -CF₃, -CH₂CH₂F, -CH₂CHF₂, -CH₂CF₃, -OCH₂F, -OCHF₂,-OCF₃, -OCH₂CH₂F, -OCH₂CHF₂, -OCH₂CF₃, OH, NH₂, -NHCH₃, or -N(CH₃)₂, and the monocyclic heterocycloalkyl is further substituted with groups 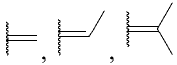 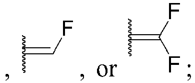 in some embodiments, R is selected from 6-membered or 7-membered spirocyclic heterocycloalkyl containing one heteroatom N, or 4-, 5-, 6-membered monocyclic heterocycloalkyl containing one heteroatom N, wherein the spirocyclic heterocycloalkyl is optionally substituted with 1-3 groups selected from F, Cl, Br, -CH₃, or -CH₂CH₃, and the monocyclic heterocycloalkyl is further substituted with groups 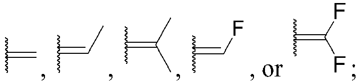
in some embodiments, R is selected from C₆₋₈ spirocycloalkyl or C₄₋₆ monocyclic cycloalkyl, wherein the spirocycloalkyl is optionally substituted with 1-3 groups selected from halogen, C₁₋₄ alkyl, C₁₋₄ alkoxy, halo C₁₋₄ alkoxy, OH, NH₂, -NHC₁₋₄ alkyl, -N(C₁₋₄ alkyl)₂, and halo C₁₋₄ alkyl, and the monocyclic cycloalkyl is further substituted with groups 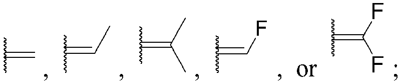 in some embodiments, R is selected from C₆ spirocycloalkyl or C₇ spirocycloalkyl, C₄ monocyclic cycloalkyl, C₅ monocyclic cycloalkyl, or C₆ monocyclic cycloalkyl, wherein the spirocycloalkyl is optionally substituted with 1-3 groups selected from F, Cl, Br, -CH₃, -CH₂CH₃, methoxy, ethoxy, -CH₂F, -CHF₂, -CF₃, -CH₂CH₂F,-CH₂CHF₂, -CH₂CF₃, -OCH₂F, -OCHF₂, -OCF₃, -OCH₂CH₂F, -OCH₂CHF₂,-OCH₂CF₃, OH, NH₂, -NHCH₃, or -N(CH₃)₂, and the monocyclic cycloalkyl is further substituted with groups 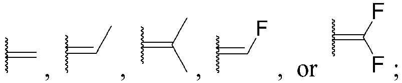 in some embodiments, R is selected from C₆ or C₇ spirocycloalkyl, C₄ monocyclic cycloalkyl, C₅ monocyclic cycloalkyl, or C₆ monocyclic cycloalkyl, wherein the spirocycloalkyl is optionally substituted with 1-3 groups selected from F, Cl, Br, - CH₃, or -CH₂CH₃, and the monocyclic cycloalkyl is further substituted with groups 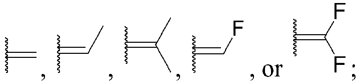
in some embodiments, R is selected from 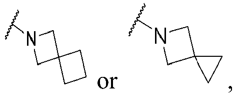 which are optionally substituted with 1-3 groups selected from F, Cl, Br, -CH₃, -CH₂CH₃,-CH₂F, -CHF₂, -CF₃, -CH₂CH₂F, -CH₂CHF₂, or -CH₂CF₃, or is selected from 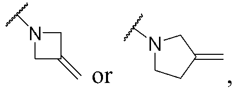 which are substituted with 1-2 groups selected from F, Cl, Br, -CH₃, -CH₂CH₃, -CH₂F, -CHF₂, -CF₃, -CH₂CH₂F, -CH₂CHF₂, or -CH₂CF₃;
in some embodiments, R is selected from 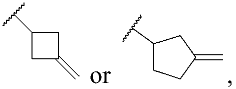 which are optionally substituted with 1-2 groups selected from F, Cl, Br, -CH₃, -CH₂CH₃, - CH₂F, -CHF₂, -CF₃, -CH₂CH₂F, -CH₂CHF₂, or -CH₂CF₃;
in some embodiments, R is selected from 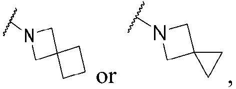 which are optionally substituted with 1-3 groups selected from F, Cl, Br, -CH₃, -CH₂CH₃, - CH₂F, -CHF₂, -CF₃, -CH₂CH₂F, -CH₂CHF₂, or -CH₂CF₃, or is selected from 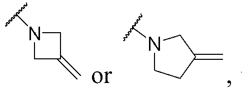 which are substituted with 1-2 groups selected from F, Cl, Br, -CH₃, -CH₂CH₃, -CH₂F, -CHF₂, -CF₃, -CH₂CH₂F, -CH₂CHF₂, or -CH₂CF₃;
in some embodiments, R is selected from cyclobutyl, 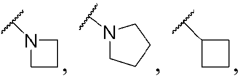 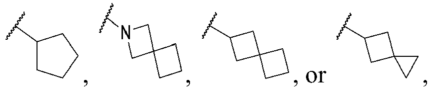 wherein the cyclobutyl, 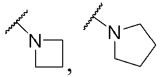 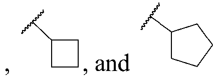 are optionally substituted with 1-2 groups selected from F, Cl, Br, 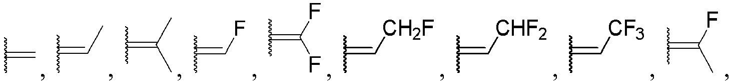 -CH₃, -CH₂CH₃, -CH₂F, -CHF₂, -CF₃, -CH₂CH₂F, -CH₂CHF₂, or -CH₂CF₃, and the cyclobutyl, 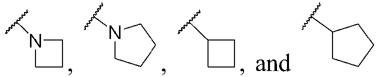 are substituted with at least one group selected from 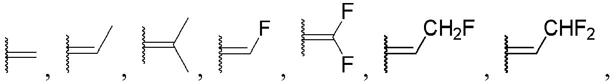 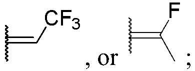
in some embodiments, R is selected from 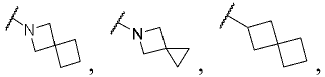 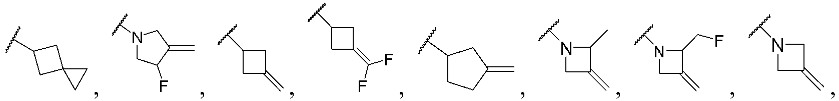 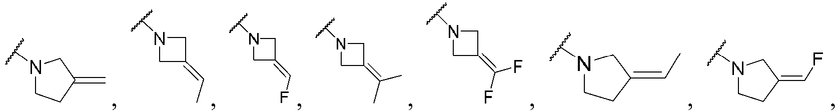 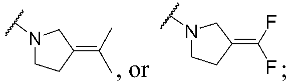
in some embodiments, R is selected from 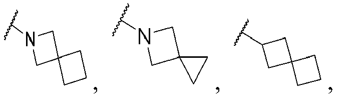 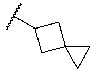 , or 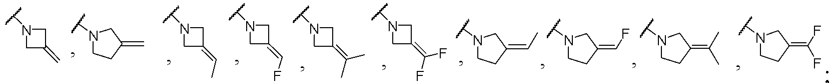
in some embodiments, R is selected from 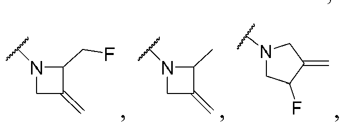 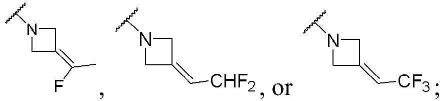
in some embodiments, P1 is selected from 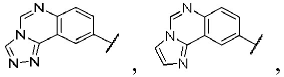 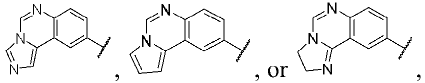 which are optionally substituted with 1-3 groups selected from F, Cl, Br, -CH₃, -CH₂CH₃, -CH₂F, -CHF₂, -CF₃, -CH₂CH₂F,-CH₂CHF₂, or -CH₂CF₃;
in some embodiments, P1 is selected from 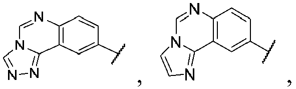 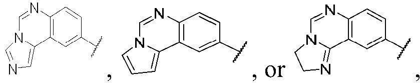 which are optionally substituted with 1-3 groups selected from F, Cl, Br, -CH₃, -CH₂CH₃, -CH₂F, -CHF₂, -CF₃, -CH₂CH₂F,-CH₂CHF₂, or -CH₂CF₃;
in some embodiments, P2 is selected from 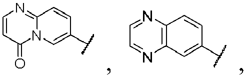 or 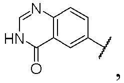 which are optionally substituted with 1-3 groups selected from F, Cl, Br, -CH₃, -CH₂CH₃, -CH₂F, -CHF₂, -CF₃, -CH₂CH₂F, -CH₂CHF₂, -CH₂CF₃, -OCH₃, cyclopropyl, 6-membered heterocycloalkyl containing 1-3 heteroatoms selected from N, S, or O, 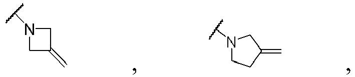 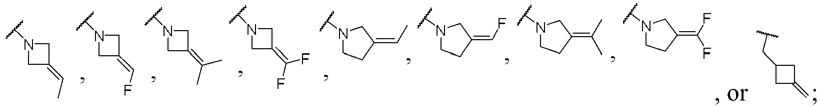
in some embodiments, P2 is selected from 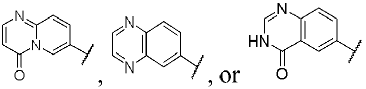 , which are optionally substituted with 1-3 groups selected from F, Cl, Br, =O, -CH₃, -CH₂CH₃, -CH₂F, -CHF₂, -CF₃, -CH₂CH₂F, -CH₂CHF₂, -CH₂CF₃, 6-membered heterocycloalkyl containing 1-3 heteroatoms selected from N, S, or O, 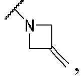 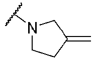 , or 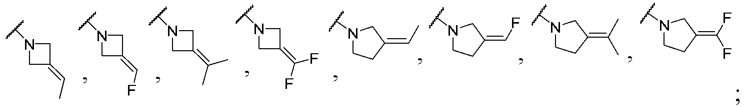
in some embodiments, P2 is selected from 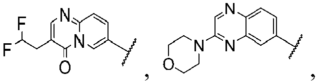 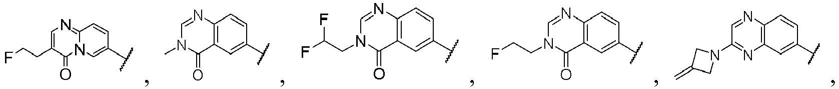 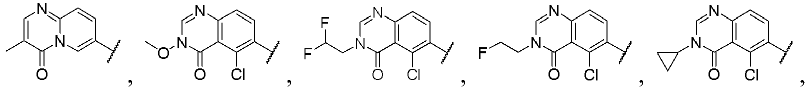 or 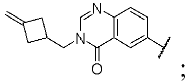 in some embodiments, P2 is selected from 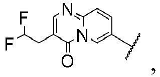 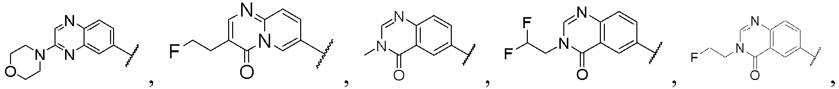 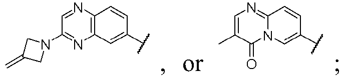 in some embodiments, P2 is selected from 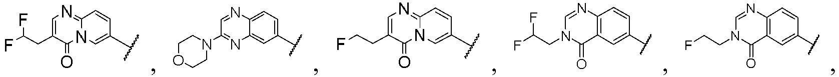 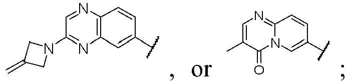 in some embodiments, P2 is selected from 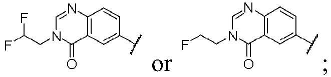
in some embodiments, P3 is selected from 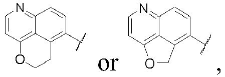 which are optionally substituted with 1-3 groups selected from F, Cl, Br, =O, -CH₃, -CH₂CH₃, -CH₂F, -CHF₂, -CF₃, -CH₂CH₂F, -CH₂CHF₂, or -CH₂CF₃;
in some embodiments, P4 is selected from 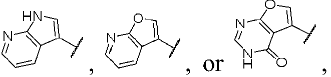 which are optionally substituted with 1-3 groups selected from F, Cl, Br, =O, -CH₃, -CH₂CH₃, -CH₂F, -CHF₂, -CF₃, -CH₂CH₂F, -CH₂CHF₂, -CH₂CF₃, or

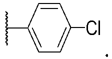

Specifically, as a first technical solution of the present invention, the present invention provides a compound represented by formula I, or a stereoisomer, a deuterated substance or a pharmaceutically acceptable salt thereof, 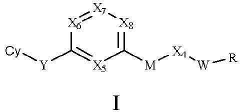
wherein Cy is selected from 8- to 15-membered fused heterocyclyl, which is optionally substituted with 1-5 groups selected from halogen, CN, OH, =O, NH₂,-SF₅, -COOH, C₁₋₄ alkyl, C₂₋₄ alkenyl, C₂₋₄ alkynyl, C₁₋₄ alkoxy, -NHC₁₋₄ alkyl,-N(C₁₋₄ alkyl)₂, halo C₁₋₄ alkoxy, halo C₁₋₄ alkyl, C₃₋₆ cycloalkyl, -C₁₋₄ alkyl-C₃₋₆ cycloalkyl, 4- to 12-membered heterocycloalkyl containing 1-3 heteroatoms selected from N, S, or O, 6- to 9-membered aryl, or 5- to 9-membered heteroaryl containing 1-3 heteroatoms selected from N, S, or O, wherein the alkyl, alkenyl, alkynyl, alkoxy, cycloalkyl, heterocyclyl, aryl and heteroaryl are optionally further substituted with 1-3 groups selected from halogen, =O, 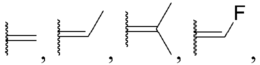 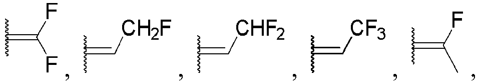 C₁₋₄ alkyl, C₁₋₄ alkoxy, halo C₁₋₄ alkoxy, or halo C₁₋₄ alkyl; or
Cy is selected from 8- to 15-membered fused heterocyclyl, which is optionally substituted with 1-5 groups selected from halogen, CN, OH, =O, NH₂, -SF₅, -COOH, C₁₋₄ alkyl, C₂₋₄ alkenyl, C₂₋₄ alkynyl, C₁₋₄ alkoxy, -NHC₁₋₄ alkyl, -N(C₁₋₄ alkyl)₂, halo C₁₋₄ alkoxy, halo C₁₋₄ alkyl, C₃₋₆ cycloalkyl, 4- to 12-membered heterocycloalkyl containing 1-3 heteroatoms selected from N, S, or O, 6- to 9-membered aryl, or 5-to 9-membered heteroaryl containing 1-3 heteroatoms selected from N, S, or O, wherein the alkyl, alkenyl, alkynyl, alkoxy, cycloalkyl, heterocyclyl, aryl and heteroaryl are optionally further substituted with 1-3 groups selected from halogen, =O, 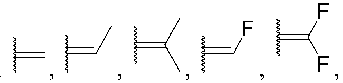 C₁₋₄ alkyl, C₁₋₄ alkoxy, halo C₁₋₄ alkoxy, or halo C₁₋₄ alkyl;
Y is C₁₋₂ alkyl, O, C=O, or NR_{y};
R_{y} is H or C₁₋₄ alkyl;
M is C₁₋₂ alkyl, O, or NRₘ;
W is a bond, O, or NR_{w};
Rₘ and R_{w} are independently H or C₁₋₄ alkyl;
X₄ is selected from C(O), S(O), or S(O)₂;
X₅ is selected from N or CRₓ₅;
X₆ is selected from N or CRₓ₆;
X₇ is selected from N or CRₓ₇;
X₈ is selected from N or CRₓ₈;
Rₓ₅, Rₓ₆, Rₓ₇, and Rₓ₈ are each independently selected from H, halogen, CN, C₁₋₄ alkyl, halo C₁₋₄ alkyl, C₂₋₄ alkenyl, C₂₋₄ alkynyl, C₁₋₄ alkoxy, halo C₁₋₄ alkoxy, - NHC₁₋₄ alkyl, -N(C₁₋₄ alkyl)₂, C₃₋₆ cycloalkyl, a 5- to 10-membered heterocyclic ring containing 1-3 heteroatoms selected from N, S, or O, and 6- to 9-membered aryl, wherein the alkyl, alkenyl, alkynyl, alkoxy, cycloalkyl, heterocyclic ring, or aryl is optionally substituted with 1-3 groups selected from halogen, C₁₋₄ alkyl, halo C₁₋₄ alkyl, C₁₋₄ alkoxy, halo C₁₋₄ alkoxy, OH, NH₂, and CN;
R is selected from C₃₋₁₀ cycloalkyl or 4- to 10-membered heterocyclyl containing 1-3 heteroatoms selected from N, S, or O, wherein the cycloalkyl and heterocyclyl are optionally substituted with 1-3 groups selected from halogen, C₁₋₄ alkyl, C₁₋₄ alkoxy, halo C₁₋₄ alkoxy, OH, NH₂, 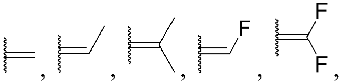 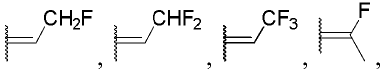 -NHC₁₋₄ alkyl, -N(C₁₋₄ alkyl)₂, and halo C₁₋₄ alkyl; or
R is selected from C₃₋₁₀ cycloalkyl or 4- to 10-membered heterocyclyl containing 1-3 heteroatoms selected from N, S, or O, wherein the cycloalkyl and heterocyclyl are optionally substituted with 1-3 groups selected from halogen, C₁₋₄ alkyl, C₁₋₄ alkoxy, halo C₁₋₄ alkoxy, OH, NH₂, 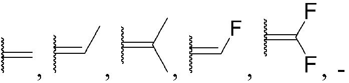 NHC₁₋₄ alkyl, -N(C₁₋₄ alkyl)₂, and halo C₁₋₄ alkyl; in some embodiments, R is selected from 4- to 10-membered heterocyclyl containing 1-3 heteroatoms selected from N, S, or O, wherein the heterocyclyl is optionally substituted with 1-3 groups selected from halogen, C₁₋₄ alkyl, C₁₋₄ alkoxy, halo C₁₋₄ alkoxy, OH, NH₂, 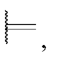 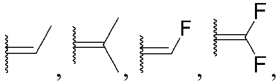 -NHC₁₋₄ alkyl, -N(C₁₋₄ alkyl)₂, and halo C₁₋₄ alkyl.

Specifically, as a second technical solution of the present invention, the present invention provides a compound represented by formula I, or a stereoisomer, a deuterated substance or a pharmaceutically acceptable salt thereof,
wherein Cy is selected from P1, P2, P3, or P4; 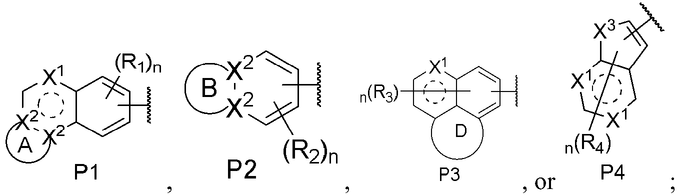
ring A is 5- to 6-membered heterocycloalkyl or 5- to 6-membered heteroaryl containing 1-3 heteroatoms selected from N, S, or O, wherein the heterocycloalkyl or heteroaryl is optionally substituted with 1-3 groups selected from halogen, C₁₋₄ alkoxy, -NHC₁₋₄ alkyl, -N(C₁₋₄ alkyl)₂, CN, C₁₋₄ alkyl, halo C₁₋₄ alkoxy, and halo C₁₋₄ alkyl; in some embodiments, ring A is 5- to 6-membered heteroaryl containing 1-3 heteroatoms selected from N, S, or O, wherein the heteroaryl is optionally substituted with 1-3 groups selected from halogen, C₁₋₄ alkoxy, -NHC₁₋₄ alkyl, - N(C₁₋₄ alkyl)₂, CN, C₁₋₄ alkyl, halo C₁₋₄ alkoxy, and halo C₁₋₄ alkyl;
ring B is 5- to 6-membered heteroaryl containing 1-3 heteroatoms selected from N, S, or O, wherein the heteroaryl is optionally substituted with 1-3 groups selected from =O, halogen, C₁₋₄ alkyl, halo C₁₋₄ alkyl, C₁₋₄ alkoxy, OH, halo C₁₋₄ alkoxy, C₃₋₆ cycloalkyl, 5- to 6-membered heterocyclyl containing 1-3 heteroatoms selected from N, S, or O, or -C₁₋₄ alkyl-C₃₋₆ cycloalkyl, wherein the heterocyclyl and cycloalkyl are optionally further substituted with groups 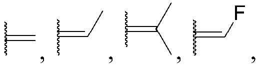 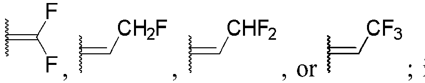 in some embodiments, ring B is 5- to 6-membered heteroaryl containing 1-3 heteroatoms selected from N, S, or O, wherein the heteroaryl is optionally substituted with 1-3 groups selected from =O, halogen, C₁₋₄ alkyl, halo C₁₋₄ alkyl, C₁₋₄ alkoxy, OH, halo C₁₋₄ alkoxy, and 5- to 6-membered heterocyclyl containing 1-3 heteroatoms selected from N, S, or O, wherein the heterocyclyl is optionally further substituted with groups 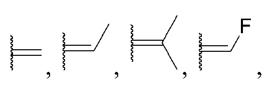 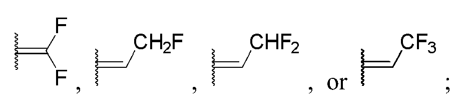 in some embodiments, ring B is 6-membered heteroaryl containing 1-3 heteroatoms selected from N, S, or O, wherein the heteroaryl is optionally substituted with 1-2 groups selected from =O, halogen, C₁₋₄ alkyl, C₁₋₄ alkoxy, halo C₁₋₄ alkyl, C₃₋₆ cycloalkyl, -C₁₋₂ alkyl-C₃₋₆ cycloalkyl, or 5- to 6-membered heterocycloalkyl containing 1-3 heteroatoms selected from N, S, or O, wherein the heterocyclyl and cycloalkyl are optionally further substituted with groups 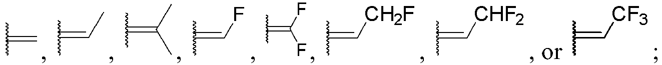
ring D is 5- to 6-membered heterocyclyl containing 1-3 heteroatoms selected from N, S, or O, wherein the heterocyclyl is optionally substituted with 1-3 groups selected from halogen, C₁₋₄ alkoxy, -NHC₁₋₄ alkyl, -N(C₁₋₄ alkyl)₂, CN, C₁₋₄ alkyl, halo C₁₋₄ alkoxy, and halo C₁₋₄ alkyl;
each n is independently selected from 0, 1, 2, 3, 4, or 5;
each X₁ is independently selected from N, NR₁₁, CR₁₁, or CR₁₁R₁₂;
each X₂ is independently selected from N, C, or CR₂₁;
X₃ is selected from NR₃₁, O, or CR₃₁R₃₂;
R₁, R₂, R₃, and R₄ are each independently selected from H, halogen, OH, =O,-NHC₁₋₄ alkyl, -N(C₁₋₄ alkyl)₂, CN, C₁₋₄ alkyl, C₁₋₄ alkoxy, halo C₁₋₄ alkoxy, halo C₁₋₄ alkyl, C₂₋₄ alkenyl, C₂₋₄ alkynyl, 5- to 8-membered heterocycloalkyl containing 1-3 heteroatoms selected from N, S, or O, or 6- to 9-membered aryl, wherein the alkyl, heterocycloalkyl, and aryl are optionally substituted with 1-3 groups selected from halogen, OH, NH₂, CN, and C₁₋₄ alkyl;
R₁₁, R₁₂, R₂₁, R₃₁, and R₃₂ are each independently selected from H, halogen, CN, C₁₋₄ alkyl, halo C₁₋₄ alkyl, C₂₋₄ alkenyl, C₂₋₄ alkynyl, C₁₋₄ alkoxy, halo C₁₋₄ alkoxy, -NHC₁₋₄ alkyl, -N(C₁₋₄ alkyl)₂, C₃₋₆ cycloalkyl, a 5- to 10-membered heterocyclic ring containing 1-3 heteroatoms selected from N, S, or O, and 6- to 9-membered aryl, wherein the alkyl, alkenyl, alkynyl, alkoxy, cycloalkyl, heterocyclic ring, or aryl is optionally substituted with 1-3 groups selected from halogen, C₁₋₄ alkyl, halo C₁₋₄ alkyl, C₁₋₄ alkoxy, halo C₁₋₄ alkoxy, OH, NH₂, and CN;
alternatively, R₁₁ and R₃₁, together with the atom(s) to which they are attached, form a C₃₋₆ carbocyclic ring, or a 5- to 6-membered heterocyclic ring containing 1-3 heteroatoms selected from N, S, or O, wherein the carbocyclic ring or heterocyclic ring is optionally substituted with 1-3 groups selected from halogen, C₁₋₄ alkyl, halo C₁₋₄ alkyl, C₁₋₄ alkoxy, OH, NH₂, and CN;
R is selected from C₅₋₁₀ bicyclic cycloalkyl, C₃₋₆ monocyclic cycloalkyl, 5- to 10-membered bicyclic heterocycloalkyl containing 1-3 heteroatoms selected from N, S, or O, or 4- to 6-membered monocyclic heterocycloalkyl containing 1-2 heteroatoms selected from N, S, or O, wherein the bicyclic cycloalkyl and bicyclic heterocycloalkyl are optionally substituted with 1-3 groups selected from halogen, C₁₋₄ alkyl, C₁₋₄ alkoxy, halo C₁₋₄ alkoxy, OH, NH₂, -NHC₁₋₄ alkyl, -N(C₁₋₄ alkyl)₂, and halo C₁₋₄ alkyl, and the monocyclic cycloalkyl and monocyclic heterocycloalkyl are optionally further substituted with 1-3 groups selected from halogen, =O, 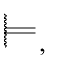 , 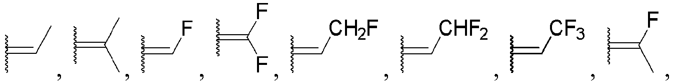 C₁₋₄ alkyl, C₁₋₄ alkoxy, halo C₁₋₄ alkoxy, or halo C₁₋₄ alkyl,
provided that the monocyclic cycloalkyl and monocyclic heterocycloalkyl are substituted with at least one group selected from 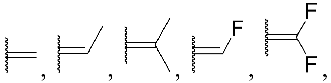 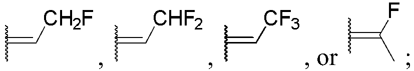 or
R is selected from C₅₋₁₀ bicyclic cycloalkyl, C₃₋₆ monocyclic cycloalkyl, 5- to 10-membered bicyclic heterocycloalkyl containing 1-3 heteroatoms selected from N, S, or O, or 4- to 6-membered monocyclic heterocycloalkyl containing 1-2 heteroatoms selected from N, S, or O, wherein the bicyclic cycloalkyl and bicyclic heterocycloalkyl are optionally substituted with 1-3 groups selected from halogen, C₁₋₄ alkyl, C₁₋₄ alkoxy, halo C₁₋₄ alkoxy, OH, NH₂, -NHC₁₋₄ alkyl, -N(C₁₋₄ alkyl)₂, and halo C₁₋₄ alkyl, and the monocyclic cycloalkyl and monocyclic heterocycloalkyl are further substituted with groups 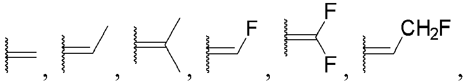 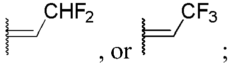 in some embodiments, R is selected from 5- to 10-membered bicyclic heterocycloalkyl containing 1-3 heteroatoms selected from N, S, or O, or 4-to 6-membered monocyclic heterocycloalkyl containing 1-2 heteroatoms selected from N, S, or O, wherein the bicyclic heterocycloalkyl is optionally substituted with 1-3 groups selected from halogen, C₁₋₄ alkyl, C₁₋₄ alkoxy, halo C₁₋₄ alkoxy, OH, NH₂, -NHC₁₋₄ alkyl, -N(C₁₋₄ alkyl)₂, and halo C₁₋₄ alkyl, and the monocyclic heterocycloalkyl is further substituted with groups 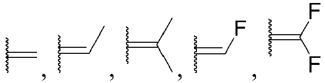 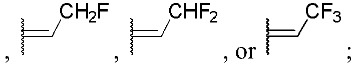
other groups are as defined in any of the preceding technical solutions.

Specifically, as a third technical solution of the present invention, the present invention provides a compound represented by formula I, or a stereoisomer, a deuterated substance or a pharmaceutically acceptable salt thereof,
wherein ring A is 5-membered heterocycloalkyl or 5-membered heteroaryl containing 1-3 heteroatoms selected from N, S, or O, wherein the heterocycloalkyl or heteroaryl is optionally substituted with 1-2 groups selected from halogen, C₁₋₄ alkoxy, -NHC₁₋₄ alkyl, -N(C₁₋₄ alkyl)₂, CN, C₁₋₄ alkyl, halo C₁₋₄ alkoxy, and halo C₁₋₄ alkyl; in some embodiments, ring A is 5-membered heteroaryl containing 1-3 heteroatoms selected from N, S, or O, wherein the heteroaryl is optionally substituted with 1-2 groups selected from halogen, C₁₋₄ alkoxy, -NHC₁₋₄ alkyl, - N(C₁₋₄ alkyl)₂, CN, C₁₋₄ alkyl, halo C₁₋₄ alkoxy, and halo C₁₋₄ alkyl;
ring B is 6-membered heteroaryl containing 1-3 heteroatoms selected from N, S, or O, wherein the heteroaryl is optionally substituted with 1-2 groups selected from =O, halogen, C₁₋₄ alkyl, C₁₋₄ alkoxy, halo C₁₋₄ alkyl, C₃₋₆ cycloalkyl, -C₁₋₂ alkyl-C₃₋₆ cycloalkyl, or 5- to 6-membered heterocycloalkyl containing 1-3 heteroatoms selected from N, S, or O, wherein the heterocyclyl and cycloalkyl are optionally further substituted with groups 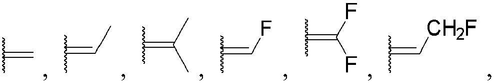 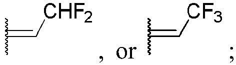 in some embodiments, ring B is 6-membered heteroaryl containing 1-3 heteroatoms selected from N, S, or O, wherein the heteroaryl is optionally substituted with 1-2 groups selected from =O, halogen, C₁₋₄ alkyl, halo C₁₋₄ alkyl, and 5- to 6-membered heterocycloalkyl containing 1-3 heteroatoms selected from N, S, or O, wherein the heterocyclyl is optionally further substituted with groups 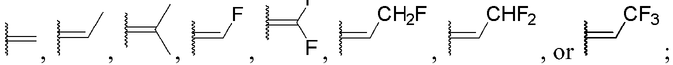
ring D is 5- to 6-membered heterocyclyl containing 1-3 heteroatoms selected from N, S, or O, wherein the heterocyclyl is optionally substituted with 1-2 groups selected from halogen, CN, C₁₋₄ alkyl, and halo C₁₋₄ alkyl;
R₁, R₂, R₃, and R₄ are each independently selected from H, halogen, =O, CN, C₁₋₄ alkyl, C₁₋₄ alkoxy, halo C₁₋₄ alkoxy, halo C₁₋₄ alkyl, 5- to 8-membered heterocycloalkyl containing 1-3 heteroatoms selected from N, S, or O, or 6- to 9-membered aryl, wherein the alkyl, heterocycloalkyl, and aryl are optionally substituted with 1-3 groups selected from halogen, CN, and C₁₋₄ alkyl;
other groups are as defined in any of the preceding technical solutions.

Specifically, as a fourth technical solution of the present invention, the present invention provides a compound represented by formula I, or a stereoisomer, a deuterated substance or a pharmaceutically acceptable salt thereof,
wherein each n is independently selected from 0, 1, 2, or 3;
each X₁ is independently selected from N or CR₁₁;
each X₂ is independently selected from N, C, or CR₂₁;
X₃ is selected from NR₃₁ or O;
X₄ is S(O)₂;
X₅ is CRₓ₅;
X₆ is CRₓ₆;
X₇ is CRₓ₇;
X₈ is CRₓ₈;
R₁₁, R₂₁, R₃₁, Rₓ₅, Rₓ₆, Rₓ₇, and Rₓ₈ are each independently selected from H, halogen, CN, C₁₋₄ alkyl, halo C₁₋₄ alkyl, C₁₋₄ alkoxy, halo C₁₋₄ alkoxy, a 5- to 6-membered heterocyclic ring containing 1-3 heteroatoms selected from N, S, or O, and 6-membered aryl, wherein the alkyl, alkoxy, heterocyclic ring, or aryl is optionally substituted with 1-3 groups selected from halogen, C₁₋₄ alkyl, or halo C₁₋₄ alkyl;
other groups are as defined in any of the preceding technical solutions.

Specifically, as a fifth technical solution of the present invention, the present invention provides a compound represented by formula I, or a stereoisomer, a deuterated substance or a pharmaceutically acceptable salt thereof, which are further represented by formula II or III, 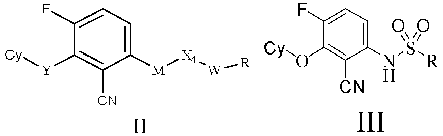 wherein Cy, Y, M, W, X₄, and R are as defined in any of the preceding technical solutions.

Specifically, as a sixth technical solution of the present invention, the present invention provides a compound represented by formula I, or a stereoisomer, a deuterated substance or a pharmaceutically acceptable salt thereof,
wherein Y is O, C=O, or NH; in some embodiments, Y is O or C=O; preferably, Y is O;
M is NRₘ;
W is a bond;
Rₘ is H;
R is selected from 6- to 10-membered spirocyclic heterocycloalkyl containing 1-3 heteroatoms selected from N, S, or O, or 4- to 5-membered monocyclic heterocycloalkyl containing 1-2 heteroatoms selected from N, S, or O, wherein the spirocyclic heterocycloalkyl is optionally substituted with 1-3 groups selected from halogen, C₁₋₄ alkyl, C₁₋₄ alkoxy, halo C₁₋₄ alkoxy, OH, NH₂, -NHC₁₋₄ alkyl, -N(C₁₋₄ alkyl)₂, and halo C₁₋₄ alkyl, and the monocyclic heterocycloalkyl is optionally further substituted with 1-3 groups selected from F, Cl, Br, =O, 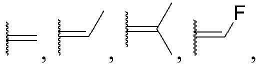 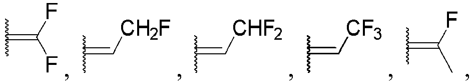 -CH₃, -CH₂CH₃, -CH₂F, -CHF₂, - CF₃, -CH₂CH₂F, -CH₂CHF₂, or -CH₂CF₃, provided that the monocyclic heterocycloalkyl is substituted with at least one group selected from 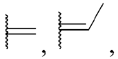 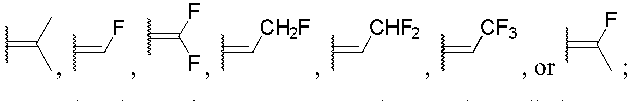 or
R is selected from 6- to 10-membered spirocyclic heterocycloalkyl containing 1-3 heteroatoms selected from N, S, or O, or 4- to 5-membered monocyclic heterocycloalkyl containing 1-2 heteroatoms selected from N, S, or O, wherein the spirocyclic heterocycloalkyl is optionally substituted with 1-3 groups selected from halogen, C₁₋₄ alkyl, C₁₋₄ alkoxy, halo C₁₋₄ alkoxy, OH, NH₂, -NHC₁₋₄ alkyl, -N(C₁₋₄ alkyl)₂, and halo C₁₋₄ alkyl, and the monocyclic heterocycloalkyl is further substituted with 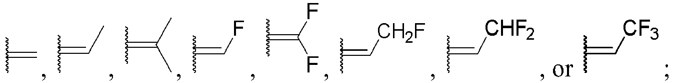
other groups are as defined in any of the preceding technical solutions.

Specifically, as a seventh technical solution of the present invention, the present invention provides a compound represented by formula I, or a stereoisomer, a deuterated substance or a pharmaceutically acceptable salt thereof,
wherein Y is O or C=O; preferably, Y is O;
M is NRₘ;
W is NR_{w};
Rₘ is H;
R_{w} is H or CH₃;
R is selected from C₆₋₁₀ spirocycloalkyl or C₄₋₅ monocyclic cycloalkyl, wherein the spirocycloalkyl is optionally substituted with 1-3 groups selected from halogen, C₁₋₄ alkyl, C₁₋₄ alkoxy, halo C₁₋₄ alkoxy, OH, NH₂, -NHC₁₋₄ alkyl, -N(C₁₋₄ alkyl)₂, and halo C₁₋₄ alkyl, and the monocyclic cycloalkyl is optionally further substituted with 1-3 groups selected from F, Cl, Br, =O, 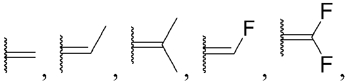 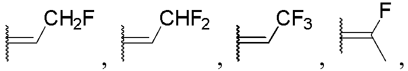 -CH₃, -CH₂CH₃, -CH₂F, -CHF₂, -CF₃, - CH₂CH₂F, -CH₂CHF₂, or -CH₂CF₃,
provided that the monocyclic cycloalkyl is substituted with at least one group selected from 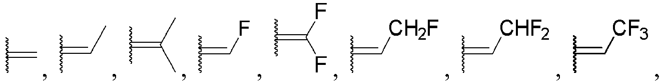 or 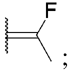 or
R is selected from C₆₋₁₀ spirocycloalkyl or C₄₋₅ monocyclic cycloalkyl, wherein the spirocycloalkyl is optionally substituted with 1-3 groups selected from halogen, C₁₋₄ alkyl, C₁₋₄ alkoxy, halo C₁₋₄ alkoxy, OH, NH₂, -NHC₁₋₄ alkyl, -N(C₁₋₄ alkyl)₂, and halo C₁₋₄ alkyl, and the monocyclic cycloalkyl is further substituted with groups 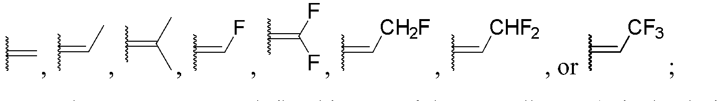
other groups are as defined in any of the preceding technical solutions.

Specifically, as an eighth technical solution of the present invention, the present invention provides compounds represented by formula I, formula II, and formula III, or a stereoisomer, a deuterated substance or a pharmaceutically acceptable salt thereof,
wherein R is selected from 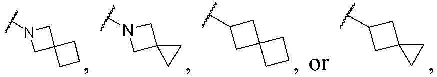 which are optionally substituted with 1-3 groups selected from F, Cl, Br, -CH₃, -CH₂CH₃, -CH₂F, -CHF₂, -CF₃, -CH₂CH₂F, -CH₂CHF₂, or -CH₂CF₃, is selected from 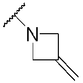 or 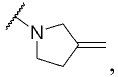 which are optionally substituted with 1-2 groups selected from F, Cl, Br, -CH₃, -CH₂CH₃, -CH₂F, -CHF₂, -CF₃, -CH₂CH₂F, -CH₂CHF₂, or -CH₂CF₃, or is selected from 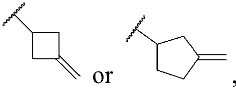 which are optionally substituted with 1-2 groups selected from F, Cl, Br, -CH₃, -CH₂CH₃, -CH₂F, -CHF₂, -CF₃, -CH₂CH₂F, - CH₂CHF₂, or -CH₂CF₃; in some embodiments, R is selected from 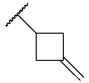 or 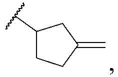 which are optionally substituted with 1-2 groups selected from F, Cl, Br, -CH₃, -CH₂CH₃, -CH₂F, -CHF₂, -CF₃, -CH₂CH₂F, -CH₂CHF₂, or -CH₂CF₃; in some embodiments, R is selected from 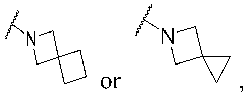 which are optionally substituted with 1-3 groups selected from F, Cl, Br, -CH₃, -CH₂CH₃, -CH₂F, -CHF₂, -CF₃, -CH₂CH₂F, -CH₂CHF₂, or -CH₂CF₃, or is selected from 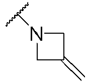 or 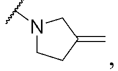 which are optionally substituted with 1-2 groups selected from F, Cl, Br, -CH₃, -CH₂CH₃, -CH₂F, -CHF₂, -CF₃, -CH₂CH₂F, -CH₂CHF₂, or -CH₂CF₃;
other groups are as defined in any of the preceding technical solutions.

Specifically, as a ninth technical solution of the present invention, the present invention provides compounds represented by formula I, formula II, and formula III, or a stereoisomer, a deuterated substance or a pharmaceutically acceptable salt thereof,
wherein R is selected from 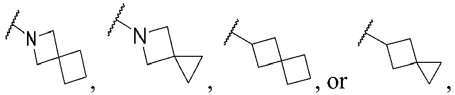 which are optionally substituted with 1-3 groups selected from F, Cl, Br, -CH₃, -CH₂CH₃, -CH₂F, -CHF₂, -CF₃, -CH₂CH₂F, -CH₂CHF₂, or -CH₂CF₃;
alternatively, R is selected from 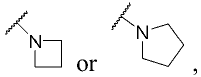 which are optionally substituted with 1-2 groups selected from F, Cl, Br, 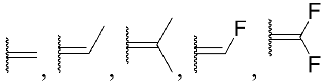 , 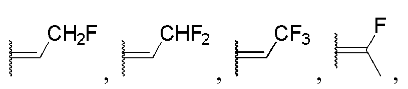 -CH₃, -CH₂CH₃, -CH₂F, -CHF₂, -CF₃, - CH₂CH₂F, -CH₂CHF₂, or -CH₂CF₃, and 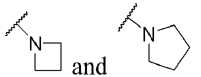 are substituted with at least one group selected from 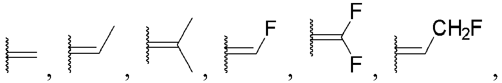 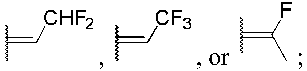
alternatively, R is selected from 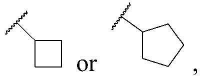 which are optionally substituted with 1-2 groups selected from F, Cl, Br, 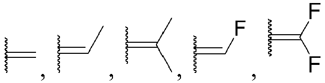 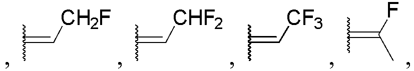 -CH₃, -CH₂CH₃, -CH₂F, -CHF₂, -CF₃, - CH₂CH₂F, -CH₂CHF₂, or -CH₂CF₃, and 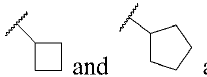 are substituted with at least one group selected from 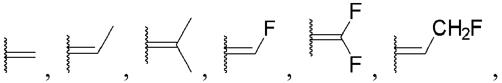 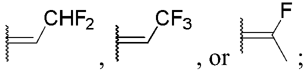
other groups are as defined in any of the preceding technical solutions.

Specifically, as a tenth technical solution of the present invention, the present invention provides compounds represented by formula I, formula II, and formula III, or a stereoisomer, a deuterated substance or a pharmaceutically acceptable salt thereof, wherein
P1 is selected from 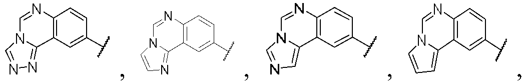 or 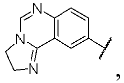 which are optionally substituted with 1-3 groups selected from F, Cl, Br, -CH₃, -CH₂CH₃, -CH₂F, -CHF₂, -CF₃, -CH₂CH₂F, -CH₂CHF₂, or -CH₂CF₃; in some embodiments, P1 is selected from 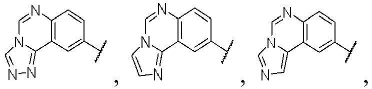 or 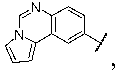 which are optionally substituted with 1-3 groups selected from F, Cl, Br, -CH₃, -CH₂CH₃, -CH₂F, -CHF₂, -CF₃, -CH₂CH₂F, -CH₂CHF₂, or -CH₂CF₃;
P2 is selected from 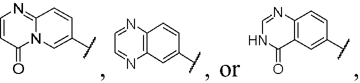 which are optionally substituted with 1-3 groups selected from F, Cl, Br, -CH₃, -CH₂CH₃, -CH₂F, -CHF₂, -CF₃, -CH₂CH₂F, -CH₂CHF₂, -CH₂CF₃, -OCH₃, cyclopropyl, 6-membered heterocycloalkyl containing 1-3 heteroatoms selected from N, S, or O, 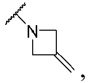 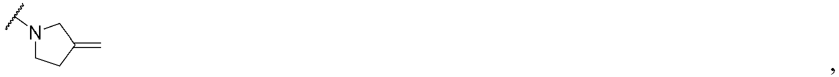 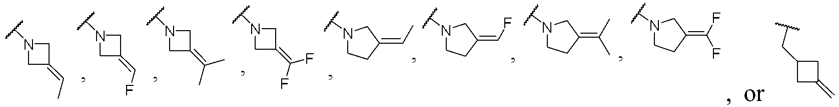 ;in some embodiments, P2 is selected from 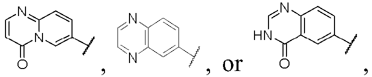 which are optionally substituted with 1-3 groups selected from F, Cl, Br, =O, -CH₃, -CH₂CH₃, -CH₂F, -CHF₂, -CF₃, -CH₂CH₂F, -CH₂CHF₂, -CH₂CF₃, 6-membered heterocycloalkyl containing 1-3 heteroatoms selected from N, S, or O, 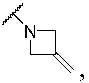 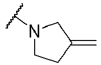 , or 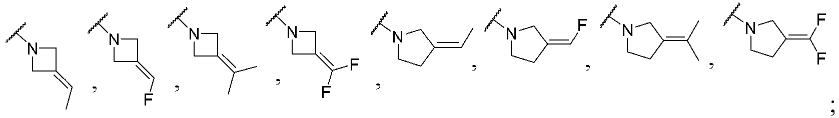
P3 is selected from 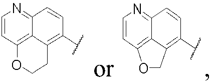 which are optionally substituted with 1-3 groups selected from F, Cl, Br, =O, -CH₃, -CH₂CH₃, -CH₂F, -CHF₂, -CF₃, - CH₂CH₂F, -CH₂CHF₂, or -CH₂CF₃;
P4 is selected from 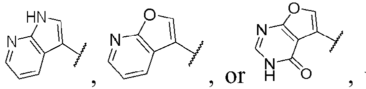 which are optionally substituted with 1-3 groups selected from F, Cl, Br, =O, -CH₃, -CH₂CH₃, -CH₂F, - CHF₂, -CF₃, -CH₂CH₂F, -CH₂CHF₂, -CH₂CF₃, or 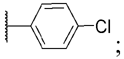
other groups are as defined in any of the preceding technical solutions.

Specifically, as an eleventh technical solution of the present invention, the present invention provides a compound represented by formula I, or a stereoisomer, a deuterated substance or a pharmaceutically acceptable salt thereof, wherein
Y is O or NH; in some embodiments, Y is O;
M is NH;
W is a bond;
X₄ is S(O)₂;
X₅ is CRₓ₅;
X₆ is CRₓ₆;
X₇ is CRₓ₇;
X₈ is CRₓ₈;
Rₓ₅, Rₓ₆, Rₓ₇, and Rₓ₈ are each independently selected from H, F, Cl, CN, -CH₃, -CH₂CH₃, -CH₂F, -CHF₂, -CF₃, -CH₂CH₂F, -CH₂CHF₂, or -CH₂CF₃;
Cy is selected from 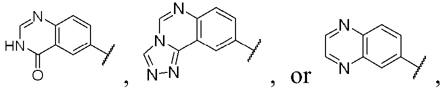 which are optionally substituted with 1-3 groups selected from F, Cl, -CH₃, -CH₂CH₃, -CH₂F, -CHF₂, -CF₃, -CH₂CH₂F, -CH₂CHF₂, -CH₂CF₃, -OCH₃, cyclopropyl, 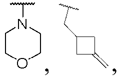 or 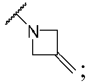 in some embodiments, Cy is 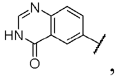 which is optionally substituted with 1-3 groups selected from F, Cl, -CH₃, -CH₂CH₃, -CH₂F, -CHF₂, -CF₃, - CH₂CH₂F, -CH₂CHF₂, or -CH₂CF₃;
R is selected from 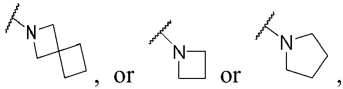 which are optionally substituted with 1-2 groups selected from F, Cl, Br, 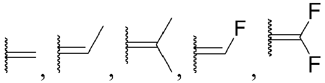 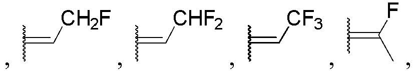 -CH₃, -CH₂CH₃, -CH₂F, -CHF₂, -CF₃,-CH₂CH₂F, -CH₂CHF₂, or -CH₂CF₃, and 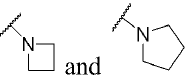 are substituted with at least one group selected from 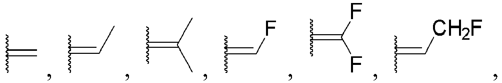 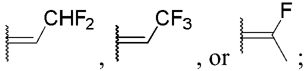
other groups are as defined in any of the preceding technical solutions.

Specifically, as a twelfth technical solution of the present invention, the present invention provides a compound represented by formula I, or a stereoisomer, a deuterated substance or a pharmaceutically acceptable salt thereof, wherein
X₅ is CRₓ₅; X₆ is CRₓ₆; X₇ is CRₓ₇; X₃ is CRₓ₈; Rₓ₅ is CN; Rₓ₆ is F or Cl;
Rₓ₇ and Rₓ₈ are each independently selected from H;
Cy is 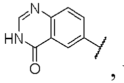 which is optionally substituted with 1-3 groups selected from -CH₂F, -CHF₂, -CF₃, -CH₂CH₂F, -CH₂CHF₂, or -CH₂CF₃;
R is selected from 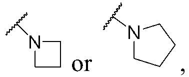 which are optionally substituted with 1-2 groups selected from F, 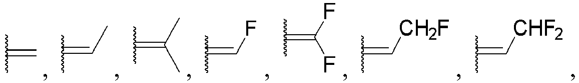 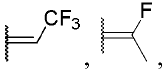 -CH₃, or -CH₂CH₃, and 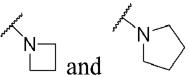 are substituted with at least one group selected from 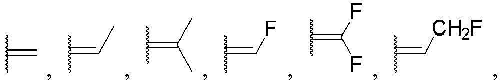 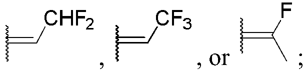
other groups are as defined in any of the preceding technical solutions.

Specifically, as a thirteenth technical solution of the present invention, the present invention provides a compound represented by formula I, II, or III, or a stereoisomer, a deuterated substance or a pharmaceutically acceptable salt thereof, wherein
Cy is 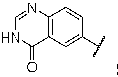 substituted with one group selected from CH₃, -CH₂F, -CHF₂, -CF₃, -CH₂CH₂F, -CH₂CHF₂, or -CH₂CF₃;
R is selected from 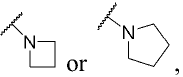 which are substituted with 1-2 groups selected from 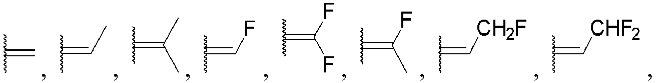 or

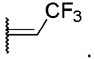

Specifically, as a fourteenth technical solution of the present invention, the present invention provides a compound represented by formula I, II, or III, or a stereoisomer, a deuterated substance or a pharmaceutically acceptable salt thereof, which further have the structure of formula IV: 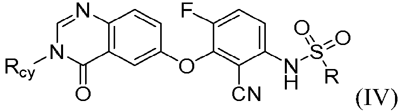
wherein R_{cy} is selected from C₁₋₂ alkyl or halo C₁₋₄ alkyl;
R is 4- to 6-membered monocyclic heterocyclyl containing 1-2 heteroatoms selected from N, S, or O, wherein the heterocycloalkyl is further substituted with 1-2 groups selected from

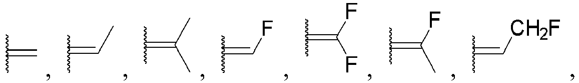

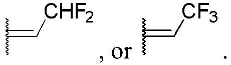

Specifically, as a fifteenth technical solution of the present invention, the present invention provides a compound represented by formula IV, or a stereoisomer, a deuterated substance or a pharmaceutically acceptable salt thereof, wherein R_{cy} is selected from CH₃, -CH₂F, -CHF₂, -CF₃, -CH₂CH₂F, -CH₂CHF₂, or - CH₂CF₃;
R is selected from 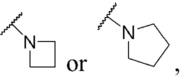 which are substituted with 1-2 groups selected from 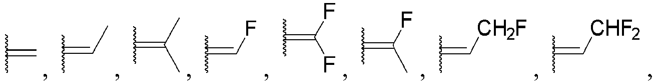 or

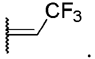

Specifically, as a technical solution of the present invention, the present invention provides the compound, or the stereoisomer, the deuterated substance or the pharmaceutically acceptable salt thereof described herein, wherein the compound is selected from one of the structures in Table 1

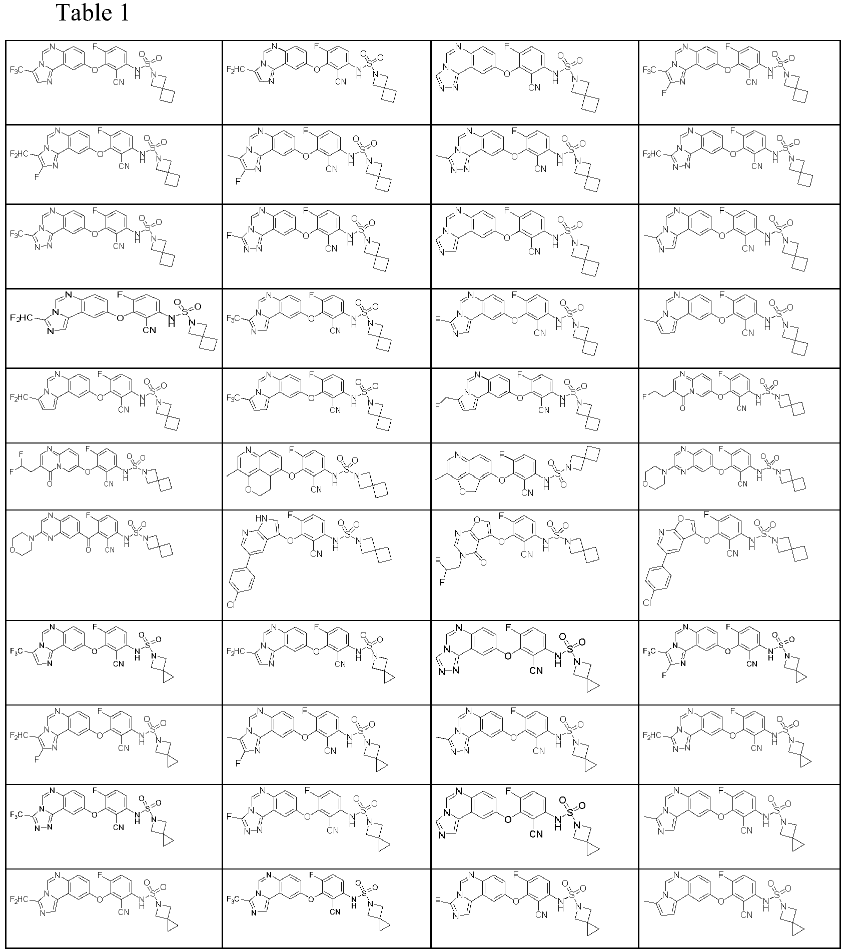

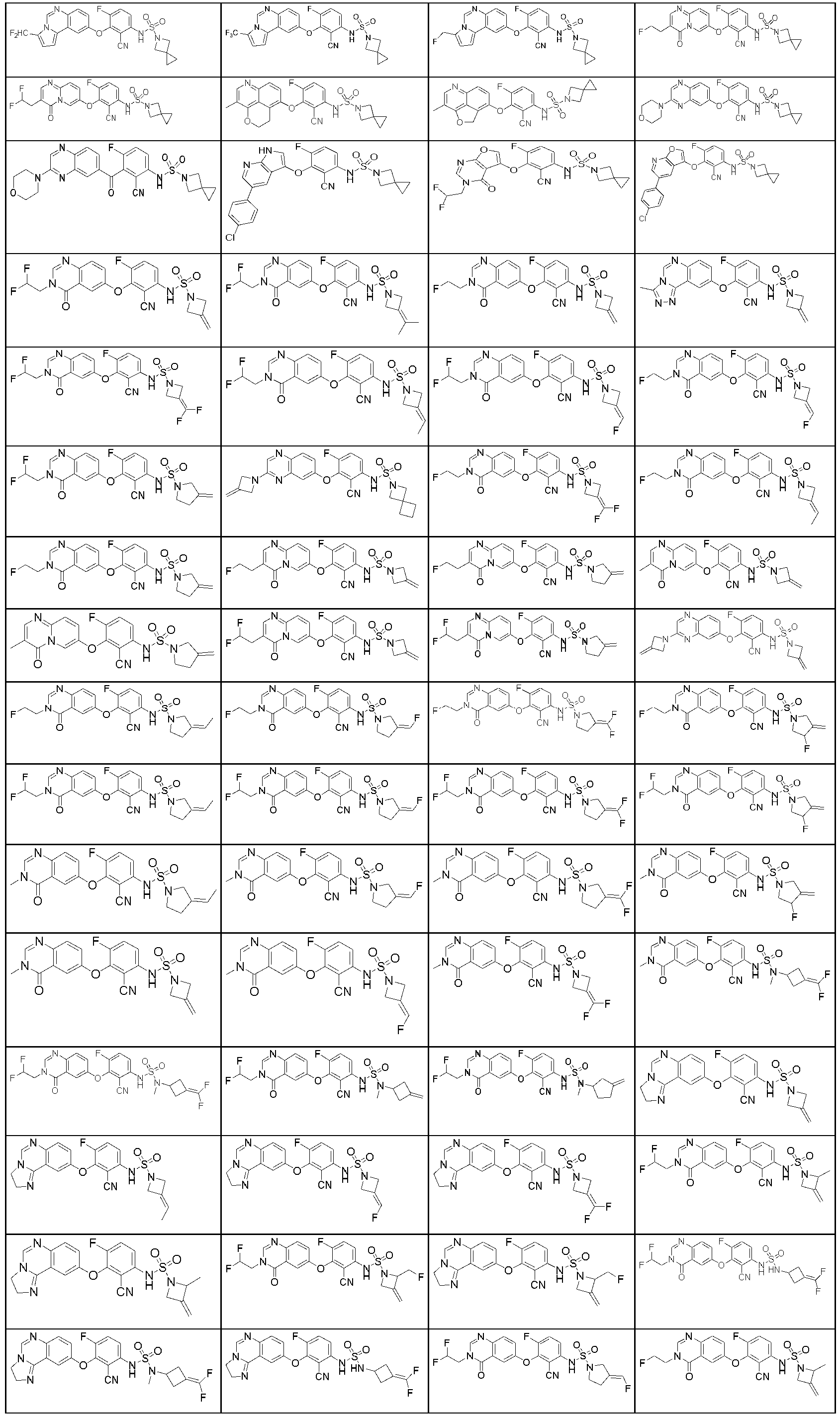

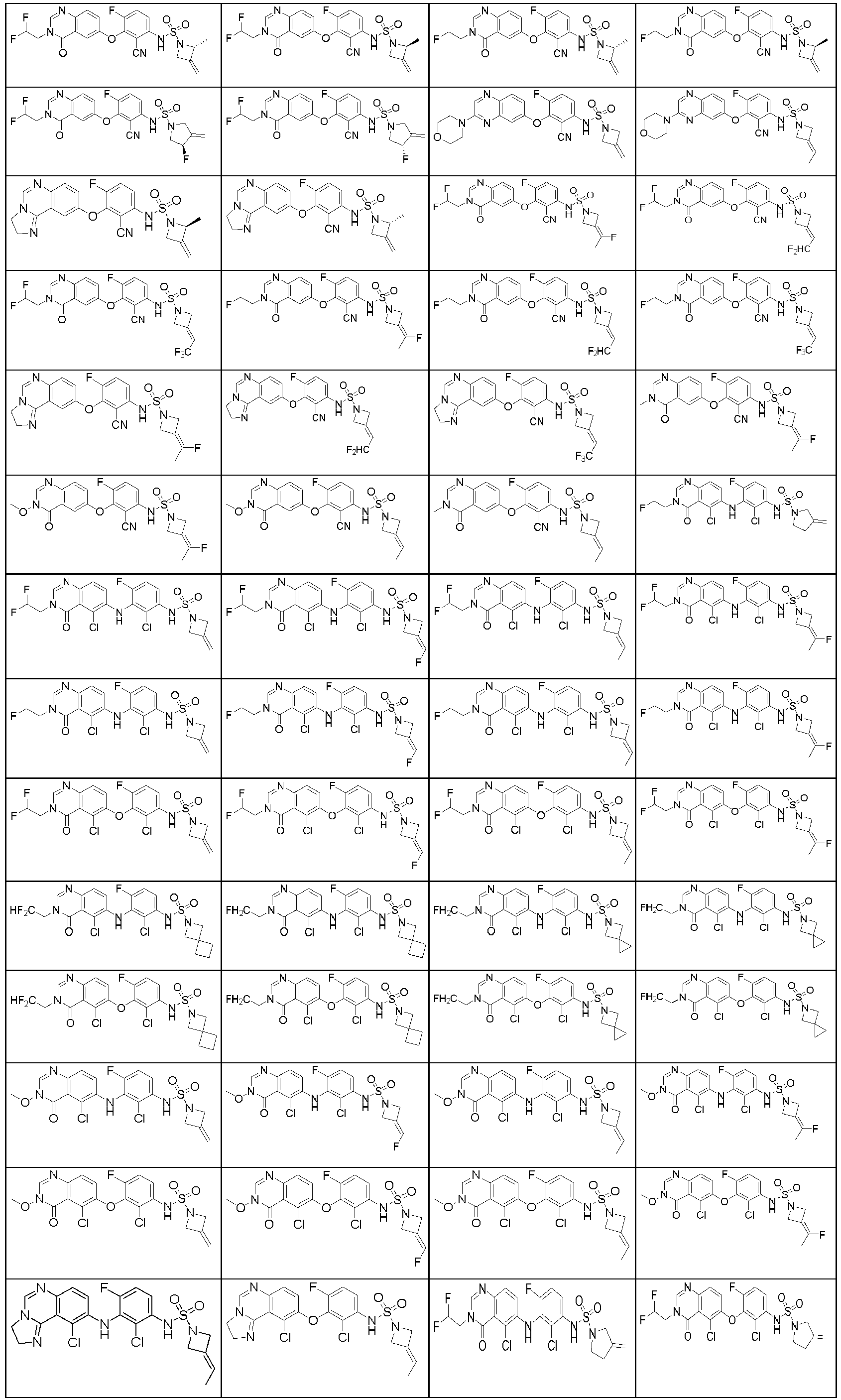

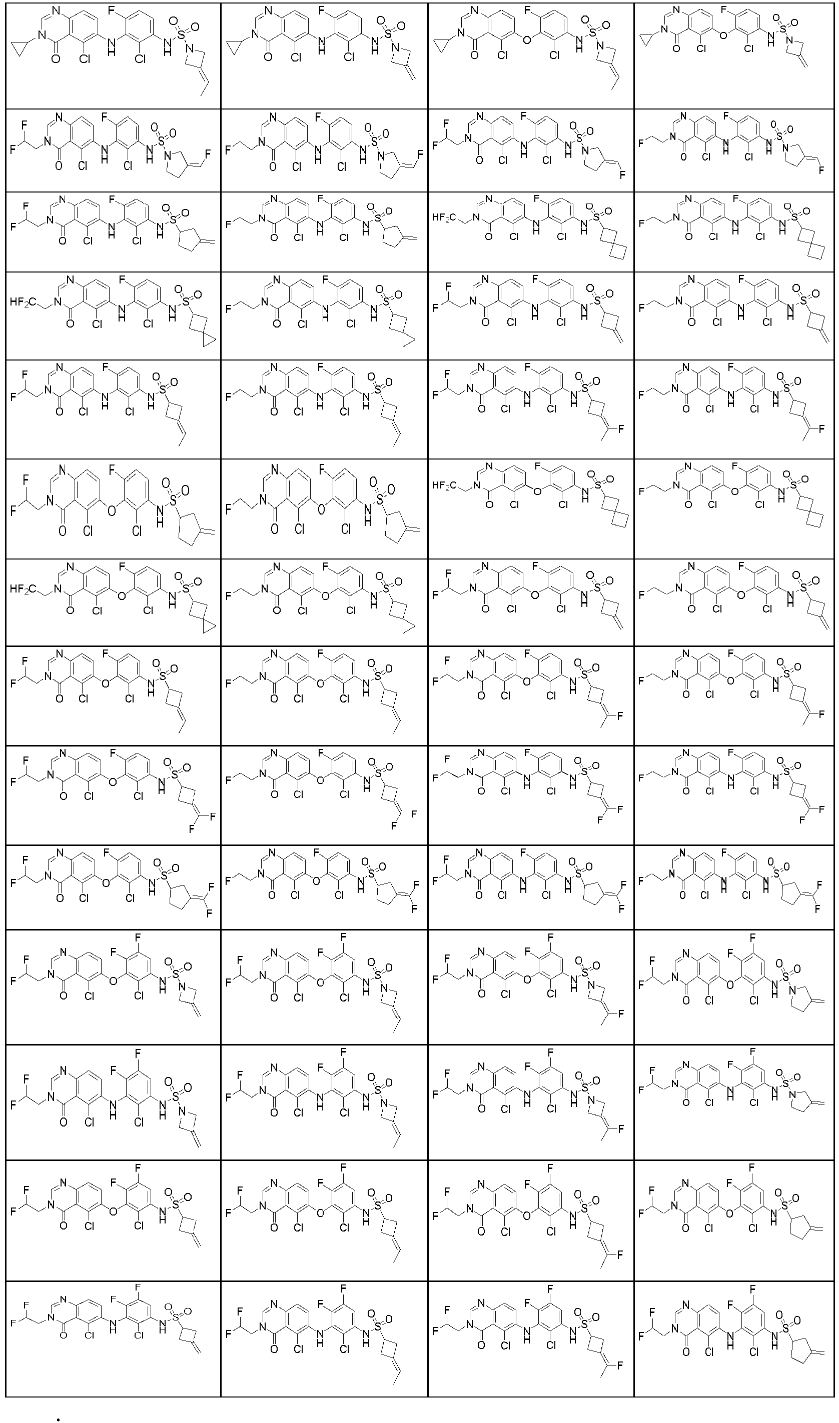

The present invention also provides a composition or pharmaceutical preparation, which comprises the compound, or the stereoisomer, the deuterated substance or the pharmaceutically acceptable salt thereof described in any one of the preceding solutions, and a pharmaceutically acceptable carrier and/or auxiliary material. The pharmaceutical composition can be in a unit preparation form (the unit preparation is also referred to as "preparation strength").

Further, the composition or pharmaceutical preparation of the present invention comprises 1-1500 mg of the compound, or the stereoisomer, the deuterated substance or the pharmaceutically acceptable salt thereof described in any one of the preceding solutions, and a pharmaceutically acceptable carrier and/or auxiliary material.

The present invention also provides the use of the compound, or the stereoisomer, the deuterated substance or the pharmaceutically acceptable salt thereof described in any one of the preceding solutions in the preparation of a medicament for the treatment/prevention of a BRAF-mediated disease. Further, the BRAF-mediated disease is a tumour; further preferably, the tumour is brain tumour, melanoma, colorectal cancer, non-small cell lung cancer, glioma, papillary thyroid carcinoma, or skin cancer.

The present invention also provides a method for treating a disease in a mammal, comprising administering to a subject a therapeutically effective amount of the compound, or the stereoisomer, deuterated substance, or pharmaceutically acceptable salt thereof according to any one of the preceding solutions, wherein the disease is preferably a tumour, and the therapeutically effective amount is preferably 1-1500 mg. In some embodiments, the mammal described in the present invention comprises a human.

The "effective amount" or "therapeutically effective amount" described in the present application refers to administration of a sufficient amount of the compound disclosed in the present application that will alleviate to some extent one or more symptoms of the diseases or conditions being treated. In some embodiments, the outcome is the reduction and/or remission of signs, symptoms, or causes of the disease, or any other desired change in the biological system. For example, an "effective amount" in terms of the therapeutic use is an amount of the composition comprising the compound disclosed in the present application that is required to provide clinically significant reduction of the symptoms of the disease. Examples of the therapeutically effective amount include, but are not limited to 1-1500 mg, 1-1400 mg, 1-1300 mg, 1-1200 mg, 1-1000 mg, 1-900 mg, 1-800 mg, 1-700 mg, 1-600 mg, 1-500 mg, 1-400 mg, 1-300 mg, 1-250 mg, 1-200 mg, 1-150 mg, 1-125 mg, 1-100 mg, 1-80 mg, 1-60 mg, 1-50 mg, 1-40 mg, 1-25 mg, 1-20 mg, 5-1500 mg, 5-1000 mg, 5-900 mg, 5-800 mg, 5-700 mg, 5-600 mg, 5-500 mg, 5-400 mg, 5-300 mg, 5-250 mg, 5-200 mg, 5-150 mg, 5-125 mg, 5-100 mg, 5-90 mg, 5-70 mg, 5-80 mg, 5-60 mg, 5-50 mg, 5-40 mg, 5-30 mg, 5-25 mg, 5-20 mg, 10-1500 mg, 10-1000 mg, 10-900 mg, 10-800 mg, 10-700 mg, 10-600 mg, 10-500 mg, 10-450 mg, 10-400 mg, 10-300 mg, 10-250 mg, 10-200 mg, 10-150 mg, 10-125 mg, 10-100 mg, 10-90 mg, 10-80 mg, 10-70 mg, 10-60 mg, 10-50 mg, 10-40 mg, 10-30 mg, and 10-20 mg; 20-1500 mg, 20-1000 mg, 20-900 mg, 20-800 mg, 20-700 mg, 20-600 mg, 20-500 mg, 20-400 mg, 20-350 mg, 20-300 mg, 20-250 mg, 20-200 mg, 20-150 mg, 20-125 mg, 20-100 mg, 20-90 mg, 20-80 mg, 20-70 mg, 20-60 mg, 20-50 mg, 20-40 mg, and 20-30 mg; 50-1500 mg, 50-1000 mg, 50-900 mg, 50-800 mg, 50-700 mg, 50-600 mg, 50-500 mg, 50-400 mg, 50-300 mg, 50-250 mg, 50-200 mg, 50-150 mg, 50-125 mg, and 50-100 mg; 100-1500 mg, 100-1000 mg, 100-900 mg, 100-800 mg, 100-700 mg, 100-600 mg, 100-500 mg, 100-400 mg, 100-300 mg, 100-250 mg, and 100-200 mg;
50-300 mg, 50-250 mg, 50-200 mg, 50-150 mg, 50-125 mg, and 50-100 mg; 100-1500 mg, 100-1000 mg, 100-900 mg, 100-800 mg, 100-700 mg, 100-600 mg, 100-500 mg, 100-400 mg, 100-300 mg, 100-250 mg, and 100-200 mg;
-200 mg, 50-150 mg, 50-125 mg, and 50-100 mg; 100-1500 mg, 100-1000 mg, 100-900 mg, 100-800 mg, 100-700 mg, 100-600 mg, 100-500 mg, 100-400 mg, 100-300 mg, 100-250 mg, and 100-200 mg;
in some embodiments, the pharmaceutical composition or preparation according to the present invention comprises the above therapeutically effective amount of the compound, or the stereoisomer, the deuterated substance or the pharmaceutically acceptable salt thereof according to the present invention;
the present invention relates to a pharmaceutical composition or pharmaceutical preparation, which comprises a therapeutically effective amount of the compound, or the stereoisomer, the deuterated substance or the pharmaceutically acceptable salt thereof according to the present invention, and a carrier and/or excipient. The pharmaceutical composition can be in a unit preparation form (the amount of the active ingredient in the unit preparation is also referred to as the "preparation strength"). In some embodiments, the pharmaceutical composition comprises the compound, or the stereoisomer, the deuterated substance or the pharmaceutically acceptable salt thereof according to the present invention in an amount including not limited to 1 mg, 1.25 mg, 2.5 mg, 5 mg, 10 mg, 12.5 mg, 15 mg, 20 mg, 25 mg, 30 mg, 35 mg, 40 mg, 45 mg, 50 mg, 55 mg, 60 mg, 65 mg, 70 mg, 75 mg, 80 mg, 85 mg, 90 mg, 95 mg, 100 mg, 110 mg, 120 mg, 125 mg, 130 mg, 140 mg, 150 mg, 160 mg, 170 mg, 180 mg, 190 mg, 200 mg, 210 mg, 220 mg, 230 mg, 240 mg, 250 mg, 275 mg, 300 mg, 325 mg, 350 mg, 375 mg, 400 mg, 425 mg, 450 mg, 475 mg, 500 mg, 525 mg, 550 mg, 575 mg, 600 mg, 625 mg, 650 mg, 675 mg, 700 mg, 725 mg, 750 mg, 775 mg, 800 mg, 850 mg, 900 mg, 950 mg, 1000 mg, 1100 mg, 1200 mg, 1300 mg, 1400 mg, and 1500 mg.

The present invention also provides a method for treating a disease in a mammal, comprising administering to a subject a therapeutically effective amount of the compound, or the stereoisomer, the deuterated substance, or the pharmaceutically acceptable salt thereof according to the present invention, and a pharmaceutically acceptable carrier and/or excipient, wherein the therapeutically effective amount is preferably 1-1500 mg, and the disease is preferably a tumour, particularly brain tumour, melanoma, colorectal cancer, non-small cell lung cancer, glioma, papillary thyroid carcinoma, or skin cancer.

Provided is a method for treating a disease in a mammal, comprising administering to a subject a drug, i.e., the compound, or the stereoisomer, the deuterated substance, or the pharmaceutically acceptable salt thereof according to the present invention, and a pharmaceutically acceptable carrier and/or excipient in a daily dose of 1-1500 mg/day, wherein the daily dose can be a single dose or divided doses; in some embodiments, the daily dose includes, but is not limited to 10-1500 mg/day, 20-1500 mg/day, 25-1500 mg/day, 50-1500 mg/day, 75-1500 mg/day, 100-1500 mg/day, 200-1500 mg/day, 10-1000 mg/day, 20-1000 mg/day, 25-1000 mg/day, 50-1000 mg/day, 75-1000 mg/day, 100-1000 mg/day, 200-1000 mg/day, 25-800 mg/day, 50-800 mg/day, 100-800 mg/day, 200-800 mg/day, 25-400 mg/day, 50-400 mg/day, 100-400 mg/day, and 200-400 mg/day; in some embodiments, the daily dose includes, but is not limited to 1 mg/day, 5 mg/day, 10 mg/day, 20 mg/day, 25 mg/day, 50 mg/day, 75 mg/day, 100 mg/day, 125 mg/day, 150 mg/day, 200 mg/day, 400 mg/day, 600 mg/day, 800 mg/day, 1000 mg/day, 1200 mg/day, 1400 mg/day, and 1500 mg/day.

The present invention relates to a kit, which may comprise a composition in a single-dose or multi-dose form, wherein the kit comprises the compound, or the stereoisomer, the deuterated substance or the pharmaceutically acceptable salt thereof according to the present invention, and the amount of the compound, or the stereoisomer, the deuterated substance or the pharmaceutically acceptable salt thereof according to the present invention is the same as that in the above-mentioned pharmaceutical composition.

In the present invention, the amount of the compound, or the stereoisomer or the pharmaceutically acceptable salt thereof according to the present invention is calculated in the form of a free base in each case.

The term "preparation strength" refers to the weight of the active ingredient contained in each vial, tablet or other unit preparation.

### Synthetic route

The preparation method of BRAF regulator is introduced in patent documents such as WO 2021116050A1, and those skilled in the art would have been able to prepare the compounds of the present invention by means of combining the document and known organic synthesis techniques, wherein the starting materials used therein are commercially available chemicals and (or) compounds described in chemical documents. "Commercially available chemicals" are obtained from regular commercial sources, and suppliers include: Titan Technology Co., Ltd., Energy Chemical Co., Ltd., Shanghai Demo Co., Ltd., Chengdu Kelong Chemical Co., Ltd., Accela ChemBio Co., Ltd., PharmaBlock Sciences (Nanjing), Inc., WuXi Apptec Co., Ltd., J&K Scientific Co., Ltd., etc.

Specific and similar reactants can be selectively identified by the indexes of known chemicals prepared by the Chemical Abstracts Service of the American Chemical Society, wherein the indexes are available in most public libraries and university libraries and online. Chemicals that are known but not commercially available in the catalogue are optionally prepared by custom chemical synthesis plants, wherein many of standard chemical supply plants (for example, those listed above) provide custom synthesis services.

### Terms

Unless otherwise specified in the present invention, the terms of the present invention have the following meanings.

The carbon, hydrogen, oxygen, sulphur, nitrogen and halogen involved in the groups and compounds of the present invention all include isotopes thereof, and are optionally further replaced by one or more of the corresponding isotopes thereof, wherein the isotopes of carbon include ¹²C, ¹³C, and ¹⁴C; the isotopes of hydrogen include protium (H), deuterium (also known as heavy hydrogen) and tritium (T, also known as superheavy hydrogen); the isotopes of oxygen include ¹⁶O, ¹⁷O, and ¹⁸O; the isotopes of sulphur include ³²S, ³³S, ³⁴S, and ³⁶S; the isotopes of nitrogen include ¹⁴N and ¹⁵N; the isotope of fluorine includes ¹⁹F; the isotopes of chlorine include ³⁵Cl and ³⁷Cl; and the isotopes of bromine include ⁷⁹Br and ⁸¹Br.

The term "halogen" herein refers to F, Cl, Br, I, or isotopes thereof.

The term "halo" or "substituted with halogen" refers to being substituted with one or more groups selected from F, Cl, Br, I, or isotopes thereof, wherein the upper limit of the number of halogen substituents is equal to the sum of the number of hydrogens that can be substituted in the group to be substituted. Without particular limitation, the number of halogen substituents is any integer between 1 and the upper limit, and when the number of halogen substituents is greater than 1, the group to be substituted can be substituted with the same or different halogen. Generally, the circumstances of being substituted with 1-5 halogen, 1-3 halogen, 1-2 halogen, and 1 halogen are included.

The term "deuterium" refers to the isotope deuterium of hydrogen (H).

The term "deuterated" or "deuterated substance" refers to the case where a hydrogen atom on a group, such as alkyl, cycloalkyl, alkylene, aryl, heteroaryl, mercapto, heterocycloalkyl, alkenyl and alkynyl is substituted with at least one deuterium atom, wherein the upper limit of the number of deuterium substituents is equal to the sum of the number of hydrogens that can be substituted in the group to be substituted. Without particular limitation, the number of deuterium substituents is any integer between 1 and the upper limit, for example, 1-20 deuterium atoms, 1-10 deuterium atoms, 1-6 deuterium atoms, 1-3 deuterium atoms, 1-2 deuterium atoms or 1 deuterium atom.

Group "C_{x-y}" refers to a group comprising x to y carbon atoms, for example, "C₁₋₆ alkyl" refers to alkyl comprising 1-6 carbon atoms.

The term "alkyl" refers to a monovalent straight or branched saturated aliphatic hydrocarbon group, usually an alkyl group with 1 to 20 carbon atoms, or an alkyl group with 1 to 8 carbon atoms, or an alkyl group with 1 to 6 carbon atoms, or an alkyl group with 1 to 4 carbon atoms. Non-limiting examples include methyl, ethyl, n-propyl, isopropyl, n-butyl, sec-butyl, neobutyl, tert-butyl, n-pentyl, isopentyl, neopentyl, n-hexyl, etc., and alkyl may be further substituted with a substituent.

The term "alkylene" refers to a divalent straight or branched saturated alkyl group. Examples of alkylene include, but are not limited to methylene, ethylene, *etc.*

The term "haloalkyl" refers to an alkyl group in which one or more hydrogens are replaced by one or more halogen atoms (e.g., fluorine, chlorine, bromine, iodine, or isotopes thereof), wherein the upper limit of the number of halogen substituents is equal to the sum of the number of hydrogens that can be substituted in the alkyl group. Without particular limitation, the number of halogen substituents is any integer between 1 and the upper limit. Generally, the alkyl group is substituted with 1-5 halogen, 1-3 halogen, 1-2 halogen or 1 halogen; and when the number of halogen substituents is greater than 1, the group to be substituted can be substituted with the same or different halogen. Specific examples include, but are not limited to -CF₃, - CH₂Cl, -CH₂CF₃, -CCl₂, CF₃, etc.

The term "alkoxy" or "alkyloxy" refers to -O-alkyl, such as -O-C₁₋₈ alkyl, -O-C₁₋₆ alkyl, -O-C₁₋₄ alkyl or -O-C₁₋₂ alkyl. Non-limiting and specific examples include methoxy, ethoxy, n-propoxy, isopropoxy, n-butoxy, sec-butoxy, tert-butoxy, n-pentoxy, n-hexyloxy, cyclopropoxy, cyclobutoxy, etc. The alkoxy may be optionally substituted with a substituent.

The term "haloalkoxy" refers to -O-haloalkyl, such as -O-halo C₁₋₈ alkyl, -O-halo C₁₋₆ alkyl, -O-halo C₁₋₄ alkyl or -O-halo C₁₋₂ alkyl; the upper limit of the number of halogen substituents is equal to the sum of the number of hydrogens that can be substituted in the group to be substituted. Without particular limitation, the number of halogen substituents is any integer between 1 and the upper limit, preferably 1-5 halogen, 1-3 halogen, 1-2 halogen, and 1 halogen; and when the number of halogen substituents is greater than 1, the group to be substituted can be substituted with the same or different halogen. Non-limiting examples include monofluoromethoxy, difluoromethoxy, trifluoromethoxy, difluoroethyloxy, etc.

The term "alkenyl" refers to a straight or branched hydrocarbon group comprising at least one carbon-carbon double bond (C=C) and generally comprises 2 to 18 carbon atoms, such as 2 to 8 carbon atoms, further such as 2 to 6 carbon atoms, and still further such as 2 to 4 carbon atoms. Examples of alkenyl include, but are not limited to ethenyl, allyl, 1-propenyl, 2-propenyl, 1-butenyl, 2-butenyl, 3-butenyl, 1-pentenyl, 2-pentenyl, 3-pentenyl, 4-pentenyl, 1-methyl-1-butenyl, 2-methyl-1-butenyl, 2-methyl-3-butenyl, 1-hexenyl, 2-hexenyl, 3-hexenyl, 4-hexenyl, 5-hexenyl, 1-methyl-1-pentenyl, 2-methyl-1-pentenyl, 1-heptenyl, 2-heptenyl, 3-heptenyl, 4-heptenyl, 1-octenyl, 3-octenyl, 1-nonenyl, 3-nonenyl, 1-decenyl, 4-decenyl, 1,3-butadiene, 1,3-pentadiene, 1,4-pentadiene, 1,4-hexadiene, etc.; and the alkenyl may further be optionally substituted with a substituent.

The term "alkenylene" refers to a straight or branched divalent unsaturated hydrocarbon group comprising at least one carbon-carbon double bond (C=C). Unless otherwise specified, the alkynylene contains 2-6 carbon atoms, preferably 2-4 carbon atoms. Non-limiting examples of alkynylene include ethynylene, and the alkenylene may be optionally substituted with a substituent.

The term "alkynyl" refers to a straight or branched hydrocarbon group comprising at least one carbon-carbon triple bond (C≡C) and generally comprises 2 to 18 carbon atoms, further comprises 2 to 8 carbon atoms, further comprises 2 to 6 carbon atoms, and still further comprises 2 to 4 carbon atoms. Examples of alkynyl include, but are not limited to ethynyl, 1-propynyl, 2-propynyl, butynyl, 2-butynyl, 3-butynyl, 1-methyl-2-propynyl, 4-pentynyl, 3-pentynyl, 1-methyl-2-butynyl, 2-hexynyl, 3-hexynyl, 2-heptynyl, 3-heptynyl, 4-heptynyl, 3-octynyl, 3-nonynyl, 4-decynyl, etc.; and the alkynyl may be optionally substituted with a substituent.

The term "alkynylene" refers to a straight or branched divalent unsaturated hydrocarbon group comprising a carbon-carbon triple bond (C≡C) and generally comprises 2-6 carbon atoms, and further comprises 2-4 carbon atoms. Non-limiting examples of alkynylene include ethynylene, propynylene and butynylene; and the alkynylene may be optionally substituted with a substituent.

The term "cycloalkyl" refers to a saturated or partially unsaturated, non-aromatic carbocyclic hydrocarbon group containing no ring heteroatoms. The cycloalkyl may be monocyclic, bicyclic or polycyclic, the bicyclic or polycyclic cycloalkyl may be in the form of a fused ring, a spiro ring, a bridged ring or a combination thereof, and may comprise one or more aromatic rings, but the ring system is non-aromatic as a whole, and the attachment site may be on an aromatic ring or a non-aromatic ring. Generally, the cycloalkyl contains 3 to 20 carbon atoms, further contains 3-8 carbon atoms, and still further contains 3-6 carbon atoms; when the cycloalkyl is monocyclic cycloalkyl, the cycloalkyl contains 3-15 carbon atoms, or 3-10 carbon atoms, or 3-8 carbon atoms, or 3-6 carbon atoms; when the cycloalkyl is bicyclic or polycyclic cycloalkyl, the cycloalkyl contains 5-12 carbon atoms, or 5-11 carbon atoms, or 6-10 carbon atoms. Non-limiting examples include cyclopropyl, cyclobutyl, cyclopentyl, cyclohexyl, cycloheptyl, butenyl, cyclopentenyl, cyclohexenyl, 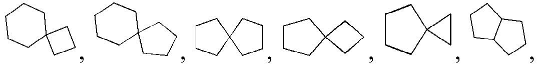 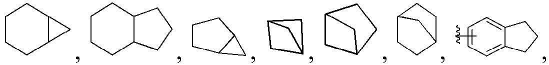 etc., and the cycloalkyl may be optionally substituted with a substituent.

The term "cycloalkylene" refers to a divalent group of cycloalkyl.

The term "aryl" refers to an aromatic carbocycle that does not contain heteroatoms, including monocyclic aryl and fused aryl. Generally, the aryl contains 6 to 13 carbon atoms, and further contains 6 to 9 carbon atoms, and it is further phenyl. Non-limiting examples of aryl include phenyl, naphthyl, anthryl and phenanthryl, and the aryl may be optionally substituted with a substituent.

"Carbocycle" or "carbocyclyl" refers to a saturated, partially unsaturated, or aromatic carbocycle, and its meaning includes aryl and cycloalkyl. The carbocycle may be monocyclic, bicyclic or polycyclic, and the bicyclic or polycyclic carbocycle may be in the form of a bridged ring, a fused ring, a spiro ring and a combination thereof. Generally, the carbocycle contains 3-12 carbon atoms, or 3-10 carbon atoms, or 3-6 carbon atoms. Non-limiting examples of the monocyclic carbocycle include cyclopropyl, cyclobutyl, cyclopentyl, cyclohexyl, cycloheptyl, phenyl, *etc.* A bicyclic bridged ring includes 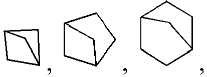 *etc.,* a bicyclic fused ring includes 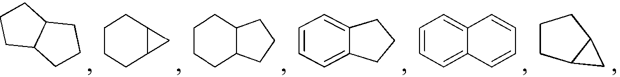 *etc.,* and a bicyclic spiro ring includes 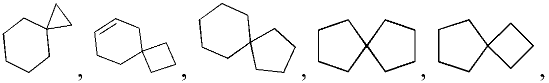 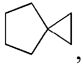 *etc.* The carbocycle may be optionally substituted with a substituent.

"Heterocycloalkyl" refers to a saturated or partially unsaturated non-aromatic carbocycle containing 1, 2, 3, or 4 heteroatoms selected from N, S, or O. The heterocycloalkyl may be monocyclic, bicyclic or polycyclic, the bicyclic or polycyclic heterocycloalkyl may be in the form of a bridged ring, a fused ring, a spiro ring or a combination thereof, and may comprise one or more aromatic rings or heteroaromatic rings, but the ring system is non-aromatic as a whole, and the attachment site may be on an aromatic ring or a non-aromatic ring. Generally, the heterocycloalkyl is a 3- to 20-membered ring. When the heterocycloalkyl is monocyclic heterocycloalkyl, it is usually a 3- to 15-membered ring, or a 3- to 10-membered ring, or a 3- to 8-membered ring, or a 3- to 6-membered ring; when the heterocycloalkyl is bicyclic or polycyclic heterocycloalkyl, the heterocycloalkyl is usually a 5- to 12-membered ring, or a 5- to 11-membered ring, or a 6- to 9-membered ring. The heteroatoms N and S include their oxidation states. Non-limiting examples of heterocycloalkyl include azetidinyl, morpholinyl, piperazinyl, piperidyl, tetrahydropyranyl, oxetanyl, pyranyl, azacyclopentenyl, azacyclohexenyl, oxacyclopentenyl, oxacyclohexenyl, 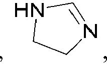 etc., and the heterocycloalkyl may be optionally substituted with a substituent.

"Heteroaromatic ring" or "heteroaryl", unless otherwise specified, refers to an aromatic ring containing 1 to 4 heteroatoms selected from N, O, or S and their oxidation states. "Heteroaromatic ring" or "heteroaryl" may contain =O, which may be monocyclic, bicyclic or polycyclic, wherein the bicyclic or polycyclic heteroaromatic ring or heteroaryl may be in the form of a bridged ring, a fused ring, a spiro ring and a combination thereof. The bicyclic or polycyclic heteroaromatic ring or heteroaryl can be formed by fusion of heteroaryl to aryl, or of heteroaryl to heteroaryl, wherein the heteroaryl or aryl may be the attachment site. Non-limiting examples include furyl, thienyl, pyrrolyl, oxazolyl, thiazolyl, imidazolyl, pyrazolyl, pyridyl, pyrimidyl, pyridazinyl, pyrazinyl, indolyl, purinyl, 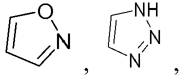 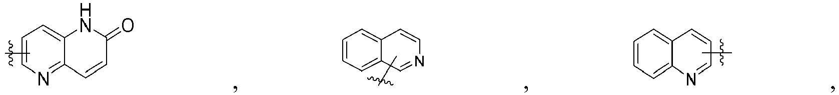 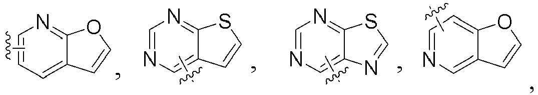 etc. The heteroaryl may be optionally substituted with a substituent.

"Heterocyclic ring" or "heterocyclyl" refers to a saturated or unsaturated, aromatic or non-aromatic ring containing 1 to 4 heteroatoms selected from N, O, or S and their oxidation states, and its meaning includes heteroaryl and heterocycloalkyl. The heterocyclic ring may be in the form of a monocyclic heterocyclic ring, a bicyclic bridged heterocyclic ring, a bicyclic fused heterocyclic ring, a bicyclic spiro heterocyclic ring or a combination thereof. The heterocyclic ring is usually a 3- to 12-membered heterocyclic ring, a 5- to 12-membered heterocyclic ring, or a 5- to 7-membered heterocyclic ring. Heterocyclyl can be connected to a heteroatom or a carbon atom. Non-limiting examples include oxiranyl, aziridinyl, oxetanyl, azetidinyl, 1,3-dioxolanyl, 1,4-dioxolanyl, 1,3-dioxanyl, piperazinyl, azepanyl, pyridyl, furyl, thienyl, pyranyl, N-alkylpyrrolyl, pyrimidyl, pyrazinyl, pyrazolyl, pyridazinyl, imidazolyl, piperidyl, piperidinyl, morpholinyl, thiomorpholinyl, 1,3-dithianyl, dihydrofuryl, dihydropyranyl, dithiolanyl, tetrahydrofuryl, tetrahydropyrrolyl, tetrahydroimidazolyl, oxazolyl, dihydrooxazolyl, tetrahydrooxazolyl, tetrahydrothiazolyl, tetrahydropyranyl, benzoimidazolyl, benzopyridyl, pyrrolopyridyl, benzodihydrofuryl, azabicyclo[3.2.1]octanyl, azabicyclo[5.2.0]nonanyl, oxatricyclo[5.3.1.1]dodecyl, azaadamantyl and oxaspiro[3.3]heptanyl, 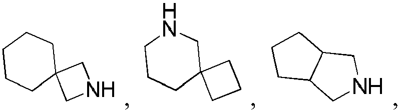 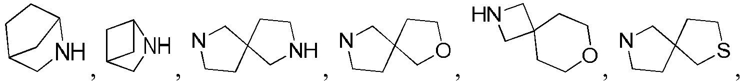 etc., and the heterocyclic ring may be optionally substituted with a substituent.

"Heterocyclene" refers to a substituted or unsubstituted, saturated or unsaturated, aromatic or non-aromatic, divalent heterocyclyl group. Non-limiting examples of heterocyclene include 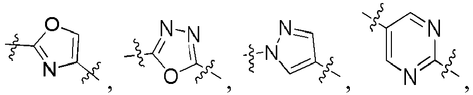 , 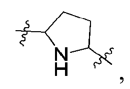 *etc*.

"Spiro ring" refers to a polycyclic group sharing one carbon atom (referred to as a spiro atom) between rings, which may contain 0 or at least 1 double or triple bond, and may contain 0 to 5 heteroatoms selected from N, O, S, P, Si and their oxidation states. Generally, a spiro ring is a 6- to 14-membered ring, or a 6- to 12-membered ring, or a 6- to 10-membered ring. Generally, a spiro ring is a spiro ring formed by a three-membered ring and a three-membered ring, a three-membered ring and a four-membered ring, a three-membered ring and a five-membered ring, a three-membered ring and a six-membered ring, a four-membered ring and a four-membered ring, a four-membered ring and a five-membered ring, a four-membered ring and a six-membered ring, a five-membered ring and a five-membered ring, or a five-membered ring and a six-membered ring. Non-limiting examples of the spiro ring include: 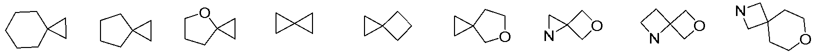 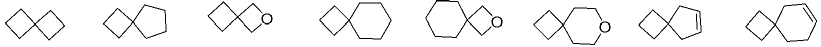 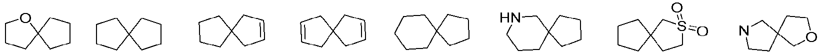 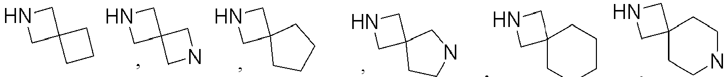 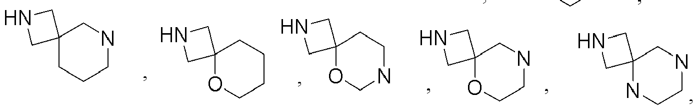 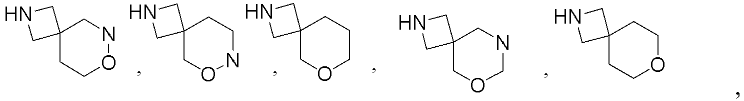 and the spiro ring may be optionally substituted with a substituent.

The term "fused ring" refers to a polycyclic group in which the rings share two adjacent ring atoms and one chemical bond, which may contain one or more double or triple bonds, and may contain 0 to 5 heteroatoms selected from N, S, O, P, Si and their oxidation states. Generally, a fused ring is a 5- to 20-membered ring, or a 5- to 14-membered ring, or a 5- to 12-membered ring, or a 5- to 10-membered ring. Generally, a fused ring is in the form of a three-membered ring fused a four-membered ring (indicating a fused ring formed by a three-membered ring and a four-membered ring, and either the three-membered ring or the four-membered ring may be possibly used as the basic ring according to the IUPC nomenclature; similarly hereinafter), a three-membered ring fused a five-membered ring, a three-membered ring fused a six-membered ring, a four-membered ring fused a four-membered ring, a four-membered ring fused a five-membered ring, a four-membered ring fused a six-membered ring, a five-membered ring fused a five-membered ring, a five-membered ring fused a six-membered ring, and a six-membered ring fused a six-membered ring. Non-limiting examples of the fused ring include purine, quinoline, isoquinoline, benzopyran, benzofuran, benzothiophene, and 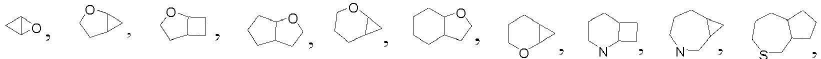 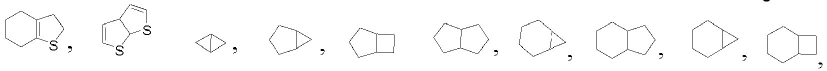 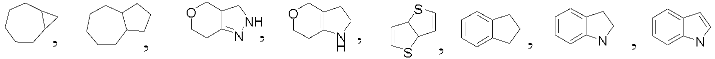 ; and the fused ring may be optionally substituted with a substituent.

The term "bridged ring" refers to a ring system in which two rings share two non-adjacent ring atoms, which may contain one or more double or triple bonds. The bridged ring may contain 0 to 5 heteroatoms selected from N, S, O, P, Si and their oxidation states. Generally, the bridged ring has 5 to 20, or 5 to 14, or 5 to 12, or 5 to 10 ring atoms. Non-limiting examples of the bridged ring include adamantane, 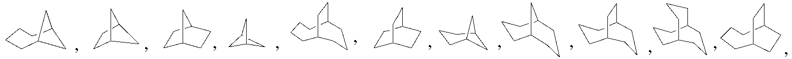 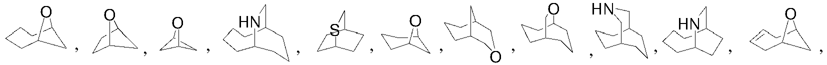 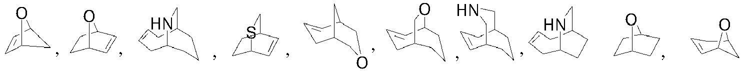 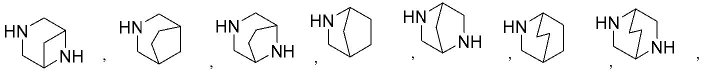 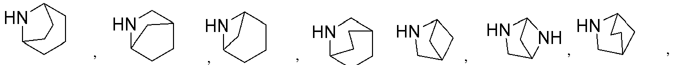 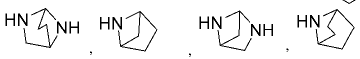 " 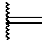 " denotes that two hydrogen atoms on the same carbon atom are simultaneously substituted with alkenyl; for example, cyclobutyl is substituted with 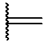 to form

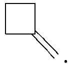

Unless otherwise specified, the term "substitution" or "substituent" refers to any substitution at a position allowed by chemical theory, and the number of substituents conforms to the rules of chemical bonding. Exemplary substituents include, but are not limited to: C₁₋₆ alkyl, C₂₋₆ alkenyl, C₂₋₆ alkynyl, C₃₋₈ heteroalkyl, C₅₋₁₂ aryl, 5- to 12-membered heteroaryl, hydroxyl, C₁₋₆ alkoxy, C₅₋₁₂ aryloxy, thiol group, C₁₋₆ alkylthio, cyano, halogen, C₁₋₆ alkylthiocarbonyl, C₁₋₆ alkylcarbamoyl, N-carbamoyl, nitro, silyl, sulphinyl, sulphonyl, sulphoxide, halo C₁₋₆ alkyl, halo C₁₋₆ alkoxy, amino, phosphonic acid, -CO₂(C₁₋₆ alkyl), -OC(=O)(C₁₋₆ alkyl), -OCO₂(C₁₋₆ alkyl), -C(=O)NH₂, -C(=O)N(C₁₋₆ alkyl)₂, -OC(=O)NH(C₁₋₆ alkyl), -NHC(=O)(C₁₋₆ alkyl), -N(C₁₋₆ alkyl)C(=O)(C₁₋₆ alkyl), -NHCO₂(C₁₋₆ alkyl), -NHC(=O)N(C₁₋₆ alkyl)₂, -HC(=O)NH(C₁₋₆ alkyl), -NHC(=O)NH₂, -NHSO₂(C₁₋₆ alkyl), -SO₂N(C₁₋₆ alkyl)₂, -SO₂NH(C₁₋₆ alkyl), -SO₂NH₂, -SO₂C₁₋₆ alkyl, etc.

The term "optional" or "optionally" refers to that the events or circumstances subsequently described may but not necessarily occur, and the description includes the occasions where the events or circumstances occur or do not occur. For example, "alkyl optionally substituted with F" means that the alkyl may but not necessarily be substituted with F, and the description includes the case where the alkyl is substituted with F and the case where the alkyl is not substituted with F.

The term "pharmaceutically acceptable salt" refers to a salt of the compound of the present invention, which salt maintains the biological effectiveness and characteristics of a free acid or a free base and is obtained by reacting the free acid with a non-toxic inorganic base or organic base, or reacting the free base with a non-toxic inorganic acid or organic acid.

The term "pharmaceutical composition" represents a mixture of one or more compounds or stereoisomers, solvates, pharmaceutically acceptable salts or co-crystals thereof as described herein and other components including physiologically/pharmaceutically acceptable carriers and/or excipients.

The term "carrier" refers to a system that does not cause significant irritation to the organism and does not eliminate the biological activity and characteristics of the administered compound and can change the way the drug enters the human body and the distribution of the drug in the body, control the release rate of the drug and deliver the drug to targeted organs. Non-limiting examples of the carrier include microcapsule, microsphere, nanoparticle, liposome, etc.

The term "excipient" refers to a substance that is not a therapeutic agent per se, but is used as a diluent, auxiliary material, binder and/or vehicle added to a pharmaceutical composition, thereby improving the disposal or storage properties thereof, or allowing to or promoting the formation of a compound or a pharmaceutical composition into a unit dosage form for administration. As is known to those skilled in the art, pharmaceutically acceptable excipients can provide various functions and can be described as a wetting agent, a buffer, a suspending agent, a lubricant, an emulsifier, a disintegrant, an absorbent, a preservative, a surfactant, a colorant, a flavouring agent, and a sweetening agent. Examples of pharmaceutically acceptable excipients include, but are not limited to: (1) sugars, such as lactose, glucose and sucrose; (2) starches, such as corn starch and potato starch; (3) cellulose and its derivatives, such as sodium carboxymethyl cellulose, ethyl cellulose, cellulose acetate, hydroxypropyl methylcellulose, hydroxypropyl cellulose, microcrystalline cellulose and croscarmellose (such as croscarmellose sodium); (4) tragacanth powder; (5) malt; (6) gelatine; (7) talc; (8) excipients, such as cocoa butter and suppository wax; (9) oils, such as peanut oil, cottonseed oil, safflower oil, sesame oil, olive oil, corn oil and soybean oil; (10) diols, such as propylene glycol; (11) polyols, such as glycerine, sorbitol, mannitol and polyethylene glycol; (12) esters, such as ethyl oleate and ethyl laurate; (13) agar; (14) buffers, such as magnesium hydroxide and aluminium hydroxide; (15) alginic acid; (16) pyrogen-free water; (17) isotonic saline; (18) Ringer's solution; (19) ethanol; (20) pH buffered solution; (21) polyester, polycarbonate and/or polyanhydride; and (22) other non-toxic compatible substances used in a pharmaceutical preparation.
, mannitol and polyethylene glycol; (12) esters, such as ethyl oleate and ethyl laurate; (13) agar; (14) buffers, such as magnesium hydroxide and aluminum hydroxide; (15) alginic acid; (16) pyrogen-free water; (17) isotonic saline; (18) Ringer's solution; (19) ethanol; (20) pH buffered solution; (21) polyester, polycarbonate and/or polyanhydride; and (22) other non-toxic compatible substances used in a pharmaceutical preparation.

19) ethanol; (20) pH buffered solution; (21) polyester, polycarbonate and/or polyanhydride; and (22) other non-toxic compatible substances used in a pharmaceutical preparation.

The term "stereoisomer" refers to an isomer produced as a result of different spatial arrangement of atoms in molecules, including cis-trans isomers, optical isomers and conformational isomers.

The compounds of the present invention also include tautomers thereof, for example, when the present invention describes the left side compound in which the pyrimidine ring is substituted with OH, the right-side tautomer compound is also included.

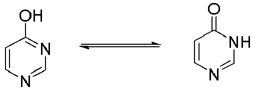

The term "solvate" refers to a substance formed by the compound of the present invention or the salt thereof and a stoichiometric or non-stoichiometric solvent bound by intermolecular non-covalent forces. When the solvent is water, the solvate is a hydrate.

The term "co-crystal" refers to a crystal formed by the combination of active pharmaceutical ingredient (API) and co-crystal former (CCF) under the action of hydrogen bonds or other non-covalent bonds. The pure state of API and CCF are both solid at room temperature, and there is a fixed stoichiometric ratio between various components. The co-crystal is a multi-component crystal, which includes both a binary co-crystal formed between two neutral solids and a multi-element co-crystal formed between a neutral solid and a salt or solvate.

### Detailed Description of Embodiments

The content of the present invention is described in detail by means of the following examples. In examples in which no specific conditions are indicated, experimental methods are carried out under conventional conditions. The listed examples are intended to better illustrate the content of the present invention but should not be construed as limiting the content of the present invention. Non-essential improvements and adjustments made to the embodiments by a person of ordinary skill in the art according to the above Summary of the Invention still fall within the scope of protection of the present invention.

### Detection method

The structure of the compound is determined by nuclear magnetic resonance (NMR) or (and) mass spectrometry (MS). The NMR shift (δ) is given in the unit of 10-6 (ppm). NMR is determined with Bruker Avance III 400 and Bruker Avance 300; the solvents for determination are deuterated dimethyl sulphoxide (DMSO-d6), deuterated chloroform (CDCl3) and deuterated methanol (CD3OD); and the internal standard is tetramethylsilane (TMS);
MS is determined with Agilent 6120B (ESI) and Agilent 6120B (APCI); and
HPLC is determined with Agilent 1260DAD high pressure liquid chromatograph (Zorbax SB-C18 100 × 4.6 mm, 3.5 µM).

For thin layer chromatography (TLC), silica gel plates from Yantai Huanghai HSGF254 or Qingdao GF254 are used. Silica gel plates with a thickness of 0.15 mm-0.20 mm are employed for TLC, while plates with a thickness of 0.4 mm-0.5 mm are used for TLC separation and purification.

For column chromatography, Yantai Huanghai silica gel of 200-300 mesh is typically used as a carrier.

### Intermediate 1: 2-Azaspiro[3.3]heptane-2-sulphonamide

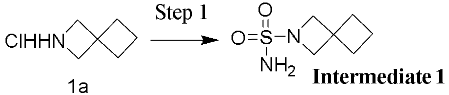

### Step 1:

1a (800 mg, 5.99 mmol), triethylamine (1.82 g, 17.97 mmol) and dichloromethane (10 mL) were added in sequence into a 50 mL reaction flask. After the addition was completed, the mixture was stirred at 0°C for 20 min, a solution of sulphamoyl chloride (692 mg, 5.99 mmol) in dichloromethane (7 mL) was slowly added dropwise and the resulting mixture was stirred and reacted at room temperature for 1 h. The reaction solution was diluted with dichloromethane (50 mL), washed with water (30 mL × 1) and saturated sodium chloride aqueous solution (30 mL × 1) in sequence, the organic layer was dried over anhydrous sodium sulphate and filtered, and the filtrate was concentrated under reduced pressure. The residue was separated and purified by column chromatography (DCM/MeOH = 15/1) to give intermediate 1 (180 mg, yield: 17%).

### Intermediate 2: 2-((3-(2,2-Difluoroethyl)-4-oxo-3,4-dihydroquinazolin-6-yl)oxy)-3,6-difluorobenzonitrile

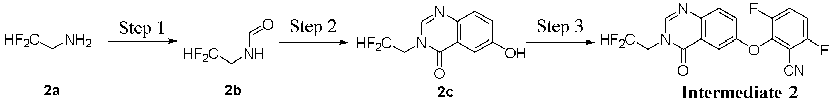

### Step 1:

2a (20 g, 184.46 mmol) was added to ethyl formate (20 mL). The mixture was reacted at 55°C overnight. After the reaction was completed, the reaction solution was cooled to room temperature and concentrated to give residue 2b (3.1 g, yield: 12%).

LCMS m/z = 100.2 [M+H]⁺;
¹H NMR (400 MHz, CDCl₃) δ 8.23 (s, 1H), 6.32 (s, 1H), 6.01 - 5.67 (m, 1H), 3.71-3.60 (m, 2H).

### Step 2:

2-Amino-5-hydroxybenzoic acid (0.5 g, 3.31 mmol) was added to 2b (3.1 mL, 28.4 mmol) in a 25 mL single-necked flask. The mixture was reacted at 150°C for 21 h. After the reaction was completed, the mixture was cooled to room temperature and filtered. The filter cake was washed with ethyl acetate (0.5 mL*2), and then the filter cake was concentrated to give 2c (0.70 g, yield: 94%).
LCMS m/z = 227.2 [M+H]⁺;

### Step 3:

2c (0.70 g, 3.09 mmol) was dissolved in dry N,N-dimethylformamide (6 mL) in a 25 mL single-necked flask. Caesium carbonate (2.01 g, 6.17 mmol) was slowly added in an ice bath. After the addition was completed, the mixture was stirred and reacted at room temperature for 0.5 h. 2,3,6-Trifluorobenzonitrile (0.51 g, 3.20 mmol) was slowly added dropwise in an ice bath. After the addition was completed, the mixture was stirred and reacted at room temperature overnight. After the reaction was completed, ethyl acetate (5 mL) was added to the reaction system to dilute the reaction. Then water (20 mL) was added to quench the reaction, and extraction was performed with ethyl acetate (20 mL*2). The organic phase was washed with water (20 mL*2). The organic phase was concentrated and then purified by column chromatography (petroleum ether/ethyl acetate = 4/1) to give intermediate 2 (1.1 g, yield: 98%).
LCMS m/z = 364.2 [M+H]⁺.

### Intermediate 3: 3,6-Difluoro-2-((3-(2-fluoroethyl)-4-oxo-3,4-dihydroquinazolin-6-yl)oxy)benzonitrile

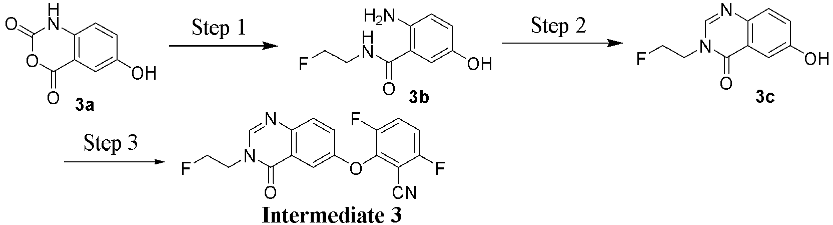

### Step 1:

2-Fluoroethylamine hydrochloride (9.0 g, 90.53 mmol) was dissolved in water (80 mL) in a 250 mL reaction flask. An aqueous sodium hydroxide solution (3.3 g, 82.54 mmol) was added in an ice/water bath. After the mixture was stirred for 30 min, 3a (4.0 g, 22.29 mmol) was added and the resulting mixture was stirred at room temperature for 4 h. After the reaction was completed, concentration was performed under reduced pressure. The residue was dissolved in a mixed solvent of dichloromethane (100 mL) and methanol (50 mL). After filtration, the filtrate was concentrated under reduced pressure. The residue was separated and purified by column chromatography (PE/EA = 1/1) to give 3b (3.8 g, yield: 86%).
LCMS m/z = 199.10 [M+H]⁺;

### Step 2:

3b (1.5 g, 8.58 mmol) was dissolved in triethyl orthoformate (10 mL) in a 20 mL microwave tube. The reaction solution was heated to 185°C and reacted for 2 h. After the reaction was completed, filtration was performed. The resulting filter cake was subjected to rotary evaporation to dryness to give 3c (1.6 g, yield: 89%).
LCMS m/z = 209.10 [M+H]⁺;

### Step 3:

3c (1.5 g, 7.20 mmol) was dissolved in N, N-dimethylformamide (20 mL) in a 100 mL reaction flask. Caesium carbonate (3.52 g, 10.80 mmol) was slowly added in an ice/water bath. After the mixture was stirred at room temperature for 30 min, a solution of 2,3,6-trifluorobenzonitrile (1.36 g, 8.64 mmol) in N,N-dimethylformamide (5 mL) was slowly added dropwise in an ice/water bath. After the addition was completed, the resulting mixture was reacted at room temperature for 2 h. The reaction solution was diluted with ethyl acetate (100 mL), and washed with water (100 mL × 1) and saturated sodium chloride aqueous solution (100 mL × 1) in sequence. The organic layer was dried over anhydrous sodium sulphate, filtered, and concentrated under reduced pressure. The residue was separated and purified by column chromatography (PE/EA = 2/1) to give intermediate 3 (1.35 g, yield: 54%).
LCMS m/z = 346.10 [M+H]⁺;

### Intermediate 4:

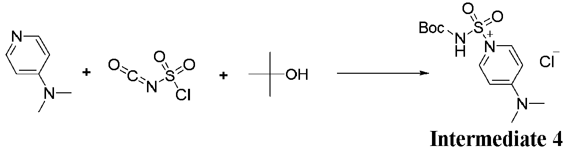

Tert-butyl alcohol (388 ml, 4.24 mol) was dissolved in dichloromethane (3.5 L), and in an ice/water bath, chlorosulphonyl isocyanate (500 g, 3.53 mol) was slowly added dropwise. The reaction system was kept at or below 5°C during the dropwise addition. After the addition was completed, the system was warmed to room temperature and reacted for 1 h. In an ice/water bath, 4-dimethylaminopyridine (863 g, 7.07 mol) was slowly added. The reaction system was kept at or below 5°C during the addition. After the addition was completed, the reaction system was warmed to room temperature and reacted overnight. Upon completion of the reaction, the reaction solution was directly concentrated to one third of the initial volume. Under vigorous stirring, a mixture of saturated sodium bicarbonate aqueous solution (3 L) and ethyl acetate (0.5 L) was added and the resulting mixture was stirred at room temperature for 1 h. Filtration was performed and the filter cake was washed with sodium bicarbonate to remove excess 4-dimethylaminopyridine. A mixture of water (1 L) and ethyl acetate (1 L) was added, and filtration was performed. The filter cake was dried to give **intermediate 4** (1040 g, yield: 97%).
LCMS *m*/*z =* 302.1 [M+1]⁺

¹H NMR (400 MHz, DMSO-d₆) δ 8.46 (d, 2H), 6.98 (d, 2H), 3.23 (s, 6H), 1.26 (s, 9H).

### Example 1

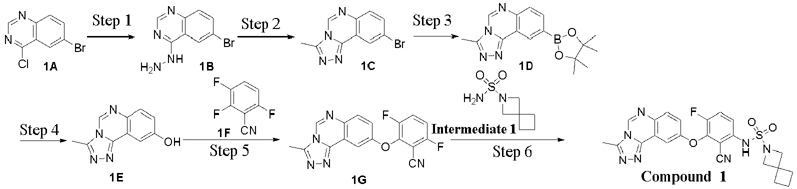

### Step 1:

Compound 1A (5 g, 20.53 mmol) was dissolved in ethanol (30 mL) and acetonitrile (30 mL), hydrazine hydrate (3 mL) was added, and the mixture was stirred at room temperature for 2 h. A significant solid precipitated out. The solid was filtered by suction, washed with ethanol and dried to give compound 1B (4 g, 81%).
LC-MS (ESI): *m*/*z=* 240.1 [M +H]⁺.

### Step 2:

Compound 1B (2 g, 8.36 mmol) was dissolved in trimethyl orthoacetate (20 mL), p-toluenesulphonic acid monohydrate (200 mg, 1.04 mmol) was added, and the mixture was refluxed and stirred overnight. The reaction solution was cooled to room temperature, concentrated, and subjected to rotary evaporation to dryness. The residue was diluted with dichloromethane (40 mL), washed with saturated sodium bicarbonate solution (8 mL × 3) and extracted. The organic phases were combined, dried over anhydrous sodium sulphate and concentrated under reduced pressure to give a crude product, which was separated by column chromatography (petroleum ether : ethyl acetate = 1 : 1 (v/v)) to give compound 1C (1 g, 45%).
LC-MS (ESI): *m*/*z=* 263.1 [M +H]⁺.

### Step 3:

Compound 1C (650 mg, 2.48 mmol), bis(pinacolato)diboron (945 mg, 3.72 mmol) and potassium acetate (486 mg, 4.96 mmol) were sequentially added to the solvent 1,4-dioxane (20 mL), the mixture was purged three times with nitrogen, and [1,1'-bis(diphenylphosphino)ferrocene]palladium(II) dichloride (0.18 g, 0.25 mmol) was added. The resulting mixture was then purged three times with nitrogen, heated to 90°C and reacted for 16 h. The reaction solution was cooled, and ethyl acetate (60 mL) was added to the reaction solution. Then the organic layer was washed with water (20 mL × 2), dried over anhydrous sodium sulphate and concentrated to give compound 1D (600 mg, 78%), which was directly used in the next step.
LC-MS (ESI): *m*/*z* = 229.2 [M-82+H]⁺.

### Step 4:

Compound 1D (600 mg, 1.93 mmol) was dissolved in ethyl acetate (10 mL), the mixture was cooled to 0°C, and 30% hydrogen peroxide aqueous solution (1.2 mL) was slowly added. Then the mixture was stirred at room temperature for 1 h. After the reaction was completed, saturated sodium thiosulphate solution (10 mL) was slowly added to the system to quench the reaction. Then the mixture was extracted with ethyl acetate (10 mL × 3) and subjected to phase separation. The organic phase was dried, concentrated and separated by column chromatography (petroleum ether : ethyl acetate = 1 : 1 (v/v)) to give compound 1E (250 mg, 45%).
LC-MS (ESI): *m*/*z* = 201.1 [M+H]⁺.

### Step 5:

1E (200 mg, 1 mmol) was dissolved in N,N-dimethylformamide (4 mL). Then caesium carbonate (0.65 g, 2 mmol) was added. 1F (0.23 g, 1.5 mmol) was added under stirring at 0°C. After the addition was completed, the system was slowly warmed to room temperature and reacted for 1 h. Upon completion of the reaction as monitored by LCMS, the reaction solution was poured into water (10 mL). Then the mixture was extracted with ethyl acetate (15 mL × 3) and subjected to phase separation. The organic phase was washed with saturated sodium chloride aqueous solution, dried, concentrated and separated by column chromatography (petroleum ether : ethyl acetate = 1 : 1 (v/v)) to give compound 1G (150 mg, 44%).
LC-MS (ESI): *m*/*z* = 338.4 [M+H]⁺.

### Step 6:

Intermediate 1 (100 mg, 0.57 mmol) was dissolved in N,N-dimethylformamide (5 mL), caesium carbonate (286 mg, 0.88 mmol) was added, and the mixture was stirred at room temperature for 30 min. Then 1G (150 mg, 0.44 mmol) was added and the resulting mixture was stirred and reacted at 50°C for 16 h. Upon completion of the reaction as monitored by LCMS, ethyl acetate (50 mL) was added to the reaction solution. Then the organic layer was washed with water (40 mL × 2), dried over anhydrous sodium sulphate and filtered. The filtrate was concentrated under reduced pressure. The residue was separated and purified by column chromatography (dichloromethane : methanol = 10 : 1 (v/v)) to give compound 1 (8 mg, 3.7%).

LC-MS (ESI): *m*/*z* = 494.0 [M+H]⁺.

¹H NMR (400 MHz, DMSO-*d₆*) *δ* 10.38 (s, 1H), 9.50 (s, 1H), 8.14 (d, 1H), 7.98 - 7.70 (m, 2H), 7.65 - 7.35 (m, 2H), 3.81 (s, 4H), 2.54 (s, 3H), 2.08 (t, 4H), 1.77-1.66 (m, 2H).

### Example 2:

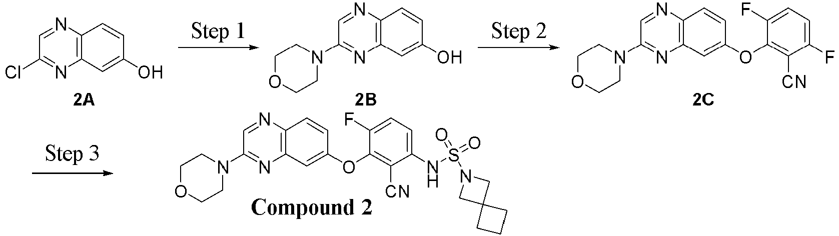

### Step 1:

**2A** (0.93 g, 5.15 mmol) was dissolved in dry N,N-dimethylformamide (10 mL), and at room temperature, morpholine (897.34 mg, 10.30 mmol) and potassium carbonate (2.49 g, 18.11 mmol) were added. After the addition was completed, the mixture was reacted at 100°C overnight. After the reaction was completed, the reaction solution was cooled to room temperature. Water (50 mL) was added to the reaction system to quench the reaction, and the resulting mixture was extracted with ethyl acetate (100 mL*2). The organic phases were combined and concentrated and the residue was purified by column chromatography (dichloromethane / methanol = 97/3, (v/v)) to give **2B** (1.2 g, yield: 100%).
LCMS m/z =232.2 [M+H]⁺;

### Step 2:

**2B** (1.20 g, 5.19 mmol) was dissolved in dry N,N-dimethylformamide (20 mL). Caesium carbonate (4.23 g, 12.98 mmol) was slowly added in an ice bath. After the addition was completed, the mixture was stirred and reacted at room temperature for 0.5 h. 2,3,6- Trifluorobenzonitrile (0.98 g, 6.23 mmol) was slowly added dropwise in an ice bath. After the addition was completed, the mixture was stirred and reacted at room temperature overnight. After the reaction was completed, the reaction solution was filtered and the filtrate was concentrated. Then the residue was purified by column chromatography (petroleum ether/ethyl acetate = 2/1, (v/v)) to give **2C** (0.85 g, yield: 44%).
LCMS m/z =369.1 [M+H]⁺;

### Step 3:

Intermediate 1 (0.32 g, 1.83 mmol) and caesium carbonate (1.59 g, 4.88 mmol) were added to dry N,N-dimethylformamide (15 mL) in a 25 mL single-necked flask, and the mixture was reacted at 50°C for 30 min. At 50°C, **2C** (0.45 g, 1.22 mmol) was added. After the addition was completed, the resulting mixture was stirred and reacted at 85°C for 5 h. After the reaction was completed, the reaction solution was filtered, the filter cake was washed with N,N-dimethylformamide (2 mL), and the filtrate was purified by preparative HPLC to give compound **2** (220 mg, yield: 34%).

LC-MS (ESI): m/z= 525.5 [M+H]⁺.

¹H NMR (400 MHz, DMSO-d₆) δ 10.36 (s, 1H), 8.74 (s, 1H), 7.91 - 7.84 (m, 2H), 7.55-7.51 (dd,1H), 7.29-7.25 (dd, 1H), 6.79 (d, 1H), 3.84 (s, 4H), 3.72 (s, 8H), 2.12-2.08 (t, 4H), 1.77 - 1.70 (m, 2H).

### Example 3:

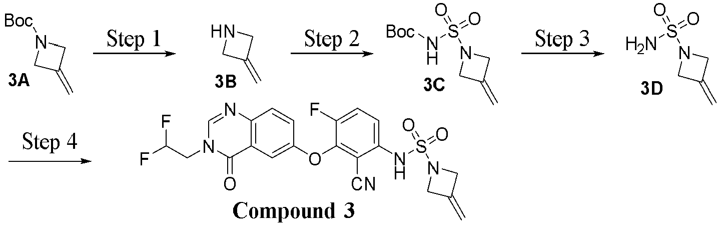

### Step 1:

Crude **3A** (2.00 g, 11.82 mmol) was dissolved in **dichloro**methane (21 mL), and at 0-5°C, trifluoroacetic acid (7 mL) was slowly added dropwise. After the addition was completed, the mixture was reacted at room temperature overnight. After the reaction was completed, the reaction solution was concentrated to give crude product **3B,** which was directly used in the next reaction.

### Step 2:

**3B** (0.82 g, 11.87 mmol) and acetonitrile (20 mL) were added into a 50 mL single-necked flask, and at 0-5°C, triethylamine (6.01 g, 59.35 mmol) and intermediate 4 (4.31 g, 14.24 mmol) were slowly added. The resulting mixture was reacted at room temperature overnight. After the reaction was completed, the reaction solution was concentrated to give crude product **3C,** which was directly used in the next reaction.
LCMS m/z = 193.1 [M+H]⁺;

### Step 3:

Crude product **3C** (2.95 g, 11.88 mmol) was dissolved in dichloromethane (30 mL) in a 50 mL single-necked flask, and at 0-5°C, trifluoroacetic acid (15 mL) was slowly added dropwise. After the addition was completed, the mixture was reacted at room temperature overnight. After the reaction was completed, the reaction solution was concentrated, and the organic phase was concentrated. Then the residue was purified by column chromatography (petroleum ether/ethyl acetate = 1/1) to give **3D** (800 mg, yield: 45%).

¹H NMR (400 MHz, DMSO-d₆) δ 6.98 (s, 2H), 5.03 - 5.02 (m, 2H), 4.33-4.32 (m, 4H).

### Step 4:

**3D** (0.22 g, 1.48 mmol) and caesium carbonate (1.29 g, 3.96 mmol) were added to dry N,N-dimethylformamide (10 mL) in a 25 mL single-necked flask, and the mixture was reacted at 50°C for 30 min. At 50°C, intermediate 2 (0.36 g, 0.99 mmol) was added. After the addition was completed, the resulting mixture was stirred and reacted at 80°C overnight. After the reaction was completed, the reaction solution was filtered, the filter cake was washed with N,N-dimethylformamide (4 mL), and the filtrate was directly purified by preparative HPLC to give compound 3 (52 mg, 10%).

LCMS m/z = 492.1 [M+H]⁺;
¹H NMR (400 MHz, DMSO-d₆) δ 10.54 (s, 1H), 8.34 (s, 1H), 7.90 - 7.86 (m, 1H), 7.83-7.80 (d, 1H), 7.74-7.71 (dd, 1H), 7.58-7.55 (dd, 1H), 7.44-7.43 (d, 1H), 6.51-6.22 (m, 1H), 5.08 - 5.07 (m, 2H), 4.53-4.51 (m, 4H), 4.48 - 4.43 (m, 2H).

### Example 4:

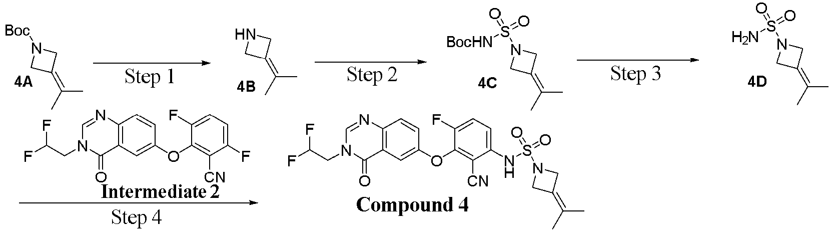

### Step 1:

Compound 4A (300 mg, 1.52 mmol) was dissolved in dichloromethane (10 mL), and in an ice/water bath, trifluoroacetic acid (3 mL) was slowly added dropwise. After the addition was completed, the mixture was reacted at room temperature for 3 h. Upon completion of the reaction, the reaction solution was concentrated to give crude product 4B, which was directly used in the next reaction.
LCMS m/z = 98.20 [M+H]⁺;

### Step 2:

Compound 4B (0.14 g, 1.44 mmol) was dissolved in acetonitrile (20 mL), and then triethylamine (0.44 g, 4.32 mmol) and intermediate 4 (0.65 g, 2.16 mmol) were added. After the addition was completed, the mixture was reacted at room temperature overnight. Upon completion of the reaction, the reaction solution was concentrated, and the resulting crude product was purified by column chromatography (eluent: petroleum ether : ethyl acetate = 1 : 1) to give target compound 4C (0.23 g, yield: 58%).
LCMS m/z = 221.10 [M-56+H]⁺;

### Step 3:

Compound 4C (0.23 g, 1.44 mmol) was dissolved in dichloromethane (10 mL), and in an ice/water bath, trifluoroacetic acid (4 mL) was slowly added dropwise. After the addition was completed, the mixture was reacted at room temperature overnight. Upon completion of the reaction, the reaction solution was concentrated to give crude product 4D, which was directly used in the next reaction.
LCMS m/z = 177.10 [M+H]⁺;

### Step 4:

Compound 4D (0.11 g, 0.62 mmol) and caesium carbonate (0.22 g, 0.66 mmol) were added to N,N-dimethylformamide (10 mL) and the mixture was reacted at 50°C for 30 min. Then intermediate 2 (0.12 g, 0.33 mmol) was added. After the addition was completed, the resulting mixture was stirred at 80°C overnight. Upon completion of the reaction, the reaction solution was filtered, the filtrate was concentrated, and the resulting residue was separated and purified by preparative liquid chromatography to give compound 4 (80 mg, yield: 47%).

LCMS m/z = 520.20 [M+H]⁺;
¹H NMR (400 MHz, DMSO-*d₆*) δ 10.48 (s, 1H), 8.34 (s, 1H), 7.91-7.84 (m, 1H), 7.81 (d, 1H), 7.74-7.69 (m, 1H), 7.58-7.53 (m, 1H), 7.43 (d, 1H), 6.52-6.18 (m, 1H), 4.52-4.44 (m, 6H), 1.48 (s, 6H).

### Example 5:

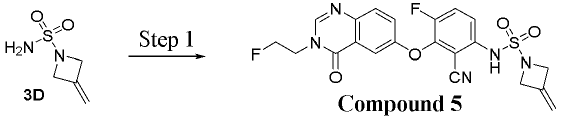

### Step 1:

**3D** (166.70 mg, 1.13 mmol) and caesium carbonate (977.46 mg, 3.00 mmol) were added to dry N,N-dimethylformamide (10 mL) in a 25 mL single-necked flask, and the mixture was reacted at 50°C for 30 min. At 50°C, intermediate 3 (260 mg, 0.75 mmol) was added. After the addition was completed, the resulting mixture was stirred and reacted at 80°C overnight. After the reaction was completed, the reaction solution was filtered, the filter cake was washed with N,N-dimethylformamide (4 mL), and the filtrate was directly purified by preparative HPLC to give compound 5 (130 mg, yield: 36%).

LCMS m/z = 474.2 [M+H]⁺;
¹H NMR (400 MHz, DMSO-*d₆*) δ 8.31 (s, 1H), 7.80-7.77 (d, 1H), 7.69-7.66 (dd, 2H), 7.49-7.45 (dd, 1H), 7.40-7.39 (d, 1H), 5.02 - 5.01 (m, 2H), 4.78-4.75 (t, 1H), 4.66-4.64 (t, 1H), 4.40 (s, 4H), 4.35-4.33 (t, 1H), 4.29-4.26 (t, 1H).

### Example 6:

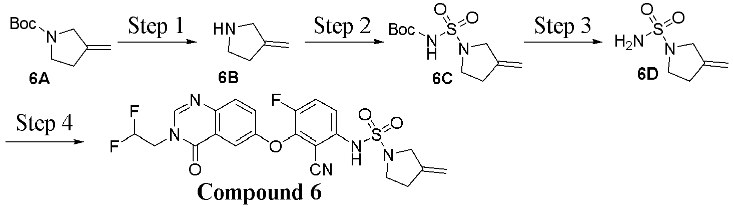

### Step 1:

6A (4.5 g, 24.56 mmol) was dissolved in dichloromethane (60 mL), and at 0-5°C, trifluoroacetic acid (20 mL) was slowly added dropwise. After the addition was completed, the mixture was reacted at room temperature for 2 h. After the reaction was completed, the reaction solution was concentrated to give crude product 6B, which was directly used in the next reaction.

### Step 2:

Crude product 6B and acetonitrile (40 mL) were added into a 100 mL single-necked flask, and at 0-5°C, triethylamine (12.42 g, 122.70 mmol) and intermediate 4 (8.87 g, 29.45 mmol) were slowly added. The resulting mixture was reacted at room temperature overnight. After the reaction was completed, the reaction solution was concentrated, and the residue was purified by column chromatography (petroleum ether / ethyl acetate = 2/1, v/v) to give crude product 6C (2.7 g, yield: 41%).
LCMS m/z = 207.1 [M-55]⁺;

### Step 3:

Crude product 6C (2.70 g, 10.29 mmol) was dissolved in dichloromethane (60 mL) in a 100 mL single-necked flask, and at 0-5°C, trifluoroacetic acid (20 mL) was slowly added dropwise. After the addition was completed, the mixture was reacted at room temperature overnight. After the reaction was completed, the reaction solution was concentrated, and the organic phase was concentrated. Then the residue was purified by column chromatography (petroleum ether/ethyl acetate = 1/1) to give 6D (1.2 g, yield: 71%).

### Step 4:

6D (201.95 mg, 1.24 mmol) and caesium carbonate (1.08 g, 3.32 mmol) were added to dry N,N-dimethylformamide (10 mL) in a 25 mL single-necked flask, and the mixture was reacted at 50°C for 30 min. At 50°C, intermediate 2 (0.30 g, 0.83 mmol) was added. After the addition was completed, the resulting mixture was stirred and reacted at 80°C overnight. After the reaction was completed, the reaction solution was filtered, the filter cake was washed with N,N-dimethylformamide (4 mL), and the filtrate was directly purified by preparative HPLC to give compound 6 (130 mg, yield: 31%).

LCMS m/z = 506.2 [M+H]⁺;
¹H NMR (400 MHz, DMSO-*d₆*) δ 10.34 (s, 1H), 8.34 (s, 1H), 7.89 - 7.80 (m, 2H), 7.73-7.70 (dd, 1H), 7.56-7.52 (dd, 1H), 7.41-7.40 (d, 1H), 6..51-6.22 (m, 1H), 5.00 (s, 2H), 4.52-4.43 (td, 2H), 3.87 (s, 2H), 3.39-3.35 (t, 2H), 2.58-2.55 (t, 2H).

### Example 7:

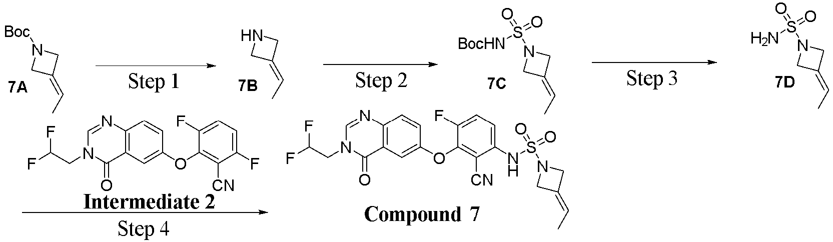

### Step 1:

Compound 7A (1.5 g, 8.19 mmol) was dissolved in dichloromethane (15 mL), and in an ice/water bath, trifluoroacetic acid (5 mL) was slowly added dropwise. After the addition was completed, the mixture was reacted at room temperature for 3 h. Upon completion of the reaction, the reaction solution was concentrated to give crude product 7B, which was directly used in the next reaction.
LCMS m/z = 84.20 [M+H]⁺;

### Step 2:

Compound 7B (0.68 g, 8.18 mmol) was dissolved in acetonitrile (20 mL), and then triethylamine (2.00 g, 19.76 mmol) and intermediate 4 (3.71 g, 12.27 mmol) were added. After the addition was completed, the mixture was reacted at room temperature overnight. Upon completion of the reaction, the reaction solution was concentrated, and the resulting crude product was purified by column chromatography (eluent: petroleum ether : ethyl acetate = 1 : 1) to give target compound 7C (1.0 g, yield: 47%).
LCMS m/z = 207.00 [M-56+H]⁺;

### Step 3:

Compound 7C (1.0 g, 3.81 mmol) was dissolved in dichloromethane (10 mL), and in an ice/water bath, trifluoroacetic acid (4 mL) was slowly added dropwise. After the addition was completed, the mixture was reacted at room temperature overnight. Upon completion of the reaction, the reaction solution was concentrated to give crude product 7D, which was directly used in the next reaction.
LCMS m/z = 163.10 [M+H]⁺;

### Step 4:

Compound 7D (0.46 g, 1.65 mmol) and caesium carbonate (1.08 g, 3.30 mmol) were added to N,N-dimethylformamide (15 mL) and the mixture was reacted at 50°C for 30 min. Then intermediate 2 (0.4 g, 1.10 mmol) was added. After the addition was completed, the resulting mixture was stirred at 80°C overnight. Upon completion of the reaction, the reaction solution was filtered, the filtrate was concentrated, and the resulting residue was separated and purified by preparative liquid chromatography to give compound 7 (100 mg, yield: 20%).

LCMS m/z = 506.20 [M+H]⁺;
¹H NMR (400 MHz, DMSO-*d6*) δ 10.50 (s, 1H), 8.34 (s, 1H), 7.91-7.84 (m, 1H), 7.81 (d, 1H), 7.74-7.69 (m, 1H), 7.58-7.53 (m, 1H), 7.43 (d, 1H), 6.52-6.18 (m, 1H), 5.45-5.36 (m, 1H), 4.53-4.42 (m, 6H), 1.51-1.45 (m, 3H).

### Example 8:

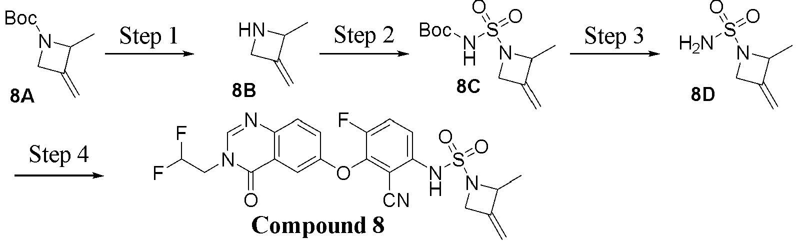

### Step 1:

**8A** (1.00 g, 5.46 mmol, synthesized with reference to WO 2021/108483, 2021, A1) was dissolved in **dichloromethane** (15 mL), and at 0-5°C, trifluoroacetic acid (5 mL) was slowly added dropwise. After the addition was completed, the mixture was reacted at room temperature overnight. After the reaction was completed, the reaction solution was concentrated to give crude product **8B,** which was directly used in the next reaction.

### Step 2:

Crude product **8B** and acetonitrile (10 mL) were added into a 50 mL single-necked flask, and at 0-5°C, triethylamine (2.74 g, 27.05 mmol) and intermediate 4 (1.96 g, 6.49 mmol) were slowly added. The resulting mixture was reacted at room temperature overnight. After the reaction was completed, the reaction solution was concentrated, and the residue was purified by column chromatography (petroleum ether / ethyl acetate = 2/1, v/v) to give crude product **8C** (0.50 g, yield: 35%).
LCMS m/z = 207.1 [M-55]⁺;

### Step 3:

Crude product **8C** (0.50 g, 1.91 mmol) was dissolved in dichloromethane (12 mL) in a 50 mL single-necked flask, and at 0-5°C, trifluoroacetic acid (4 mL) was slowly added dropwise. After the addition was completed, the mixture was reacted at room temperature overnight. After the reaction was completed, the reaction solution was concentrated, and the organic phase was concentrated. Then the residue was purified by column chromatography (petroleum ether/ethyl acetate = 1/1) to give **8D** (300 mg, yield: 97%).
LCMS m/z = 163.1 [M+H]⁺;

### Step 4:

**8D** (82.73 mg, 0.51 mmol) and caesium carbonate (0.55 g, 1.70 mmol) were added to dry N,N-dimethylformamide (10 mL) in a 25 mL single-necked flask, and the mixture was reacted at 50°C for 10 min. At 50°C, intermediate 2 (125 mg, 0.34 mmol) was added. After the addition was completed, the resulting mixture was stirred and reacted at 80°C overnight. After the reaction was completed, the reaction solution was filtered, the filter cake was washed with N,N-dimethylformamide (4 mL), and the filtrate was directly purified by preparative HPLC to give compound 8
LCMS m/z = 506.1 [M+H]⁺;

### Example 9:

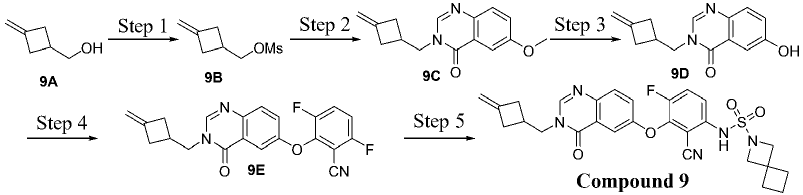

### Step 1:

9A (1.00 g, 10.19 mmol) and dichloromethane (20 mL) were added into a 100 mL single-necked flask, and then triethylamine (2.06 g, 20.38 mmol) and methylsulphonyl chloride (1.40 g, 12.23 mmol) were added. The resulting mixture was reacted at room temperature for 30 min. After the reaction was completed, the reaction solution was concentrated, and the residue was purified by column chromatography (petroleum ether / ethyl acetate = 10/1, v/v) to give 9B (1.80 g, yield: 100%).

### Step 2:

9B (1.80 g, 10.22 mmol), 6-methoxy-4(1H)-quinazolinone (1.80 g, 10.22 mmol), potassium carbonate (2.83 g, 20.44 mmol) and acetonitrile (40 mL) were added into a 100 mL single-necked flask. After the addition was completed, the mixture was reacted at 80°C overnight. After the reaction was completed, the reaction solution was concentrated, and the residue was purified by column chromatography (petroleum ether / ethyl acetate = 1/1, v/v) to give 9C (1.3 g, yield: 49%).
LCMS m/z = 257.1 [M+H]+;

### Step 3:

9C (1.30 g, 5.07 mmol) and N,N-dimethylformamide (20 mL) were added into a 100 mL single-necked flask, and then sodium ethanethiolate (1.49 g, 17.75 mmol) was added. After the addition was completed, the system was stirred and reacted at 135°C under nitrogen atmosphere for 12 h. After the reaction was completed, the reaction solution was concentrated, dilute hydrochloric acid was added to adjust the system to acidic pH, and the system was then adjusted with saturated sodium bicarbonate solution to alkaline pH and concentrated to give a residue, which was purified by column chromatography (petroleum ether / ethyl acetate = 1/2, v/v) to give 9D (1.20 g, yield: 97%).
LCMS m/z =243.1 [M+H]⁺;

### Step 4:

9D (1.20 g, 4.95 mmol) was dissolved in dry N,N-dimethylformamide (30 mL) in a 100 mL single-necked flask. Caesium carbonate (3.23 g, 9.90 mmol) was slowly added at 0-5°C. After the addition was completed, the mixture was stirred and reacted at room temperature for 0.5 h. 2,3,6- Trifluorobenzonitrile (0.86 g, 5.45 mmol) was slowly added dropwise in an ice bath. After the addition was completed, the mixture was stirred and reacted at room temperature overnight. After the reaction was completed, the reaction solution was filtered and the filtrate was concentrated. Then the residue was purified by column chromatography (petroleum ether/ethyl acetate = 1/1) to give 9E (1.70 g, yield: 90%).
LCMS m/z =380.2 [M+H]+;

### Step 5:

Intermediate 1 (0.21 g, 1.19 mmol) and caesium carbonate (1.03 g, 3.16 mmol) were added to dry N,N-dimethylformamide (10 mL) in a 25 mL single-necked flask, and the mixture was reacted at 50°C for 30 min. At 50°C, 9E (0.30 g, 0.79 mmol) was added. After the addition was completed, the resulting mixture was stirred and reacted at 80°C overnight. After the reaction was completed, the reaction solution was filtered and the filtrate was purified by preparative HPLC to give compound 9 (240 mg, yield: 56%).

LCMS m/z = 536.3 [M+H]⁺;
¹H NMR (400 MHz, CDCl₃) δ 8.29 (s, 1H), 7.80-7.77 (d, 1H), 7.55 - 7.49 (m, 3H), 7.39-7.35 (t, 1H), 4.77 (s, 2H), 4.08-4.06 (d, 2H), 3.90 (s, 4H), 2.81 - 2.76 (m, 3H), 2.46-2.43 (d, 2H), 2.13-2.09 (t, 4H), 1.81- 1.73 (m, 2H).

### Example 10:

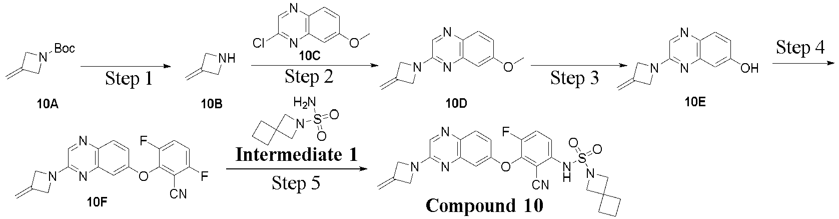

### Step 1:

Compound 10A (2.0 g, 11.82 mmol) was dissolved in dichloromethane (15 mL), and in an ice/water bath, trifluoroacetic acid (5 mL) was slowly added dropwise. After the addition was completed, the mixture was reacted at room temperature for 3 h. Upon completion of the reaction, the reaction solution was concentrated to give crude product 10B, which was directly used in the next reaction.

### Step 2:

Compound 10B (1.41 g, 7.70 mmol) and 10C (1.00 g, 5.13 mmol) were dissolved in N,N-dimethylformamide (15 mL) and then potassium carbonate (2.13 g, 15.39 mmol) was added. After the addition was completed, the mixture was stirred at 100°C overnight. After the reaction was completed, 40 mL of water was added to the reaction solution, and the mixture was extracted with ethyl acetate (30 mL × 3). The organic layers were combined, washed with saturated sodium chloride aqueous solution (50 mL), dried over anhydrous sodium sulphate, filtered, and concentrated. The residue was separated and purified by silica gel column chromatography (petroleum ether:ethyl acetate (v/v) = 5 : 1) to give compound 10D (1.0 g, yield :85%).
LCMS m/z = 228.10 [M+H]⁺;

### Step 3:

Compound 10D (1.0 g, 4.40 mmol) was dissolved in N,N-dimethylformamide (20 mL), and then sodium ethanethiolate (1.48 g, 17.60 mmol) was added. The reaction solution was purged three times with nitrogen, heated to 130°C and reacted for 12 hours. Upon completion of the reaction, the reaction solution was filtered and the resulting filtrate was concentrated to give crude product 10E, which was directly used in the next reaction.
LCMS m/z = 214.10 [M+H]⁺;

### Step 4:

Compound 10E (1.0 g, 4.69 mmol) was dissolved in N, N-dimethylformamide (20 mL). Caesium carbonate (3.0 g, 9.21 mmol) was slowly added in an ice/water bath. After the addition was completed, the mixture was stirred at room temperature for 30 min, and then a solution of 2,3,6-trifluorobenzonitrile (1.11 g, 7.07 mmol) in N,N-dimethylformamide (5 mL) was slowly added dropwise in an ice/water bath. After the addition was completed, the resulting mixture was reacted at room temperature for 2 h. Upon completion of the reaction, the reaction solution was diluted with ethyl acetate (50 mL), and washed with water (50 mL × 1) and saturated sodium chloride aqueous solution (50 mL × 1) in sequence. The organic layer was dried over anhydrous sodium sulphate, filtered, and concentrated under reduced pressure. The residue was separated and purified by column chromatography (petroleum ether : ethyl acetate (v/v) = 1 : 1) to give 10F (0.70 g, yield: 42%).
LCMS m/z = 351.10 [M+H]⁺;

### Step 5:

Intermediate 1 (0.23 g, 1.31 mmol) and caesium carbonate (0.56 g, 1.72 mmol) were added to N,N-dimethylformamide (15 mL) and the mixture was reacted at 50°C for 30 min. Then compound 10F (0.3 g, 0.86 mmol) was added. After the addition was completed, the resulting mixture was stirred at 80°C overnight. Upon completion of the reaction, the reaction solution was filtered, the filtrate was concentrated, and the resulting residue was separated and purified by preparative liquid chromatography to give compound 10 (100 mg, yield: 23%).

LCMS m/z = 507.10 [M+H]⁺;
¹H NMR (400 MHz, DMSO-*d6*) δ 10.34 (s, 1H), 8.31 (s, 1H), 7.93-7.82 (m, 2H), 7.60-7.48 (m, 1H), 7.31-7.23 (m, 1H), 6.83 (d, 1H), 5.16-5.10 (m, 2H), 4.84-4.76 (m, 4H), 3.84 (s, 4H), 2.15-2.05 (m, 4H), 1.80-1.69 (m, 2H).

### Example 11:

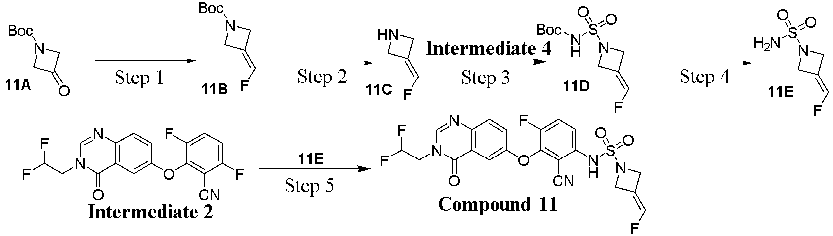

### Step 1:

(Fluoromethyl)triphenylphosphonium tetrafluoroborate (19 g, 49.72 mmol) was dissolved in dry tetrahydrofuran (380 mL), and at -78°C, sodium bis(trimethylsilyl)amide (25 mL, 49.72 mmol, 2M in THF) was slowly added dropwise. After the addition was completed, the mixture was reacted at -78°C for 1 h. At -78°C, a solution of compound 11A (8.5 g, 49.72 mmol) in tetrahydrofuran (20 mL) was slowly added dropwise. After the addition was completed, the mixture was reacted at room temperature overnight. After the reaction was completed as monitored by TLC (petroleum ether : ethyl acetate = 5 : 1 (v/v)), saturated ammonium chloride aqueous solution was added to quench the reaction. The resulting mixture was extracted with ethyl acetate (500 mL × 2), and the organic phases were combined, washed with saturated sodium chloride aqueous solution (100 mL × 1), dried over anhydrous sodium sulphate and filtered. The filtrate was concentrated under reduced pressure and purified by column chromatography (petroleum ether : ethyl acetate = 5 : 1 (v/v)) to give compound 11B (4.5 g, yield: 48%).

LCMS *m*/*z =* 132.2 [M+1]⁺
¹H NMR (400 MHz, CDCl₃) δ 6.53 - 6.27 (m, 1H), 4.48 - 4.39 (m, 2H), 4.36 (dt, 2H), 1.34 (s, 9H).

¹⁹F NMR (376 MHz, CDCl₃) δ -137.81 (s).

### Step 2:

Compound 11B (1.0 g, 5.34 mmol) was dissolved in dichloromethane (20 mL) and trifluoroacetic acid (4 mL) was added dropwise. After the addition was completed, the mixture was reacted at room temperature for 2 h. After the reaction was completed, the reaction solution was concentrated to give crude product 11C, which was directly used in the next reaction.
LCMS *m*/*z =* 88.2 [M+1]⁺

### Step 3:

Compound 11C (466 mg, 5.34 mmol) was dissolved in acetonitrile (20 mL), and in an ice bath, triethylamine (3.7 mL, 26.7 mmol) was slowly added dropwise. Intermediate 4 (2.42 g, 8.01 mmol) was added. After the addition was completed, the mixture was reacted at room temperature overnight. After the reaction was completed as monitored by TLC (petroleum ether : ethyl acetate = 1 : 1 (v/v)), saturated sodium bicarbonate aqueous solution was added. The resulting mixture was extracted with ethyl acetate (100 mL × 2), dried over anhydrous sodium sulphate and filtered. The filtrate was concentrated under reduced pressure and purified by column chromatography (petroleum ether : ethyl acetate = 2 : 1 (v/v)) to give compound 11D (530 mg, yield: 37%).
LCMS *m*/*z* = 265.2 [M-1]⁻

### Step 4:

Compound 11D (530 mg, 1.99 mmol) was dissolved in dichloromethane (20 mL) and trifluoroacetic acid (4 mL) was added dropwise. After the addition was completed, the mixture was reacted at room temperature for 2 h. After the reaction was completed, the reaction solution was concentrated and then purified by column chromatography (petroleum ether : ethyl acetate = 1 : 1 (v/v)) to give compound 11E (280 mg, yield: 85%).
LCMS *m*/*z =* 167.2 [M+1]⁺

### Step 5:

Intermediate 2 (580 mg, 1.60 mmol) was dissolved in dry N,N dimethylformamide (20 mL), and caesium carbonate (1.04 g, 3.20 mmol) and compound 11E (280 mg, 1.68 mmol) were added in sequence. After the addition was completed, the resulting mixture was reacted at 80°C for 6 h. After the reaction was completed as monitored by TLC (petroleum ether : ethyl acetate = 1 : 2 (v/v)), saturated ammonium chloride aqueous solution was added. The resulting mixture was extracted with ethyl acetate (100 mL × 2), and the organic phases were combined, washed with saturated sodium chloride aqueous solution (100 mL × 1), dried over anhydrous sodium sulphate and filtered. The filtrate was concentrated under reduced pressure and purified by column chromatography (dichloromethane : methanol = 50 : 1 (v/v)) to give compound 11 (148 mg, yield: 18%).
LCMS *m*/*z* = 510.3 [M+1]⁺

### Example 12:

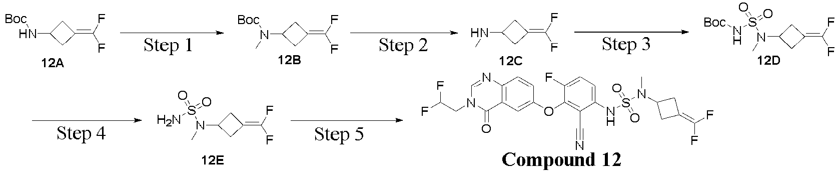

### Step 1:

Compound 12A (450 mg, 2.05 mmol) was dissolved in N, N-dimethylformamide (10 mL), and in an ice/water bath, sodium hydride (120 mg, 3.07 mmol) was slowly added. The mixture was stirred for 30 min and then iodomethane (440 mg, 3.07 mmol) was added. After the addition was completed, the resulting mixture was stirred at room temperature for 3 h. Upon completion of the reaction, 20 mL of water was added to quench the reaction. The mixture was extracted with ethyl acetate (30 mL × 3), and the organic phase was washed with saturated sodium chloride aqueous solution (50 mL), dried over anhydrous sodium sulphate, filtered and concentrated. The residue was separated and purified by silica gel column chromatography (petroleum ether : ethyl acetate (v/v) = 10 : 1) to give compound 12B (350 mg, yield: 73%).

### Step 2:

Compound 12B (350 mg, 1.50 mmol) was dissolved in dichloromethane (15 mL), and in an ice/water bath, trifluoroacetic acid (5 mL) was slowly added dropwise. After the addition was completed, the mixture was reacted at room temperature for 3 h. Upon completion of the reaction, the reaction solution was concentrated to give crude product 12C, which was directly used in the next reaction.
LCMS *m*/*z* = 134.10 [M+H]⁺;

### Step 3:

Compound 12C (200 mg, 1.50 mmol) was dissolved in acetonitrile (10 mL), and at 0-5°C, triethylamine (460 mg, 4.50 mmol) and intermediate 4 (680 mg, 2.25 mmol) were slowly added. After the addition was completed, the mixture was reacted at room temperature overnight. Upon completion of the reaction, the reaction solution was concentrated and the crude product was separated and purified by silica gel column chromatography (petroleum ether : ethyl acetate (v/v) = 1 : 1) to give compound 12D (150 mg, yield: 32%).
LCMS *m*/*z* = 257.20 [M-56+H]⁺;

### Step 4:

Compound 12D (150 mg, 0.48 mmol) was dissolved in dichloromethane (10 mL), and in an ice/water bath, trifluoroacetic acid (3 mL) was slowly added dropwise. After the addition was completed, the mixture was reacted at room temperature for 3 h. Upon completion of the reaction, the reaction solution was concentrated to give crude product 12E, which was directly used in the next reaction.
LCMS *m*/*z* = 213.10 [M+H]⁺;

### Step 5:

Compound 12E (100 mg, 0.47 mmol) and caesium carbonate (270 mg, 0.84 mmol) were added to N,N-dimethylformamide (10 mL) and the mixture was reacted at 50°C for 30 min. Then intermediate 2 (100 mg, 0.28 mmol) was added. After the addition was completed, the resulting mixture was stirred at 80°C overnight. Upon completion of the reaction, the reaction solution was filtered, the filtrate was concentrated, and the resulting residue was separated and purified by preparative liquid chromatography to give compound 12 (30 mg, yield: 20%).
LCMS *m*/*z* = 556.50 [M+H]⁺;

### Example 13:

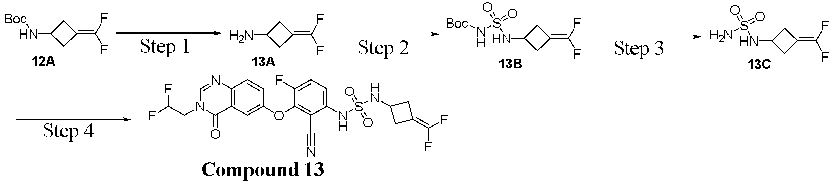

### Step 1:

Compound 12A (250 mg, 1.14 mmol) was dissolved in dichloromethane (15 mL), and in an ice/water bath, trifluoroacetic acid (5 mL) was slowly added dropwise. After the addition was completed, the mixture was reacted at room temperature for 3 h. Upon completion of the reaction, the reaction solution was concentrated to give crude product 13A, which was directly used in the next reaction.
LCMS *m*/*z* = 120.10 [M+H]⁺;

### Step 2:

Compound 13A (140 mg, 1.18 mmol) was dissolved in acetonitrile (10 mL), and at 0-5°C, triethylamine (360 mg, 3.54 mmol) and intermediate 4 (540 mg, 1.77 mmol) were slowly added. After the addition was completed, the mixture was reacted at room temperature overnight. Upon completion of the reaction, the reaction solution was concentrated and the crude product was separated and purified by silica gel column chromatography (petroleum ether : ethyl acetate (v/v) = 1 : 1) to give compound 13B (200 mg, yield: 35%).
LCMS *m*/*z* = 243.40 [M-56+H]⁺;

### Step 4:

Compound 13B (200 mg, 0.67 mmol) was dissolved in dichloromethane (10 mL), and in an ice/water bath, trifluoroacetic acid (3 mL) was slowly added dropwise. After the addition was completed, the mixture was reacted at room temperature for 3 h. Upon completion of the reaction, the reaction solution was concentrated to give crude product 13C, which was directly used in the next reaction.
LCMS *m*/*z* = 199.00 [M+H]⁺;

### Step 5:

Compound 13C (130 mg, 0.67 mmol) and caesium carbonate (300 mg, 0.92 mmol) were added to N,N-dimethylformamide (10 mL) and the mixture was reacted at 50°C for 30 min. Then intermediate 2 (100 mg, 0.28 mmol) was added. After the addition was completed, the resulting mixture was stirred at 80°C overnight. Upon completion of the reaction, the reaction solution was filtered, the filtrate was concentrated, and the resulting residue was separated and purified by preparative liquid chromatography to give compound 13 (25 mg, yield: 17%).
LCMS *m*/*z* = 542.00 [M+H]⁺;

### Example 14:

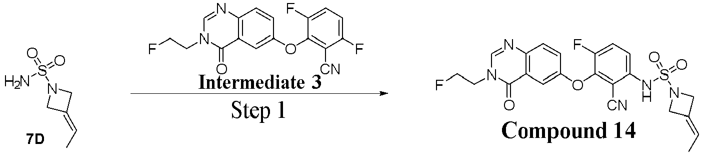

### Step 1:

Compound 7D (0.20 g, 1.23 mmol) and caesium carbonate (0.49 g, 1.51 mmol) were added to N,N-dimethylformamide (15 mL) and the mixture was reacted at 50°C for 30 min. Then intermediate 3 (0.25 g, 0.72 mmol) was added. After the addition was completed, the resulting mixture was stirred at 80°C overnight. Upon completion of the reaction, the reaction solution was filtered, the filtrate was concentrated, and the resulting residue was separated and purified by preparative liquid chromatography to give compound 14 (65 mg, yield: 18%).

LCMS m/z = 488.10 [M+H]⁺;
¹H NMR (400 MHz, DMSO-*d₆*) δ 10.51 (s, 1H), 8.33 (s, 1H), 7.91-7.84 (m, 1H), 7.81 (d, 1H), 7.74-7.69 (m, 1H), 7.58-7.53 (m, 1H), 7.41 (d, 1H), 5.46-5.34 (m, 1H), 4.79-4.63 (m, 2H), 4.47 (d, 4H), 4.35-4.32 (m, 2H), 1.48 (d, 3H).

### Examples 15 and 16:

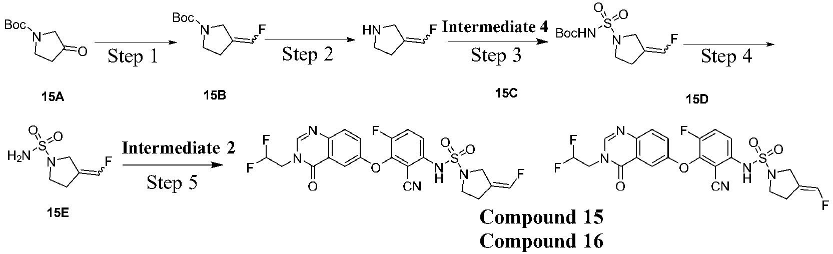

### Step 1:

(Fluoromethyl)triphenylphosphonium tetrafluoroborate (13 g, 34.02 mmol) was dissolved in dry tetrahydrofuran (260 mL), and at -78°C, sodium bis(trimethylsilyl)amide (17 mL, 34.02 mmol, 2M in THF) was slowly added dropwise. After the addition was completed, the mixture was reacted at -78°C for 1 h. At -78°C, a solution of compound 15A (6.30 g, 34.02 mmol) in tetrahydrofuran (15 mL) was slowly added dropwise. After the addition was completed, the mixture was reacted at room temperature overnight. After the reaction was completed as monitored by TLC (petroleum ether : ethyl acetate = 5 : 1 (v/v)), saturated ammonium chloride aqueous solution was added to quench the reaction. The resulting mixture was extracted with ethyl acetate (300 mL × 2), and the organic phases were combined, washed with saturated sodium chloride aqueous solution (100 mL × 1), dried over anhydrous sodium sulphate and filtered. The filtrate was concentrated under reduced pressure and purified by column chromatography (petroleum ether:ethyl acetate = 5 : 1 (v/v)) to give compound 15B (3.06 g, yield: 45%).
LCMS *m*/*z =* 146.2 [M+1-56]⁺

### Step 2:

Compound 15B (860 mg, 4.27 mmol) was dissolved in dichloromethane (20 mL) and trifluoroacetic acid (4 mL) was added dropwise. After the addition was completed, the mixture was reacted at room temperature for 2 h. After the reaction was completed, the reaction solution was concentrated to give crude product 15C, which was directly used in the next reaction.
LCMS *m*/*z =* 102.2 [M+1]⁺

### Step 3:

Compound 15C (432 mg, 4.27 mmol) was dissolved in acetonitrile (20 mL), and in an ice bath, triethylamine (2.7 mL, 19.2 mmol) was slowly added dropwise. Intermediate 4 (1.94 g, 6.41 mmol) was added. After the addition was completed, the mixture was reacted at room temperature overnight. After the reaction was completed as monitored by TLC (petroleum ether: ethyl acetate = 1 : 1 (v/v)), saturated sodium bicarbonate aqueous solution was added. The resulting mixture was extracted with ethyl acetate (100 mL × 2), dried over anhydrous sodium sulphate and filtered. The filtrate was concentrated under reduced pressure to give crude product 15D, which was directly used in the next reaction.
LCMS *m*/*z* = 281.2 [M-1]⁻

### Step 4:

Compound 15D (1.2 g, 4.27 mmol) was dissolved in dichloromethane (20 mL) and trifluoroacetic acid (4 mL) was added dropwise. After the addition was completed, the mixture was reacted at room temperature for 2 h. After the reaction was completed, the reaction solution was concentrated and then purified by column chromatography (petroleum ether : ethyl acetate = 1 : 1 (v/v)) to give compound 15E (420 mg, yield: 55%).
LCMS *m*/*z =* 181.2 [M+1]⁺

### Step 5:

Intermediate 2 (627 mg, 1.73 mmol) was dissolved in dry N,N dimethylformamide (20 mL), and caesium carbonate (1.97 g, 6.04 mmol) and compound 15E (420 mg, 2.33 mmol) were added in sequence. After the addition was completed, the resulting mixture was reacted at 80°C for 6 h. After the reaction was completed as monitored by TLC (petroleum ether : ethyl acetate = 1 : 2 (v/v)), saturated ammonium chloride aqueous solution was added. The resulting mixture was extracted with ethyl acetate (100 mL × 2), and the organic phases were combined, washed with saturated sodium chloride aqueous solution (100 mL × 1), dried over anhydrous sodium sulphate and filtered. The filtrate was concentrated under reduced pressure and purified by column chromatography (eluent: dichloromethane: methanol = 65 : 1 (v/v)) to give the target compound. The target compound was subjected to chiral preparative SFC to give P1 (125 mg, yield: 17%, retention time: 11.08 min, assigned as compound 15), and P2 (145 mg, yield: 20%, retention time: 19.00 min, assigned as compound 16).

Preparative SFC method: 1. Instrument: SFC Prep 150 AP; chromatographic column: TORUS 2-PIC (19 mm × 250 mm) 2. The sample was dissolved in methanol, and filtered with a 0.45 µm filter to prepare a sample solution. 3. Preparative chromatography conditions: a. composition of mobile phases A and B: mobile phase A: CO₂; mobile phase B: methanol. b. isocratic elution, mobile phase B: 40%. c. flow rate: 46 ml/min. After separation, the fraction was dried in a water bath at 45°C by a rotary evaporator to give a product. Then the solvent was dried at -80°C with a lyophiliser to give compounds 15 and 16.

Compound 15, LCMS m/z = 524.2 [M+H]+.

¹H NMR (400 MHZ, DMSO-*d₆*) δ 10.41 (s, 1H), 8.34 (s, 1H), 7.87 (t, 1H), 7.83 - 7.78 (m, 1H), 7.72 (m, 1H), 7.54 (dd, 1H), 7.42 (d, 1H), 7.04 - 6.79 (m, 1H), 6.51 - 6.21 (m, 1H), 4.47 (m, 2H), 3.88 (s, 2H), 3.40 (t, 2H), 2.63 (t, 2H).

Compound 16, LCMS m/z = 524.2 [M+H]+.

¹H NMR (400 MHZ, DMSO-*d₆*) δ 10.39 (s, 1H), 8.34 (s, 1H), 7.90 - 7.83 (m, 1H), 7.81 (d, 1H), 7.71 (m, 1H), 7.54 (m, 1H), 7.42 (d, 1H), 6.84 (m, 1H), 6.36 (m, 1H), 4.47 (m, 2H), 4.00 (s, 2H), 3.37 (m, 2H), 2.55 - 2.51 (m, 2H).

### Example 17:

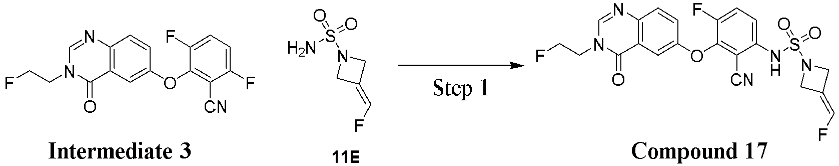

### Step 1:

Intermediate 3 (400 mg, 1.10 mmol) was dissolved in dry N,N dimethylformamide (20 mL), and caesium carbonate (1.26 g, 3.85 mmol) and compound 11E (247 mg, 1.49 mmol) were added in sequence. After the addition was completed, the resulting mixture was reacted at 80°C for 6 h. After the reaction was completed as monitored by TLC (petroleum ether : ethyl acetate = 1 : 2 (v/v)), saturated ammonium chloride aqueous solution was added. The resulting mixture was extracted with ethyl acetate (100 mL × 2), and the organic phases were combined, washed with saturated sodium chloride aqueous solution (100 mL × 1), dried over anhydrous sodium sulphate and filtered. The filtrate was concentrated under reduced pressure and purified by column chromatography (dichloromethane : methanol = 55 : 1 (v/v)) to give compound 17 (142 mg, yield: 26%).

LCMS *m*/*z* = 492.0 [M+1]⁺
¹H NMR (400 MHZ, DMSO-*d₆*) δ 8.33 (s, 1H), 7.88 (dd, 1H), 7.80 (d, 1H), 7.71 (dd, 1H), 7.57 (dd, 1H), 7.43 (d, 1H), 7.03 - 6.99 (m, 0.5H), 6.83 - 6.78 (m, 0.5H), 4.77 (t, 1H), 4.64 (t, 1H), 4.62 - 4.57 (m, 2H), 4.56 - 4.49 (m, 2H), 4.34 (t, 1H), 4.28 (t, 1H).

### Example 18:

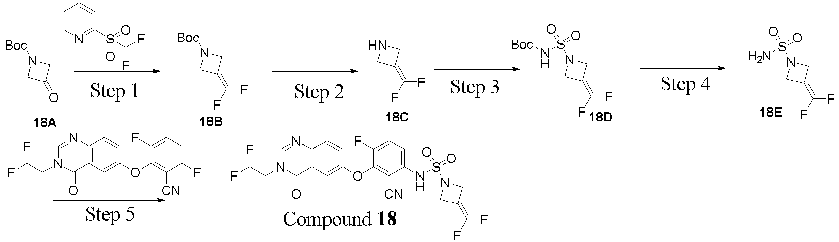

### Step 1:

2-Difluoromethanesulphonylpyridine (5.08 g, 26.29 mmol) and 18A (5.0 g, 29.21 mmol) were dissolved in dry DMF (50 mL), and at -45°C, potassium tert-butoxide (4.92 g, 43.81 mmol, dissolved in 15 mL of DMF) was slowly added dropwise. After the addition was completed, the mixture was reacted at room temperature overnight. After the reaction was completed as monitored by TLC (petroleum ether: ethyl acetate = 5 : 1 (v/v)), saturated ammonium chloride aqueous solution (100 mL) was added to quench the reaction. 3 M HCl (14 mL) was added and the resulting mixture was stirred for another 3 h. Then ethyl acetate (200 mL) was added and the mixture was stirred for another one hour. The reaction system was then left to stand for 48 h and subjected to phase separation. The organic phase was washed with saturated sodium chloride aqueous solution (100 mL × 1), dried over anhydrous sodium sulphate and filtered. The filtrate was concentrated under reduced pressure and then purified by column chromatography (petroleum ether : ethyl acetate = 10 : 1 (v/v)) to give compound 18B (0.9 g, yield: 15%).

¹H NMR (400 MHz, CDCl₃) δ 4.48 (t, 4H), 1.45 (s, 9H).

### Step 2:

Compound 18B (0.9 g, 4.39 mmol) was dissolved in dichloromethane (10 mL) and trifluoroacetic acid (3 mL) was added dropwise. After the addition was completed, the mixture was reacted at room temperature for 2 h. After the reaction was completed, the reaction solution was concentrated to give crude product 18C, which was directly used in the next reaction.
LCMS *m*/*z =* 106.1 [M+1]⁺

### Step 3:

Compound 18C (1.1 g, 5.02 mmol) was dissolved in acetonitrile (20 mL), and in an ice bath, triethylamine (1.52 g, 26.7 mmol) was slowly added dropwise. Intermediate 4 (1.67 g, 5.52 mmol) was added. After the addition was completed, the mixture was reacted at room temperature overnight. After the reaction was completed as monitored by TLC (petroleum ether : ethyl acetate = 1 : 1 (v/v)), saturated sodium bicarbonate aqueous solution was added. The resulting mixture was extracted with ethyl acetate (100 mL × 2), dried over anhydrous sodium sulphate and filtered. The filtrate was concentrated under reduced pressure and purified by column chromatography (petroleum ether : ethyl acetate = 2 : 1 (v/v)) to give compound 18D (350 mg, yield: 24.5%).

### Step 4:

Compound 18D (350 mg, 1.23 mmol) was dissolved in dichloromethane (15 mL) and trifluoroacetic acid (3 mL) was added dropwise. After the addition was completed, the mixture was reacted at room temperature for 2 h. After the reaction was completed, the reaction solution was concentrated to give compound 18E (200 mg, yield: 88%).

### Step 5:

Intermediate 2 (300 mg, 0.83 mmol) was dissolved in dry N,N dimethylformamide (6 mL), and caesium carbonate (0.81 g, 2.49 mmol) and compound 18E (200 mg, 1.08 mmol) were added in sequence. After the addition was completed, the resulting mixture was reacted at 80°C for 16 h. After the reaction was completed as monitored by TLC (petroleum ether : ethyl acetate = 1 : 2 (v/v)), saturated ammonium chloride aqueous solution was added. The resulting mixture was extracted with ethyl acetate (100 mL × 2), and the organic phases were combined, washed with saturated sodium chloride aqueous solution (100 mL × 1), dried over anhydrous sodium sulphate and filtered. The filtrate was concentrated under reduced pressure and purified by column chromatography (dichloromethane : methanol = 50 : 1 (v/v)) to give compound 18 (130 mg, yield: 30%).

LCMS *m*/*z* = 528.0 [M+1]⁺
¹H NMR (400 MHz, DMSO-*d₆*) δ 8.32 (s, 1H), 7.79 (d, 1H), 7.68 (d, 1H), 7.53 (d, 1H), 7.40 (s, 2H), 6.37 (t, 1H), 4.47 (t, 2H), 4.35 (s, 4H).

### Examples 19-P1 and 19-P2:

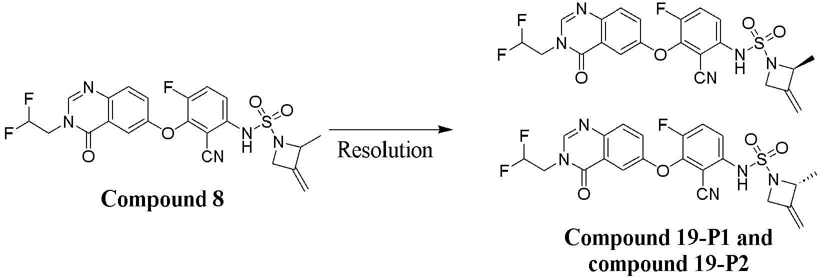

### Step 1:

Compound 8 (140 mg, synthesized with reference to compound 8) was subjected to chiral preparative SFC to give compound 19-P1 (17 mg, yield: 12%, retention time: 1.634 min) and compound 19-P2 (14 mg, yield: 10%, retention time: 1.865 min).

Preparative SFC method: 1. instrument: Waters 150 Prep SFC. chromatographic column: chiral OX chromatographic column. mobile phase: A: CO2; B: 0.1% NH3·H2O in methanol. isocratic condition: 30% B. flow rate: 100 mL/min. back pressure: 100 bar. column temperature: room temperature. wavelength: 220 nm. cycle time: 3.5 min. 2. sample preparation: 1.0 mg/ml compound, dissolved in acetonitrile and methanol. injection: 1.0 ml/injection. After separation, the fraction was dried in a water bath at 45°C by a rotary evaporator to give a product. Then the solvent was dried at -80°C with a lyophiliser to give compounds 19-P1 and 19-P2.

Compound 19-P1, LCMS m/z = 506.2 [M+H]+.

¹H NMR (400 MHZ, DMSO-*d₆*) δ 8.34 (s, 1H), 7.95 (dd, 1H), 7.83 (d, 1H), 7.72 (dd, 1H), 7.68 (dd, 1H), 7.44 (d, 1H), 6.36 (tt, 1H), 5.23 (d, 2H), 4.48 (dt, 3H), 4.29 (d, 1H), 4.26 - 4.19 (m, 1H), 1.39 (d, 3H).

Compound 19-P2, LCMS m/z = 506.2 [M+H]+.

¹H NMR (400 MHZ, DMSO-*d₆*) δ 8.34 (s, 1H), 7.95 (dd, 1H), 7.83 (d, 1H), 7.72 (dd, 1H), 7.68 (dd, 1H), 7.44 (d, 1H), 6.36 (tt, 1H), 5.23 (d, 2H), 4.48 (dt, 3H), 4.29 (d, 1H), 4.27 - 4.18 (m, 1H), 1.39 (d, 3H).

### Example 20:

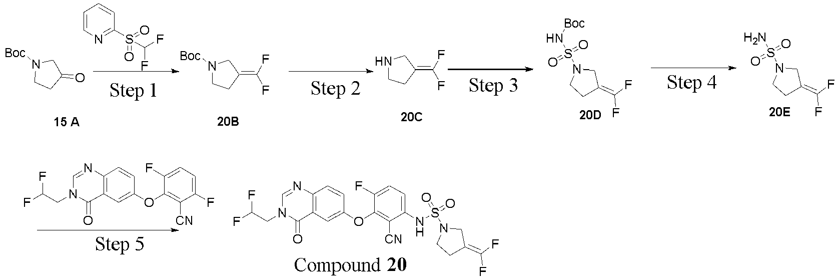

### Step 1:

2-Difluoromethanesulphonylpyridine (4.69 g, 24.29 mmol) and 15A (5.0 g, 26.99 mmol) were dissolved in dry DMF (50 mL), and at -45°C, potassium tert-butoxide (4.54 g, 40.48 mmol, dissolved in 15 mL of DMF) was slowly added dropwise. After the addition was completed, the mixture was reacted at room temperature overnight. After the reaction was completed as monitored by TLC (petroleum ether: ethyl acetate = 5 : 1 (v/v)), saturated ammonium chloride aqueous solution (100 mL) was added to quench the reaction. 3 M HCl (14 mL) was added and the resulting mixture was stirred for another 3 h. Then ethyl acetate (200 mL) was added and the mixture was stirred for another one hour. The reaction system was then subjected to phase separation. The organic phase was washed with saturated sodium chloride aqueous solution (100 mL × 1), dried over anhydrous sodium sulphate and filtered. The filtrate was concentrated under reduced pressure and then purified by column chromatography (petroleum ether : ethyl acetate = 10 : 1 (v/v)) to give compound 20B (1.5 g, yield: 15%).

### Step 2:

Compound 20B (0.35 g, 1.60 mmol) was dissolved in dichloromethane (12 mL) and trifluoroacetic acid (4 mL) was added dropwise. After the addition was completed, the mixture was reacted at room temperature for 2 h. After the reaction was completed, the reaction solution was concentrated to give crude product 20C, which was directly used in the next reaction.
LCMS *m*/*z =* 120.1 [M+1]⁺

### Step 3:

Compound 20C (0.2 g, 1.68 mmol) was dissolved in acetonitrile (5 mL), and in an ice bath, triethylamine (0.51 g, 5.04 mmol) was slowly added dropwise. Intermediate 4 (0.61 g, 2.02 mmol) was added. After the addition was completed, the mixture was reacted at room temperature overnight. After the reaction was completed as monitored by TLC (petroleum ether : ethyl acetate = 1 : 1 (v/v)), saturated sodium bicarbonate aqueous solution was added. The resulting mixture was extracted with ethyl acetate (20 mL × 2), dried over anhydrous sodium sulphate and filtered. The filtrate was concentrated under reduced pressure and purified by column chromatography (petroleum ether : ethyl acetate = 2 : 1 (v/v)) to give compound 20D (260 mg, yield: 52%).

### Step 4:

Compound 20D (260 mg, 0.87 mmol) was dissolved in dichloromethane (15 mL) and trifluoroacetic acid (3 mL) was added dropwise. After the addition was completed, the mixture was reacted at room temperature for 2 h. After the reaction was completed, the reaction solution was concentrated to give compound 20E (170 mg, yield: 98%).

### Step 5:

Intermediate 2 (230 mg, 0.63 mmol) was dissolved in dry N,N dimethylformamide (6 mL), and caesium carbonate (0.62 g, 1.89 mmol) and compound 20E (160 mg, 0.82 mmol) were added in sequence. After the addition was completed, the resulting mixture was reacted at 80°C for 16 h. After the reaction was completed as monitored by TLC (petroleum ether : ethyl acetate = 1 : 2 (v/v)), saturated ammonium chloride aqueous solution was added. The resulting mixture was extracted with ethyl acetate (100 mL × 2), and the organic phases were combined, washed with saturated sodium chloride aqueous solution (100 mL × 1), dried over anhydrous sodium sulphate and filtered. The filtrate was concentrated under reduced pressure and purified by column chromatography (dichloromethane : methanol = 50 : 1 (v/v)) to give compound 20 (120 mg, yield: 35%).

LCMS *m*/*z =* 542.1 [M+1]⁺
¹H NMR (400 MHz, DMSO-*d₆*) δ 8.34 (s, 1H), 7.91 - 7.84 (m, 1H), 7.81 (d, 1H), 7.72 (dd, 1H), 7.55 (dd, 1H), 7.42 (d, 1H), 6.52 - 6.20 (m, 1H), 4.47 (td, 2H), 3.96 (s, 2H), 3.42 (t, 2H), 2.62 - 2.54 (m, 2H).

### Example 21

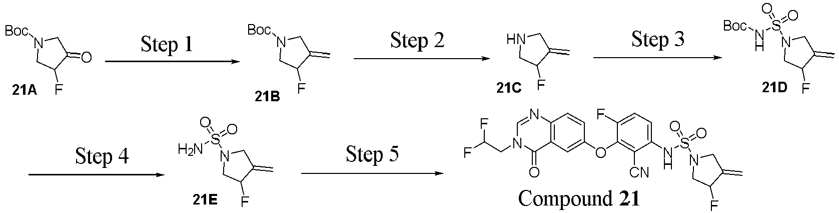

### Step 1:

Methyltriphenylphosphonium iodide (3.00 g, 7.39 mmol) and toluene (30 mL) were added into a single-necked flask, and the mixture was stirred at 0°C. Then a solution of 1 M potassium bis(trimethylsilyl)amide (7.4 mL, 7.39 mmol) in tetrahydrofuran was slowly added dropwise. After the dropwise addition was completed, the mixture was stirred for 0.5 hours, and **21A** (1.00 g, 4.92 mmol) was added finally. Upon completion of the reaction as monitored by LCMS, saturated ammonium chloride solution was added to quench the reaction, and the mixture was extracted with ethyl acetate (30 mL × 3). The organic layer was dried over anhydrous sodium sulphate and filtered and the filtrate was concentrated under reduced pressure. The residue was separated and purified by column chromatography (petroleum ether / ethyl acetate = 5/1, v/v) to give compound **21B** (0.45 g, 45.0%).
LC-MS (ESI): m/z = 146.1 [M-55]⁺.

### Step 2:

**21B** (0.45 g, 2.24 mmol) was dissolved in dichloromethane (15 mL), and at 0°C, trifluoroacetic acid (5 mL) was slowly added dropwise. After the addition was completed, the mixture was reacted at room temperature for 2 hours. After the reaction was completed, the reaction solution was concentrated to give crude product **21C,** which was directly used in the next reaction.
LC-MS (ESI): m/z = 102.2 [M+H]⁺.

### Step 3:

Crude **21C** and acetonitrile (15 mL) were added into a single-necked flask, and at 0°C, triethylamine (0.90 g, 8.96 mmol) was slowly added. Intermediate **4** (0.81 g, 2.67 mmol) was added finally and the mixture was reacted at room temperature overnight. After the reaction was completed, the reaction solution was concentrated, and the residue was purified by column chromatography (petroleum ether / ethyl acetate = 2/1, v/v) to give crude product **21D** (0.42 g, 67.0%).
LC-MS (ESI): m/z = 225.1 [M-55]⁺.

### Step 4:

**21D** (0.42 g, 1.50 mmol) was dissolved in dichloromethane (15 mL), and at 0°C, trifluoroacetic acid (5 mL) was slowly added dropwise. After the addition was completed, the mixture was reacted at room temperature for 2 hours. After the reaction was completed, the reaction solution was concentrated to give crude product **21E,** which was directly used in the next reaction.
LC-MS (ESI): m/z = 181.2 [M+H]⁺.

### Step 5:

Crude compound **21E** from the previous step and caesium carbonate (1.46 g, 4.5 mmol) were added to N, N-dimethylformamide (15 mL), and then intermediate **2** (0.65 g, 1.80 mmol) was added finally. After the addition was completed, the mixture was stirred at 80°C overnight. Upon completion of the reaction, the reaction solution was filtered, the filtrate was concentrated, and the resulting residue was separated and purified by preparative liquid chromatography to give compound **21** (30 mg, 3.8%).

¹H NMR ((400 MHz, DMSO-*d₆*) δ 10.45 (s, 1H), 8.34 (s, 1H), 7.90-7.85 (m, 1H), 7.81 (d, 1H), 7.70 (dd, 1H), 7.53 (dd, 1H), 7.42 (d, 1H), 6.51-6.22 (m, 1H), 5.55-5.38 (m, 2H), 4.52-4.43 (m, 2H), 4.11-4.07 (m, 1H), 3.95-3.92 (m, 1H), 3.63-3.50 (m, 3H), LC-MS (ESI): m/z = 524.1 [M+H]⁺.

### Example 22:

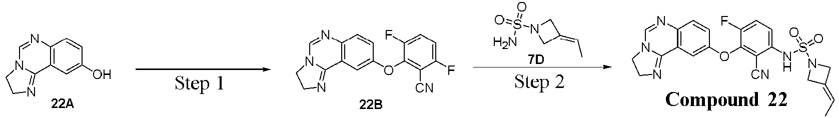

### Step 1:

Compound 22A (1.50 g, 8.01 mmol) (compound 22A was synthesized with reference to the patent WO 2019/060611, A1) was dissolved in N, N-dimethylformamide (30 mL), and in an ice/water bath, caesium carbonate (5.22 g, 16.02 mmol) was slowly added. The mixture was stirred at room temperature for 30 min and in an ice/water bath, a solution of 2, 3, 6-trifluorobenzonitrile (2.52 g, 16.02 mmol) in N, N-dimethylformamide (5 mL) was slowly added dropwise. After the addition was completed, the resulting mixture was reacted at room temperature for 2 h. The reaction solution was diluted with ethyl acetate (100 mL), and washed with water (50 mL × 1) and saturated sodium chloride aqueous solution (50 mL × 1) in sequence. The organic layer was dried over anhydrous sodium sulphate, filtered, and concentrated under reduced pressure. The residue was separated and purified by column chromatography (PE/EA = 2/1) to give 22B (1.20 g, yield: 46%).
LCMS m/z = 325.00 [M+H]⁺;

### Step 2:

Compound 7D (0.20 g, 1.23 mmol) and caesium carbonate (0.53 g, 1.62 mmol) were added to N,N-dimethylformamide (15 mL) and the mixture was reacted at 50°C for 30 min. Then compound 22B (0.25 g, 0.77 mmol) was added. After the addition was completed, the resulting mixture was stirred at 80°C overnight. Upon completion of the reaction, the reaction solution was filtered, the filtrate was concentrated, and the resulting residue was separated and purified by preparative liquid chromatography to give compound 22 (110 mg, yield: 30%).

LCMS m/z = 467.10 [M+H]⁺;
¹H NMR (400 MHz, DMSO-*d₆*) δ 10.66 (s, 1H), 8.69 (s, 1H), 8.02 (d, 1H), 7.99-7.96 (m, 1H), 7.94-7.88 (m, 1H), 7.78 (d, 1H), 7.64-7.57 (m, 1H), 5.46-5.34 (m, 1H), 4.74-4.63 (m, 2H), 4.21 (d, 4H), 4.20-4.10 (m, 2H), 1.49 (d, 3H).

### Example 23:

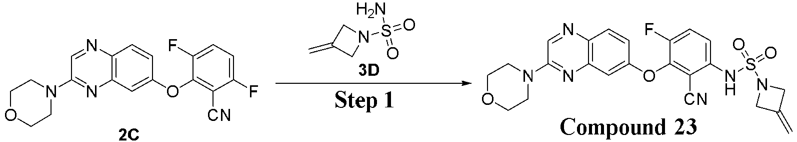

### Step 1:

Compound 3D (0.15 g, 1.02 mmol) and caesium carbonate (0.44 g, 1.36 mmol) were added to N,N-dimethylformamide (15 mL) and the mixture was reacted at 50°C for 30 min. Then compound 2C (0.25 g, 0.68 mmol) was added. After the addition was completed, the resulting mixture was stirred at 80°C overnight. Upon completion of the reaction, the reaction solution was filtered, the filtrate was concentrated, and the resulting residue was separated and purified by preparative liquid chromatography to give compound 23 (90 mg, yield: 27%).

LCMS m/z = 497.30 [M+H]⁺;
¹H NMR (400 MHz, DMSO-*d₆*) δ 10.53 (s, 1H), 8.74 (s, 1H), 7.94-7.80 (m, 2H), 7.60-7.51 (m, 1H), 7.31-7.21 (m, 1H), 6.83 (d, 1H), 5.09-5.04 (m, 2H), 4.57-4.47 (m, 4H), 3.72 (s, 8H).

### Example 24:

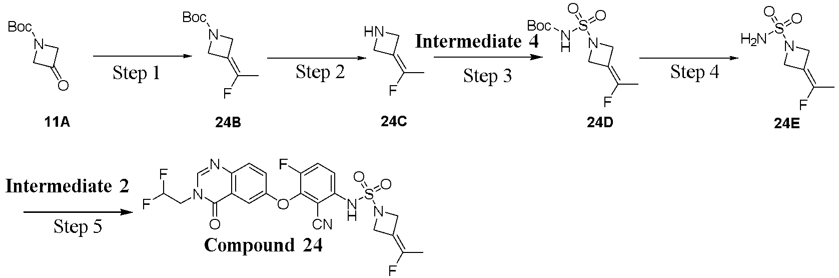

### Step 1:

1-Fluoroethyltriphenylphosphonium tetrafluoroborate (10 g, 25.24 mmol) was dissolved in dry tetrahydrofuran (200 mL), and at -78°C, sodium bis(trimethylsilyl)amide (23 mL, 25.24 mmol, 2M in THF) was slowly added dropwise. After the addition was completed, the mixture was reacted at -78°C for 1 h. At -78°C, a solution of compound 11A (4.32 g, 25.24 mmol) in tetrahydrofuran (20 mL) was slowly added dropwise. After the addition was completed, the mixture was reacted at room temperature overnight. After the reaction was completed as monitored by TLC (petroleum ether : ethyl acetate = 5 : 1 (v/v)), saturated ammonium chloride aqueous solution was added to quench the reaction. The resulting mixture was extracted with ethyl acetate (800 mL × 2), and the organic phases were combined, washed with saturated sodium chloride aqueous solution (250 mL × 1), dried over anhydrous sodium sulphate and filtered. The filtrate was concentrated under reduced pressure and purified by column chromatography (petroleum ether : ethyl acetate = 5 : 1 (v/v)) to give compound 24B (1.2 g, yield: 24%).
LCMS *m*/*z* = 146.2 [M+1-56]⁺

### Step 2:

Compound 24B (1.2 g, 5.96 mmol) was dissolved in dichloromethane (25 mL) and trifluoroacetic acid (5 mL) was added dropwise. After the addition was completed, the mixture was reacted at room temperature for 2 h. After the reaction was completed, the reaction solution was concentrated to give crude product 24C as a trifluoroacetate, which was directly used in the next reaction.
LCMS *m*/*z* = 102.2 [M+1]⁺

### Step 3:

The trifluoroacetate of compound 24C (1283 mg, 5.96 mmol) was dissolved in acetonitrile (40 mL), and in an ice bath, triethylamine (3.7 mL, 26.7 mmol) was slowly added dropwise. Intermediate 4 (2.42 g, 8.01 mmol) was added. After the addition was completed, the mixture was reacted at room temperature overnight. After the reaction was completed as monitored by TLC (petroleum ether : ethyl acetate = 1 : 1 (v/v)), saturated sodium bicarbonate aqueous solution was added. The resulting mixture was extracted with ethyl acetate (100 mL × 2), dried over anhydrous sodium sulphate and filtered. The filtrate was concentrated under reduced pressure to give crude product 24D, which was directly used in the next reaction.
LCMS *m*/*z* = 225.2 [M+1-56]⁺

### Step 4:

Compound 24D (1.67 g, 5.96 mmol) was dissolved in dichloromethane (20 mL) and trifluoroacetic acid (4 mL) was added dropwise. After the addition was completed, the mixture was reacted at room temperature for 2 h. After the reaction was completed, the reaction solution was concentrated and then purified by column chromatography (petroleum ether : ethyl acetate = 1 : 1 (v/v)) to give compound 24E (800 mg, yield: 74%).
LCMS *m*/*z* = 181.2 [M+1]⁺

### Step 5:

Intermediate 2 (580 mg, 1.60 mmol) was dissolved in dry N,N dimethylformamide (30 mL), and caesium carbonate (2.17 g, 6.66 mmol) and compound 24E (600 mg, 3.33 mmol) were added in sequence. After the addition was completed, the resulting mixture was reacted at 80°C for 6 h. After the reaction was completed as monitored by TLC (petroleum ether : ethyl acetate = 1 : 2 (v/v)), saturated ammonium chloride aqueous solution was added. The resulting mixture was extracted with ethyl acetate (100 mL × 2), and the organic phases were combined, washed with saturated sodium chloride aqueous solution (100 mL × 1), dried over anhydrous sodium sulphate and filtered. The filtrate was concentrated under reduced pressure and purified by column chromatography (dichloromethane : methanol = 50 : 1 (v/v)) to give compound 24 (524 mg, yield: 63%).

LCMS *m*/*z =* 524.1 [M+1]⁺
1H NMR (400 MHZ, DMSO-d₆) δ 10.57 (s, 1H), 8.34 (s, 1H), 7.91 - 7.83 (m, 1H), 7.81 (d, 1H), 7.72 (dd, 1H), 7.56 (dd, 1H), 7.44 (d, 1H), 6.36 (tt, 1H), 4.55 - 4.41 (m, 6H), 1.85 - 1.77 (m, 3H).

### Example 25:

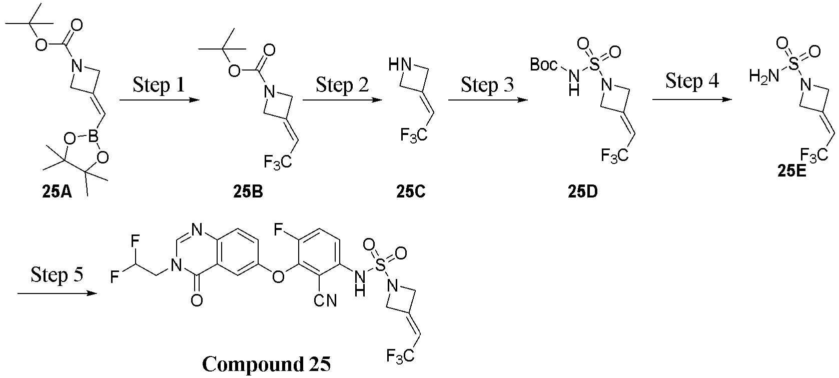

### Step 1:

25A (0.8 g, 2.71 mmol, synthesized with reference to WO 2020185593A1) and methanol (10 mL) were added to a 50 mL single-necked flask, and then 1-(trifluoromethyl)-1,2-benziodoxol-3(1H)-one (1.03 g, 3.25 mmol) and ferrous chloride (68.70 mg, 0.54 mmol) were added. The mixture was reacted at room temperature overnight. After the reaction was completed, the reaction solution was concentrated, and the residue was purified by column chromatography (petroleum ether / ethyl acetate = 9/1, v/v) to give 25B (420 mg, yield: 65%).

LCMS m/z = 182.1 [M-55]⁺;
¹H NMR (400 MHz, CDCl₃) δ 5.52 (m, 1H), 4.62-4.59 (m, 2H), 4.52-4.48 (m, 2H), 1.39 (s, 9H).

### Step 2:

25B (0.42 g, 1.77 mmol) was dissolved in dichloromethane (9 mL), and at 0-5°C, trifluoroacetic acid (3 mL) was slowly added dropwise. After the addition was completed, the mixture was reacted at room temperature for 2 h. After the reaction was completed, the reaction solution was concentrated to give crude product 25C, which was directly used in the next reaction.
LCMS m/z = 138.2 [M+H]⁺;

### Step 3:

Crude product 25C and acetonitrile (10 mL) were added into a 50 mL single-necked flask, and at 0-5°C, triethylamine (716.43 mg, 7.08 mmol) and (tert-butoxycarbonyl)((4-(dimethylimino)pyridin-1(4H)yl)sulphonyl)amide (803 mg, 2.65 mmol) were slowly added. The mixture was reacted at room temperature overnight. After the reaction was completed, the reaction solution was concentrated, and the residue was purified by column chromatography (petroleum ether / ethyl acetate = 7/3, v/v) to give 25D (400 mg, yield: 71%).
LCMS m/z = 261.0 [M-55]⁺;

### Step 4:

25D (400 mg, 1.26 mmol) was dissolved in dichloromethane (6 mL) in a 50 mL single-necked flask, and at 0-5°C, trifluoroacetic acid (2 mL) was slowly added dropwise. After the addition was completed, the mixture was reacted at room temperature overnight. After the reaction was completed, the reaction solution was concentrated, and the organic phase was concentrated. Then the residue was purified by column chromatography (petroleum ether/ethyl acetate = 3/2) to give 25E (170 mg, yield: 62%).
LCMS m/z = 217.1 [M+H]⁺;

### Step 5:

25E (169 mg, 0.78 mmol) and caesium carbonate (678 mg, 2.08 mmol) were added to dry N,N-dimethylformamide (5 mL) in a 25 mL single-necked flask, and the mixture was reacted at 50°C for 30 min. At 50°C, intermediate 2 (190 mg, 0.52 mmol) was added. After the addition was completed, the resulting mixture was stirred and reacted at 80°C overnight. After the reaction was completed, the reaction solution was filtered, the filter cake was washed with N,N-dimethylformamide (2 mL), and the filtrate was concentrated under reduced pressure. The crude was purified by preparative HPLC to give compound 25 (15 mg, 5%).

LCMS m/z = 560.2 [M+H]⁺;
¹H NMR (400 MHz, DMSO-*d₆*) δ 10.69 (s, 1H), 8.34 (s, 1H), 7.91 - 7.86 (m, 1H), 7.82-7.81 (d, 1H), 7.74-7.71 (dd, 1H), 7.60-7.56 (dd, 1H), 7.45-7.44 (d, 1H), 6.51-6.22 (m, 1H), 6.12 - 6.06 (m, 1H), 4.78 (s, 2H), 4.68 (s, 2H), 4.52-4.43 (m, 2H).

### Example 26:

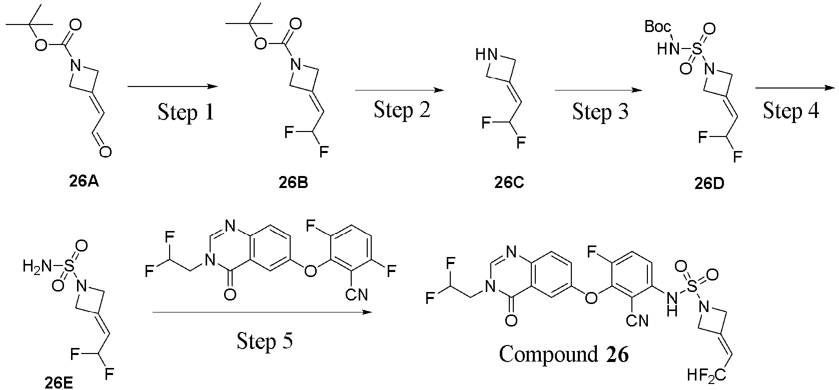

### Step 1:

**26A** (4.6 g, 26.87 mmol, referring to document WO 2013/59587A1 for the synthesis method) was dissolved in dry dichloromethane (200 mL), and at 0°C, DAST (25.99 g, 161.22 mmol) was slowly added dropwise. After the addition was completed, the mixture was reacted at room temperature overnight. After the reaction was completed as monitored by TLC (petroleum ether : ethyl acetate = 5 : 1 (v/v)), saturated sodium bicarbonate aqueous solution (150 mL) was added to quench the reaction. Then the system was subjected to phase separation. Dichloromethane (150 mL) was added to the aqueous phase for extraction. The organic phases were combined, dried over anhydrous sodium sulphate and filtered. The filtrate was concentrated under reduced pressure and purified by column chromatography (petroleum ether : ethyl acetate = 10 : 1 (v/v)) to give compound **26B** (2.8 g, yield: 52.8%).
LCMS *m*/*z =* 164.1 [M-56+1]⁺

### Step 2:

Compound **26B** (0.8 g, 4.39 mmol) was dissolved in dichloromethane (10 mL) and trifluoroacetic acid (3 mL) was added dropwise. After the addition was completed, the mixture was reacted at room temperature for 2 h. After the reaction was completed, the reaction solution was concentrated to give crude product **26C,** which was directly used in the next reaction.
LCMS *m*/*z =* 120.1 [M+1]⁺

### Step 3:

Crude product **26C** was dissolved in acetonitrile (20 mL), and in an ice bath, triethylamine (1.52 g, 26.7 mmol) was slowly added dropwise. Intermediate 4 (1.67 g, 5.52 mmol) was added. After the addition was completed, the mixture was reacted at room temperature overnight. After the reaction was completed as monitored by TLC (petroleum ether : ethyl acetate = 1 : 1 (v/v)), saturated sodium bicarbonate aqueous solution was added. The resulting mixture was extracted with ethyl acetate (100 mL × 2), dried over anhydrous sodium sulphate and filtered. The filtrate was concentrated under reduced pressure and purified by column chromatography (petroleum ether : ethyl acetate = 2 : 1 (v/v)) to give compound **26D** (300 mg, yield: 27.2%).

### Step 4:

Compound **26D** (300 mg, 1.23 mmol) was dissolved in dichloromethane (15 mL) and trifluoroacetic acid (3 mL) was added dropwise. After the addition was completed, the mixture was reacted at room temperature for 2 h. After the reaction was completed, the reaction solution was concentrated to give compound **26E** (200 mg, yield: 100%).

### Step 5:

Intermediate **2** (290 mg, 0.81 mmol) was dissolved in dry N,N dimethylformamide (6 mL), and caesium carbonate (0.66 g, 2.02 mmol) and compound **26E** (200 mg, 1.01 mmol) were added in sequence. After the addition was completed, the resulting mixture was reacted at 80°C for 16 h. After the reaction was completed as monitored by TLC (petroleum ether : ethyl acetate = 1 : 2 (v/v)), saturated ammonium chloride aqueous solution was added. The resulting mixture was extracted with ethyl acetate (100 mL × 2), and the organic phases were combined, washed with saturated sodium chloride aqueous solution (100 mL × 1), dried over anhydrous sodium sulphate and filtered. The filtrate was concentrated under reduced pressure and purified by column chromatography (dichloromethane : methanol = 50 : 1 (v/v)) to give compound **26** (85 mg, yield: 15.6%).

LCMS *m*/*z =* 542.0 [M+1]⁺
¹H NMR (400 MHz, DMSO-*d₆*) δ 10.62 (s, 1H), 8.35 (s, 1H), 7.93 - 7.77 (m, 2H), 7.73 (dd, 1H), 7.57 (dd, 1H), 7.45 (d, 1H), 6.63 - 6.20 (m, 2H), 5.78 (d, 1H), 4.67 (d, 4H), 4.48 (td, 2H).

### Example 27:

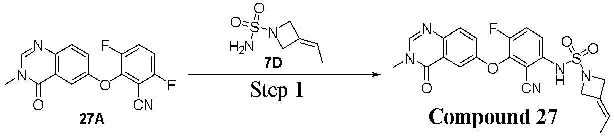

### Step 1:

Compound 7D (0.30 g, 1.85 mmol) and caesium carbonate (0.78 g, 2.41 mmol) were added to N,N-dimethylformamide (10 mL) and the mixture was reacted at 50°C for 30 min. Then compound 27A (0.3 g, 0.96 mmol) (synthesized with reference to patent WO 2021116050A1) was added. After the addition was completed, the resulting mixture was stirred at 80°C overnight. Upon completion of the reaction, the reaction solution was filtered, the filtrate was concentrated, and the resulting residue was separated and purified by preparative liquid chromatography to give compound 27 (280 mg, yield: 64%).

LCMS m/z = 456.10 [M+H]⁺;
¹H NMR (400 MHz, DMSO-*d₆*) δ 10.49 (s, 1H), 8.36 (s, 1H), 7.89-7.84 (m, 1H), 7.77 (d, 1H), 7.68-7.65 (m, 1H), 7.58-7.53 (m, 1H), 7.41 (d, 1H), 5.46-5.35 (m, 1H), 4.53-4.42 (m, 4H), 3.48 (s, 3H), 1.51-1.45 (m, 3H).

### Example 28:

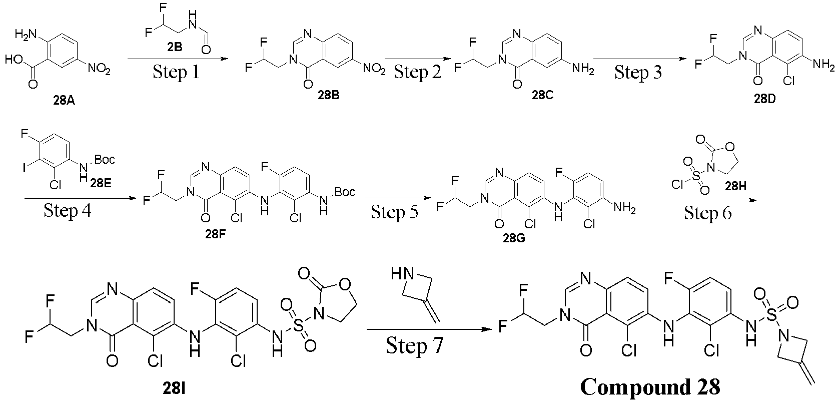

### Step 1:

**28A** (10.0 g, 54.91 mmol) was added to 2B (30 g, 274 mmol) in a 50 mL single-necked flask and the mixture was reacted at 155°C for 12 h. After the reaction was completed, the mixture was cooled to room temperature and filtered. The filter cake was washed with ethyl acetate (5 mL*2), and then the filter cake was concentrated to give **28B** (4.0 g, yield: 29%).
LCMS m/z = 256.0 [M+H]⁺;

### Step 2:

In a single-necked flask, compound **28B** (4.0 g, 15.68 mmol), palladium on carbon (0.4 g) and methanol (200 mL) were added in sequence. The system was purged three times with hydrogen and then stirred and reacted at room temperature for 2 h. The reaction solution was filtered through Celite and the solid was washed with methanol. The filtrate was concentrated under reduced pressure to give compound **28C** (3.5 g, yield: 99%), which was directly used in the next reaction without further purification.

LC-MS (ESI): m/z= 226.1 [M+H]⁺.

### Step 3:

**28C** (3.5 g, 15.5 mmol) was dissolved in acetonitrile (50 mL) and NCS (2.49 g, 18.7 mmol) was added at room temperature. After the addition was completed, the mixture was reacted at 85°C overnight. After the reaction was completed as monitored by TLC (petroleum ether : ethyl acetate = 1 : 1 (v/v)), the reaction solution was concentrated under reduced pressure and purified by column chromatography (petroleum ether : ethyl acetate = 2 : 1 (v/v)) to give compound **28D** (3.8 g, yield: 94.2.2 %).

LC-MS (ESI): m/z= 260.1 [M+H]⁺.

### Step 4:

**28D** (2.5 g, 9.63 mmol), **28E** (3.58 g, 9.63 mmol), caesium carbonate (9.41 g, 28.89 mmol) and 1, 4-dioxane (40 ml) were added into a flask, followed by 4,5-bis(diphenylphosphino)-9,9-dimethylxanthene (1.11 g, 1.93 mmol) and tris(dibenzylideneacetone)dipalladium (0.88 g, 0.96 mmol), and then the mixture was reacted at 100°C under nitrogen atmosphere for 16 h. The reaction solution was cooled, water was added, and the resulting mixture was extracted with ethyl acetate, washed, dried and concentrated. The residue was purified by column chromatography to give compound **28F** (1.3 g, 26.8%).

LC-MS (ESI): m/z = 503.0[M+H]+.

### Step 5:

**28F** (1.3 g, 2.58 mmol) was dissolved in a hydrogen chloride 1,4-dioxane solution (20 mL, 4 mmol/L), and the mixture was reacted at room temperature for 5 h. Upon completion of the reaction, the reaction solution was subjected to rotary evaporation to dryness to give product **28G** (1.0 g, 81.3%), which was directly used in the next step.

LCMS m/z = 403.0 [M+H]+.

### Step 6:

**28G** (0.4 g, 0.99 mmol) was dissolved in DCM (15 mL), and then triethylamine (0.3 g, 2.97 mmol) and **28H** (0.18 g, 0.99 mmol) were added in sequence. The mixture was reacted at room temperature for 1 h. Upon completion of the reaction, the reaction solution was directly concentrated to give crude product **28I,** which was used in the next step.

### Step 7:

Crude **28I** was dissolved in acetonitrile (15 mL), and 3-methyleneazetidine hydrochloride (0.19 g, 1.82 mmol) and triethylamine (280 mg, 2.73 mmol) were added in sequence. After the addition was completed, the mixture was reacted at 80°C for 16 h. Upon completion of the reaction, the reaction solution was concentrated under reduced pressure and then purified by column chromatography (dichloromethane : methanol = 50 : 1 (v/v)) to give compound **28** (28 mg, two-step yield: 5.9%).

LCMS *m*/*z =* 534.1 [M+1]⁺
¹H NMR (400 MHz, DMSO-d6) δ 8.19 (s, 1H), 7.82 (s, 1H), 7.55-7.35 (m, 3H), 6.79 (d, 1H), 6.38 (t, 1H), 5.04 (s, 2H), 4.46 (d, 6H).

### Biological test

### 1. BRAF_{V600E} enzyme activity test

BRAF_{V600E} (ABCAM, ab204154) and MEK1_{K97R} (USBio, M2865-06J) proteins were diluted at an appropriate dilution factor using 1×Assay buffer (PH = 7.4 Tris-HCl buffer, supplemented with 10 mM MgCl₂) such that the final concentration of BRAF_{V600E} was 10 ng/µL and the final concentration of substrate MEK1_{K97R} was 1 µM. 1 µL of BRAF_{V600E}, 1 µL of compound serial dilution (final concentration starting from 2 µM, 5-fold dilution, 8 concentrations), and 6 µL of Assay buffer were pipetted to a 384-well reaction plate with a capacity of 20 µL. The plate was pre-incubated in a constant temperature incubator at 37°C for 30 min. 1 µL of 200 µM ATP and 1 µM MEK1 _{K97R} were added to the reaction wells corresponding to the compound incubation, and mixed evenly by shaking, and preincubation was performed in a constant temperature incubator at 37°C for 60 min for enzymatic reaction. After the reaction was completed, 5 µL of the above reaction product was pipetted to another 384-well plate, and 5 µL of formulated ADP-Glo^{™} Reagent (Promega, V9101) was added and mixed evenly by pipetting, and then the plate was placed at room temperature for 40 min. 10 µL Kinase Detection Reagent was added to the 384-well plate and incubated at room temperature for 40 min. Finally, a microplate reader (BMG LRBTECH) was used and Luminescence module was selected to detect the LUM fluorescence value of each well (the gain value was fixed at 3600), and the formula $Inhibition rate % = \left(1-\frac{LUM Compound - LUM Background}{LUM Solvent - LUM Background}\right) ∗ 100 %$ was used to calculate the inhibition rate of the compound on BRAF_{V600E}. The Graphpad software log(inhibitor) vs. response -- Variable slope (four parameters) equation was used to perform fitting analysis and calculate the IC₅₀value of the sample.

The compounds of the present invention, such as the compounds in the examples, have very good enzymatic activities, with IC₅₀ ≤ 100 nM. The inhibitory activities of some compounds on BRAF_{V600E} are shown in Table 1.

**Table 1 Inhibitory activities of compounds on BRAF_{V600E}**

| Compound No. | IC₅₀/nM |
|---|---|
| Compound 3 | A |
| Compound 7 | A |
| Compound 14 | A |
| Compound 24 | A |

A represents IC₅₀ ≤ 10 nM, B represents 10 nM < IC₅₀ ≤ 30 nM, C represents 30 nM < IC₅₀ ≤ 100 nM.

### 2. A375 cell proliferation inhibition test

A375 cells (ATCC, CRL-1619) were cultured in DMEM complete medium (+10% FBS) in a CO₂ incubator at 37°C for 48 h. The cells were trypsinised and counted, and then the density was adjusted to 1.67 × 10⁴ cells/mL. 90 µL (1500 cells) of cells were inoculated into each well of a 96-well transparent bottom plate, and the plate was transferred to a CO₂ incubator and the cells were cultured at 37°C overnight. After the cells were incubated overnight, 10 µL of the diluted compound (final concentration starting from 10 µM, 3-fold dilution, 11 concentrations) was added to each well using a multi-channel pipette. The positive control was a serum-free medium containing DMSO. After mixing evenly, the cells were placed in a CO₂ incubator at 37°C for 72 h. After the incubation, the CellCounting-Lite^{®}2.0 kit detection solution (Vazyme, DD1101-03) was taken out and returned to room temperature. 100 µL of CellCounting-Lite2.0 detection solution was added to each well, the plate was sealed with a sealing film, and the plate was placed on a shaker for shaking for 15 min (the whole process should be kept away from light). The Luminescence module of the microplate reader (BMG LRBTECH) was used to detect the fluorescence signal value LUM of each well. The formula $Inhibition rate % = \left(\frac{LUM Solvent - LUM Compound}{LUM Solvent}\right) ∗ 100 %$ was used to calculate the inhibition rate of the compound. The Graphpad software log(inhibitor) vs. response -- Variable slope (four parameters) equation was used to perform fitting analysis and calculate the IC₅₀value of the sample. The ordinate of the data is the percentage of inhibition rate, and the abscissa is the logarithm of the sample concentration (Log₁₀).

The compounds of the present invention, such as the compounds in the examples, have very good cell activities, with IC₅₀ ≤ 100 nM. The inhibitory activities of some compounds on A357 cells are shown in Table 2.

**Table 2 Inhibitory activities of compounds on A375 cells**

| Compound No. | IC₅₀/nM | Compound No. | IC₅₀/nM |
|---|---|---|---|
| Compound 1 | A | Compound 15 | A |
| Compound 2 | B | Compound 20 | A |
| Compound 3 | A | Compound 21 | A |
| Compound 4 | B | Compound 22 | B |
| Compound 5 | A | Compound 23 | A |
| Compound 6 | A | Compound 24 | A |
| Compound 7 | A | Compound 25 | B |
| Compound 9 | B | Compound 26 | B |
| Compound 11 | A | Compound 27 | A |
| Compound 12 | B | | |
| Compound 14 | A | | |

A represents IC₅₀ ≤ 10 nM, B represents 10 nM < IC₅₀ ≤ 50 nM, C represents 50 nM < IC₅₀ ≤ 100 nM.

Conclusion: the compounds of the present invention, such as the compounds in the examples, show high inhibitory activities at the cellular level. for example, the IC50 of compound 3 is 0.14 nM, the IC50 of compound 7 is 0.66 nM, the IC50 of compound 14 is 1.44 nM, and the IC50 of compound 24 is 2.16 nM.

### 3: Pharmacokinetic test in mice

3.1 Experimental animals: Male ICR mice, 20-25 g, 6 mice/compound, purchased from Chengdu Dossy Experimental Animals Co., Ltd.

3.2 Experimental design: On the day of the experiment, the ICR mice were randomly grouped according to their body weights. The animals were fasted with water available for 12-14 h one day before the administration, and were fed 4 h after the administration.

**Table 3.1 Administration information**

| Group | Number | Administration information | | | | | |
|---|---|---|---|---|---|---|---|
| | Male | Test compound | Administrat ion dosage (mg/kg) | Administrati on concentration (mg/mL) | Adminis tration volume (mL/kg) | Collecte d sample | Administrati on mode |
| G1 | 3 | Control compound 1 | 2.5 | 0.5 | 5 | Plasma | Intravenous administrati on |
| G2 | 3 | | 10 | 1 | 10 | Plasma | Intragastric administrati on |
| G3 | 3 | Compound 3 | 2.5 | 0.5 | 5 | Plasma | Intravenous administrati on |
| G4 | 3 | | 10 | 1 | 10 | Plasma | Intragastric administrati on |
| G5 | 3 | Compound 6 | 2.5 | 0.5 | 5 | Plasma | Intravenous administrati on |
| G6 | 3 | | 10 | 1 | 10 | Plasma | Intragastric administrati on |
| G7 | 3 | Compound 7 | 2.5 | 0.5 | 5 | Plasma | Intravenous administrati on |
| G8 | 3 | | 10 | 1 | 10 | Plasma | Intragastric administrati on |
| G9 | 3 | Compound 11 | 2.5 | 0.5 | 5 | Plasma | Intravenous administrati on |
| G10 | 3 | | 10 | 1 | 10 | Plasma | Intragastric administrati on |
| G11 | 3 | Compound 14 | 2.5 | 0.5 | 5 | Plasma | Intravenous administrati on |
| G12 | 3 | | 10 | 1 | 10 | Plasma | Intragastric administrati on |
| G13 | 3 | Compound 24 | 2.5 | 0.5 | 5 | Plasma | Intravenous administrati on |
| G14 | 3 | | 10 | 1 | 10 | Plasma | Intragastric administrati on |

| | | | | | | | |
|---|---|---|---|---|---|---|---|
| Note: Control compound 1 was Example 1 compound in the document WO 2021116055A1; | | | | | | | |

vehicle for intravenous administration: 5% DMA + 5% Solutol + 90% Saline; vehicle for intragastric administration: 0.5% MC

Before and after administration, 0.06 mL of blood was taken from the orbit under isoflurane anaesthesia and placed in an EDTAK2 centrifuge tube. The tube was centrifuged at 5000 rpm at 4°C for 10 min, and plasma was collected. The blood sampling time points for both the intravenous administration group and intragastric administration group were 0 min, 5 min, 15 min, 30 min, 1 h, 2 h, 4 h, 7 h, 24 h, and 48 h. Before analysis and detection, all samples were stored at -80°C, and the samples were quantitatively analysed by LC-MS/MS.

**Table 3.2 Pharmacokinetic parameters of test compounds in plasma of mice**

| Test compou nd | Mode of administr ation | CL (mL/min/ kg) | Vdₛₛ (L/kg) | AUC₀₋ₜ (hr*ng/mL) | T_{1/2} (h) | F (%) | Fold change in brain-blood ratio relative to control compound |
|---|---|---|---|---|---|---|---|
| Control compou nd 1 | i.v. (2.5 mg/kg) | 0.532 | 0.195 | 75119 | 5.32 | - | - |
| | i.g. (10 mg/kg) | - | - | 240899 | 2.46 | 80.2 | - |
| Compo und 3 | i.v. (2.5 mg/kg) | 0.186 | 0.112 | 225235 | 8.68 | - | - |
| | i.g. (10 mg/kg) | - | - | 761599 | 10.3 | 84.5 | 10.8 |
| Compo und 6 | i.v. (2.5 mg/kg) | 0.282 | 0.118 | 147887 | 6.77 | - | - |
| | i.g. (10 mg/kg) | - | - | 373177 | 4.47 | - | 24.9 |
| Compo und 7 | i.v. (2.5 mg/kg) | 0.171 | 0.156 | 227617 | 12.8 | - | - |
| | i.g. (10 mg/kg) | - | - | 824899 | 10.7 | 90.6 | 10.5 |
| Compo und 11 | i.v. (2.5 mg/kg) | 0.163 | 0.123 | 245280 | 11.1 | - | - |
| | i.g. (10 mg/kg) | - | - | 962192 | 8.81 | 98.1 | 6.1 |
| Compo und 14 | i.v. (2.5 mg/kg) | 0.154 | 0.098 | 267982 | 9.27 | - | - |
| | i.g. (10 mg/kg) | - | - | 911761 | 9.0 | 85.1 | 3.5 |
| Compo und 24 | i.v. (2.5 mg/kg) | 0.462 | 0.237 | 91805 | 8.1 | - | - |
| | i.g. (10 mg/kg) | - | - | 491896 | 9.83 | > 98% | 8.1 |

| | | | | | | | |
|---|---|---|---|---|---|---|---|
| Note: Control compound 1 was Example 1 compound in the document WO 2021116055A1; -: not applicable. Conclusion: The compounds of the present invention, such as the compounds in the examples, have good pharmacokinetic profiles in mice. | | | | | | | |

### 4: Pharmacokinetic test in beagle dogs

**Experimental animals:** Male beagle dogs, about 8 to 11 kg, 6 beagle dogs/compound, purchased from Beijing Marshall Biotechnology Co., Ltd.

**Experimental method:** On the day of the experiment, the beagle dogs were randomly grouped according to their body weights. The animals were fasted with water available for 12-14 h one day before the administration, and were fed 4 h after the administration. The animals were administered according to Table 4.

Test compounds: compounds in the examples and control compounds.

**Table 4. Administration information**

| Group | Number | Administration information | | | | |
|---|---|---|---|---|---|---|
| | Male | Administration dosage (mg/kg) | Administrati on concentration (mg/mL) | Administration volume (mL/kg) | Collected sample | Administra tion mode |
| G1 | 3 | 1 | 1 | 1 | Plasma | Intravenou s administrat ion |
| G2 | 3 | 3 | 0.6 | 5 | Plasma | Intragastric administrat ion |

| | | | | | | |
|---|---|---|---|---|---|---|
| Note: Vehicle for intravenous administration: 5% DMA + 5% Solutol + 90% Saline; and vehicle for intragastric administration: 0.5% MC (DMA: dimethylacetamide; Solutol: polyethylene glycol-15-hydroxystearate; Saline: physiological saline; MC: methylcellulose solution;) | | | | | | |

Before and after the administration, 1 ml of blood was taken from the jugular veins or limb veins and placed in an EDTAK2 centrifuge tube. The tube was centrifuged at 5000 rpm at 4°C for 10 min, and plasma was collected. The blood sampling time points for both the intravenous administration group and intragastric administration group were 0 min, 5 min, 15 min, 30 min, 1 h, 2 h, 4 h, 6 h, 8 h, 10 h, 12 h, 24 h, 48 h, and 72 h. Before analysis and detection, all samples were stored at -80°C, and the samples were quantitatively analysed by LC-MS/MS.

Conclusion: The compounds of the present invention, such as the compounds in the examples, have good pharmacokinetic profiles in beagle dogs.

### 5: Pharmacokinetic test in rats

5.1 Experimental animals: Male SD rats, about 220 g, 6-8 weeks old, 6 rats/compound, purchased from Chengdu Dossy Experimental Animals Co., Ltd.

5.2 Experimental design: On the day of the experiment, the SD rats were randomly grouped according to their body weights. The animals were fasted with water available for 12-14 h one day before the administration, and were fed 4 h after the administration.

5.3 Test compounds: compounds in the examples and control compounds.

**Table 5.1 Administration information**

| Group | Numb er | Administration information | | | | | |
|---|---|---|---|---|---|---|---|
| | Male | Test compound | Administra tion dosage (mg/kg) | Administra tion concentrati on (mg/mL) | Administra tion volume (mL/kg) | Collected sample | Administra tion mode |
| G1 | 3 | Compound 3 | 2.5 | 0.5 | 5 | Plasma | Intravenous administrat ion |
| G2 | 3 | | 10 | 1 | 10 | Plasma | Intragastric administrat ion |
| G3 | 3 | Compound 7 | 2.5 | 0.5 | 5 | Plasma | Intravenous administrat ion |
| G4 | 3 | | 10 | 1 | 10 | Plasma | Intragastric administrat ion |

| | | | | | | | |
|---|---|---|---|---|---|---|---|
| Vehicle for administration: 0.5% MC | | | | | | | |

Before and after administration, 0.1 ml of blood was taken from the orbit under isoflurane anaesthesia and placed in an EDTAK2 centrifuge tube. The tube was centrifuged at 5000 rpm at 4°C for 10 min, and plasma was collected. Blood sampling time points for the intravenous administration group: 0 min, 5 min, 15 min, 30 min, 1 h, 2 h, 4 h, 6 h, 8 h, 24 h, and 48 h; blood sampling time points for the intragastric administration group: 0 min, 5 min, 15 min, 30 min, 1 h, 2 h, 4 h, 6 h, 8 h, 24 h, and 48 h. Before analysis and detection, all samples were stored at -80°C, and the samples were quantitatively analysed by LC-MS/MS.

**Table 5.2 Pharmacokinetic parameters of test compounds in plasma of rats**

| Test compound | Mode of administration | CL (mL/min/kg) | Vdₛₛ (L/kg) | AUC₀₋ₜ (hr*ng/mL) | T_{1/2} (h) | F (%) |
|---|---|---|---|---|---|---|
| Compound 3 | i.v. (2.5 mg/kg) | 0.091 | 0.126 | 40986 | 19.1 | - |
| | i.g. (10 mg/kg) | - | - | 1030347 | 13.9 | 62.8 |
| Compound 7 | i.v. (2.5 mg/kg) | 0.109 | 0.096 | 375892 | 14.2 | - |
| | i.g. (10 mg/kg) | - | - | 1222519 | 13.5 | 81.3 |

| | | | | | | |
|---|---|---|---|---|---|---|
| Note: Control compound 1 was Example 1 compound in the document WO 2021116055A1; -: not applicable. Conclusion: The compounds of the present invention, such as the compounds in the examples, have good pharmacokinetic profiles in rats. | | | | | | |

### 6. Liver microsomal stability test

In this experiment, liver microsomes of five species, including human, monkey, dog, rat and mouse, were used as *in vitro* models to evaluate the metabolic stability of the test compound.

At 37°C, 1 µM of the test compound was co-incubated with microsomal protein and coenzyme NADPH. At given time points of the reaction (5 min, 10 min, 20 min, 30 min, and 60 min), the reaction was terminated by adding ice-cold acetonitrile containing an internal standard. The LC-MS/MS method was used to measure the concentration of the test compound in the sample. T_{1/2} was calculated using the ln value of the percentage of drug remaining in the incubation system and the incubation time. In addition, the intrinsic clearance in liver microsomes CL_{int(mic)} and the intrinsic clearance in liver CL_{int(Liver)} were further calculated.

**Table 6 Results of test compounds in monkey liver microsomal model**

| Test compound | Percentage of drug remaining after 60 min of incubation |
|---|---|
| Compound 3 | 56.1% |
| Compound 7 | 48.2% |

| | |
|---|---|
| Conclusion: The compounds of the present invention, such as the compounds of the examples, have good stability in the liver microsomes of monkeys. | |

### 7. Pharmacokinetic test in monkeys

**Experimental animals:** Male cynomolgus monkeys, 3-5 kg, 3-6 years old, 6 monkeys/compound. purchased from Suzhou Xishan Biotechnology Inc.

**Experimental method:** On the day of the experiment, the monkeys were randomly grouped according to their body weights. The animals were fasted with water available for 14-18 h one day before the administration, and were fed 4 h after the administration.

**Table 7. Administration information**

| Group | Number | Administration information | | | | | |
|---|---|---|---|---|---|---|---|
| | Male | Test compound | Administrati on dosage (mg/kg) | Administra tion concentrati on (mg/mL) | Administrati on volume (mL/kg) | Collected sample | Admini stration mode |
| G1 | 3 | Compound of the present invention or control compound | 1 | 1 | 1 | Plasma | Intraven ous adminis tration |
| G2 | 3 | | 3 | 0.6 | 5 | Plasma | Intragas tric adminis tration |

| | | | | | | | |
|---|---|---|---|---|---|---|---|
| Note: Vehicle for intravenous administration: 5% DMA + 5% Solutol + 90% Saline; and vehicle for intragastric administration: 0.5% MC; *Dosage is calculated based on a free base. | | | | | | | |

Before and after the administration, 1.0 mL of blood was taken from the limb veins and placed in an EDTAK2 centrifuge tube. The tube was centrifuged at 5000 rpm at 4°C for 10 min, and the plasma was collected. The blood sampling time points for both the intravenous administration group and intragastric administration group were: 0 min, 5 min, 15 min, 30 min, 1 h, 2 h, 4 h, 6 h, 8 h, 10 h, 12 h, and 24 h. Before analysis and detection, all samples were stored at -80°C, and the samples were quantitatively analysed by LC-MS/MS.

Conclusion: The compounds of the present invention, such as the compounds in the examples, have good pharmacokinetic profiles in cynomolgus monkeys.

## Claims

1. A compound represented by formula I, or a stereoisomer, a deuterated substance or a pharmaceutically acceptable salt thereof, 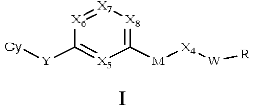
**characterised in that** Cy is selected from 8- to 15-membered fused heterocyclyl, which is optionally substituted with 1-5 groups selected from halogen, CN, OH, =O, NH₂, -SF₅, -COOH, C₁₋₄ alkyl, C₂₋₄ alkenyl, C₂₋₄ alkynyl, C₁₋₄ alkoxy, -NHC₁₋₄ alkyl, -N(C₁₋₄ alkyl)₂, halo C₁₋₄ alkoxy, halo C₁₋₄ alkyl, C₃₋₆ cycloalkyl, -C₁₋₄ alkyl-C₃₋₆ cycloalkyl, 4- to 12-membered heterocycloalkyl containing 1-3 heteroatoms selected from N, S, or O, 6- to 9-membered aryl, or 5- to 9-membered heteroaryl containing 1-3 heteroatoms selected from N, S, or O, wherein the alkyl, alkenyl, alkynyl, alkoxy, cycloalkyl, heterocyclyl, aryl and heteroaryl are optionally further substituted with 1-3 groups selected from halogen, =O, 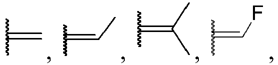 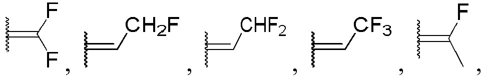 C₁₋₄ alkyl, C₁₋₄ alkoxy, halo C₁₋₄ alkoxy, or halo C₁₋₄ alkyl;
Y is C₁₋₂ alkyl, O, C=O, or NR_{y};
R_{y} is H or C₁₋₄ alkyl;
M is C₁₋₂ alkyl, O, or NRₘ;
W is a bond, O, or NR_{w};
Rₘ and R_{w} are independently H or C₁₋₄ alkyl;
X₄ is selected from C(O), S(O), or S(O)₂;
X₅ is selected from N or CRₓ₅;
X₆ is selected from N or CRₓ₆;
X₇ is selected from N or CRₓ₇;
X₈ is selected from N or CRₓ₈;
Rₓ₅, Rₓ₆, Rₓ₇, and Rₓ₈ are each independently selected from H, halogen, CN, C₁₋₄ alkyl, halo C₁₋₄ alkyl, C₂₋₄ alkenyl, C₂₋₄ alkynyl, C₁₋₄ alkoxy, halo C₁₋₄ alkoxy, - NHC₁₋₄ alkyl, -N(C₁₋₄ alkyl)₂, C₃₋₆ cycloalkyl, a 5- to 10-membered heterocyclic ring containing 1-3 heteroatoms selected from N, S, or O, and 6- to 9-membered aryl, wherein the alkyl, alkenyl, alkynyl, alkoxy, cycloalkyl, heterocyclic ring, or aryl is optionally substituted with 1-3 groups selected from halogen, C₁₋₄ alkyl, halo C₁₋₄ alkyl, C₁₋₄ alkoxy, halo C₁₋₄ alkoxy, OH, NH₂, and CN;
R is selected from C₃₋₁₀ cycloalkyl or 4- to 10-membered heterocyclyl containing 1-3 heteroatoms selected from N, S, or O, wherein the cycloalkyl and heterocyclyl are optionally substituted with 1-3 groups selected from halogen, C₁₋₄ alkyl, C₁₋₄ alkoxy, halo C₁₋₄ alkoxy, OH, NH₂, 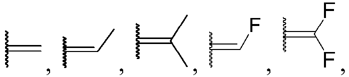 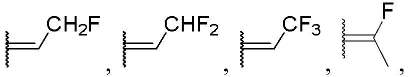 -NHC₁₋₄ alkyl, -N(C₁₋₄ alkyl)₂, and halo C₁₋₄ alkyl.

2. The compound, or the stereoisomer, the deuterated substance, or the pharmaceutically acceptable salt thereof according to claim 1, **characterised in that** Cy is selected from P1, P2, P3, or P4; 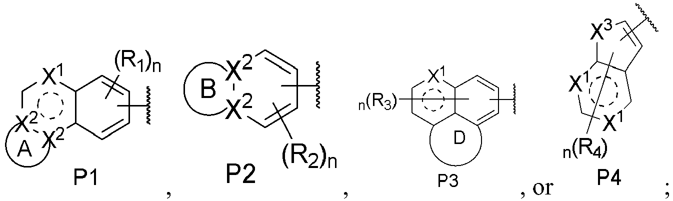
ring A is 5- to 6-membered heterocycloalkyl or 5- to 6-membered heteroaryl containing 1-3 heteroatoms selected from N, S, or O, wherein the heterocycloalkyl or heteroaryl is optionally substituted with 1-3 groups selected from halogen, C₁₋₄ alkoxy, -NHC₁₋₄ alkyl, -N(C₁₋₄ alkyl)₂, CN, C₁₋₄ alkyl, halo C₁₋₄ alkoxy, and halo C₁₋₄ alkyl;
ring B is 5- to 6-membered heteroaryl containing 1-3 heteroatoms selected from N, S, or O, wherein the heteroaryl is optionally substituted with 1-3 groups selected from =O, halogen, C₁₋₄ alkyl, halo C₁₋₄ alkyl, C₁₋₄ alkoxy, OH, halo C₁₋₄ alkoxy, C₃₋₆ cycloalkyl, 5- to 6-membered heterocyclyl containing 1-3 heteroatoms selected from N, S, or O, or -C₁₋₄ alkyl-C₃₋₆ cycloalkyl, wherein the heterocyclyl and cycloalkyl are optionally further substituted with groups 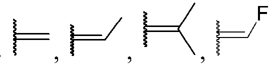 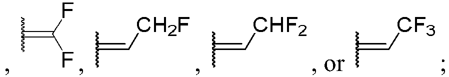
ring D is 5- to 6-membered heterocyclyl containing 1-3 heteroatoms selected from N, S, or O, wherein the heterocyclyl is optionally substituted with 1-3 groups selected from halogen, C₁₋₄ alkoxy, -NHC₁₋₄ alkyl, -N(C₁₋₄ alkyl)₂, CN, C₁₋₄ alkyl, halo C₁₋₄ alkoxy, and halo C₁₋₄ alkyl;
each n is independently selected from 0, 1, 2, 3, 4, or 5;
each X₁ is independently selected from N, NR₁₁, CR₁₁, or CR₁₁R₁₂;
each X₂ is independently selected from N, C, or CR₂₁;
X₃ is selected from NR₃₁, O, or CR₃₁R₃₂;
R₁, R₂, R₃, and R₄ are each independently selected from H, halogen, OH, =O, - NHC₁₋₄ alkyl, -N(C₁₋₄ alkyl)₂, CN, C₁₋₄ alkyl, C₁₋₄ alkoxy, halo C₁₋₄ alkoxy, halo C₁₋₄ alkyl, C₂₋₄ alkenyl, C₂₋₄ alkynyl, 5- to 8-membered heterocycloalkyl containing 1-3 heteroatoms selected from N, S, or O, or 6- to 9-membered aryl, wherein the alkyl, heterocycloalkyl, and aryl are optionally substituted with 1-3 groups selected from halogen, OH, NH₂, CN, and C₁₋₄ alkyl;
R₁₁, R₁₂, R₂₁, R₃₁, and R₃₂ are each independently selected from H, halogen, CN, C₁₋₄ alkyl, halo C₁₋₄ alkyl, C₂₋₄ alkenyl, C₂₋₄ alkynyl, C₁₋₄ alkoxy, halo C₁₋₄ alkoxy, -NHC₁₋₄ alkyl, -N(C₁₋₄ alkyl)₂, C₃₋₆ cycloalkyl, a 5- to 10-membered heterocyclic ring containing 1-3 heteroatoms selected from N, S, or O, and 6- to 9-membered aryl, wherein the alkyl, alkenyl, alkynyl, alkoxy, cycloalkyl, heterocyclic ring, or aryl is optionally substituted with 1-3 groups selected from halogen, C₁₋₄ alkyl, halo C₁₋₄ alkyl, C₁₋₄ alkoxy, halo C₁₋₄ alkoxy, OH, NH₂, and CN;
alternatively, R₁₁ and R₃₁, together with the atom(s) to which they are attached, form a C₃₋₆ carbocyclic ring, or a 5- to 6-membered heterocyclic ring containing 1-3 heteroatoms selected from N, S, or O, wherein the carbocyclic ring or heterocyclic ring is optionally substituted with 1-3 groups selected from halogen, C₁₋₄ alkyl, halo C₁₋₄ alkyl, C₁₋₄ alkoxy, OH, NH₂, and CN;
R is selected from C₅₋₁₀ bicyclic cycloalkyl, C₃₋₆ monocyclic cycloalkyl, 5- to 10-membered bicyclic heterocycloalkyl containing 1-3 heteroatoms selected from N, S, or O, or 4- to 6-membered monocyclic heterocycloalkyl containing 1-2 heteroatoms selected from N, S, or O, wherein the bicyclic cycloalkyl and bicyclic heterocycloalkyl are optionally substituted with 1-3 groups selected from halogen, C₁₋₄ alkyl, C₁₋₄ alkoxy, halo C₁₋₄ alkoxy, OH, NH₂, -NHC₁₋₄ alkyl, -N(C₁₋₄ alkyl)₂, and halo C₁₋₄ alkyl, and the monocyclic cycloalkyl and monocyclic heterocycloalkyl
are optionally further substituted with 1-3 groups selected from halogen, =O, 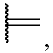 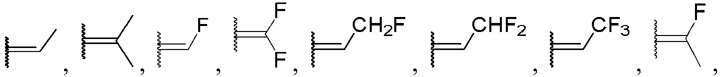 C₁₋₄ alkyl, C₁₋₄ alkoxy, halo C₁₋₄ alkoxy, or halo C₁₋₄ alkyl,
provided that the monocyclic cycloalkyl and monocyclic heterocycloalkyl are substituted with at least one group selected from

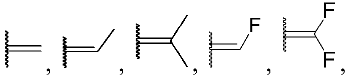

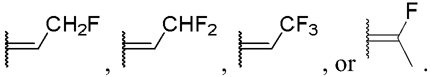

3. The compound, or the stereoisomer, the deuterated substance, or the pharmaceutically acceptable salt thereof according to claim 1 or 2,
**characterised in that** ring A is 5-membered heterocycloalkyl or 5-membered heteroaryl containing 1-3 heteroatoms selected from N, S, or O, wherein the heterocycloalkyl or heteroaryl is optionally substituted with 1-2 groups selected from halogen, C₁₋₄ alkoxy, -NHC₁₋₄ alkyl, -N(C₁₋₄ alkyl)₂, CN, C₁₋₄ alkyl, halo C₁₋₄ alkoxy, and halo C₁₋₄ alkyl;
ring B is 6-membered heteroaryl containing 1-3 heteroatoms selected from N, S, or O, wherein the heteroaryl is optionally substituted with 1-2 groups selected from =O, halogen, C₁₋₄ alkyl, C₁₋₄ alkoxy, halo C₁₋₄ alkyl, C₃₋₆ cycloalkyl, -C₁₋₂ alkyl-C₃₋₆ cycloalkyl, or 5- to 6-membered heterocycloalkyl containing 1-3 heteroatoms selected from N, S, or O, wherein the heterocyclyl and cycloalkyl are optionally further substituted with groups 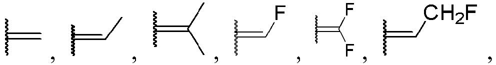 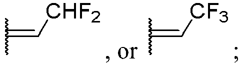
ring D is 5- to 6-membered heterocyclyl containing 1-3 heteroatoms selected from N, S, or O, wherein the heterocyclyl is optionally substituted with 1-2 groups selected from halogen, CN, C₁₋₄ alkyl, and halo C₁₋₄ alkyl;
R₁, R₂, R₃, and R₄ are each independently selected from H, halogen, =O, CN, C₁₋₄ alkyl, C₁₋₄ alkoxy, halo C₁₋₄ alkoxy, halo C₁₋₄ alkyl, 5- to 8-membered heterocycloalkyl containing 1-3 heteroatoms selected from N, S, or O, or 6- to 9-membered aryl, wherein the alkyl, heterocycloalkyl, and aryl are optionally substituted with 1-3 groups selected from halogen, CN, and C₁₋₄ alkyl.

4. The compound, or the stereoisomer, the deuterated substance, or the pharmaceutically acceptable salt thereof according to claim 1 or 2,
**characterised in that** each n is independently selected from 0, 1, 2, or 3;
each X₁ is independently selected from N or CR₁₁;
each X₂ is independently selected from N, C, or CR₂₁;
X₃ is selected from NR₃₁ or O;
X₄ is S(O)₂;
X₅ is CRₓ₅;
X₆ is CRₓ₆;
X₇ is CRₓ₇;
X₈ is CRₓ₈;
R₁₁, R₂₁, R₃₁, Rₓ₅, Rₓ₆, Rₓ₇, and Rₓ₈ are each independently selected from H, halogen, CN, C₁₋₄ alkyl, halo C₁₋₄ alkyl, C₁₋₄ alkoxy, halo C₁₋₄ alkoxy, a 5- to 6-membered heterocyclic ring containing 1-3 heteroatoms selected from N, S, or O, and 6-membered aryl, wherein the alkyl, alkoxy, heterocyclic ring, or aryl is optionally substituted with 1-3 groups selected from halogen, C₁₋₄ alkyl, or halo C₁₋₄ alkyl.

5. The compound, or the stereoisomer, the deuterated substance, or the pharmaceutically acceptable salt thereof according to claim 1,
**characterised in that** Y is O, C=O, or NH;
M is NRₘ;
W is a bond;
Rₘ is H;
R is selected from 6- to 10-membered spirocyclic heterocycloalkyl containing 1-3 heteroatoms selected from N, S, or O, 4- to 5-membered monocyclic heterocycloalkyl containing 1-2 heteroatoms selected from N, S, or O, C₆₋₁₀ spirocycloalkyl, or C₄₋₅ monocyclic cycloalkyl, wherein the spirocyclic heterocycloalkyl and spirocycloalkyl are optionally substituted with 1-3 groups selected from halogen, C₁₋₄ alkyl, C₁₋₄ alkoxy, halo C₁₋₄ alkoxy, OH, NH₂, -NHC₁₋₄ alkyl, -N(C₁₋₄ alkyl)₂, and halo C₁₋₄ alkyl, and the monocyclic heterocycloalkyl and monocyclic cycloalkyl are optionally further substituted with 1-3 groups selected from F, Cl, Br, =O, 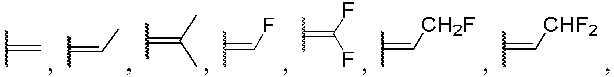 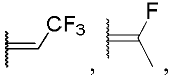 -CH₃, -CH₂CH₃, -CH₂F, -CHF₂, -CF₃, -CH₂CH₂F, -CH₂CHF₂, or - CH₂CF₃,
provided that the monocyclic heterocycloalkyl and monocyclic cycloalkyl are substituted with at least one group selected from

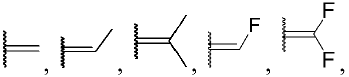

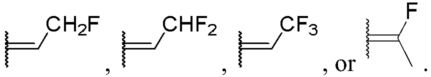

6. The compound, or the stereoisomer, the deuterated substance, or the pharmaceutically acceptable salt thereof according to claim 1,
**characterised in that** Y is O or C=O;
M is NRₘ;
W is NR_{w};
Rₘ is H;
R_{w} is H or CH₃;
R is selected from C₆₋₁₀ spirocycloalkyl or C₄₋₅ monocyclic cycloalkyl, wherein the spirocycloalkyl is optionally substituted with 1-3 groups selected from halogen, C₁₋₄ alkyl, C₁₋₄ alkoxy, halo C₁₋₄ alkoxy, OH, NH₂, -NHC₁₋₄ alkyl, -N(C₁₋₄ alkyl)₂, and halo C₁₋₄ alkyl, and the monocyclic cycloalkyl is optionally further substituted with 1-3 groups selected from F, Cl, Br, =O, 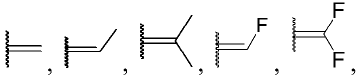 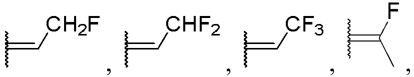 -CH₃, -CH₂CH₃, -CH₂F, -CHF₂, -CF₃, - CH₂CH₂F, -CH₂CHF₂, or -CH₂CF₃,
provided that the monocyclic cycloalkyl is substituted with at least one group selected from 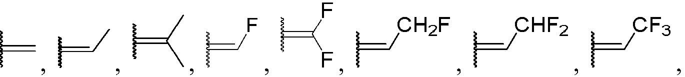 or

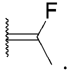

7. The compound, or the stereoisomer, the deuterated substance, or the pharmaceutically acceptable salt thereof according to claim 1,
**characterised in that** R is selected from 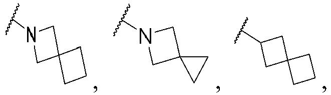 or 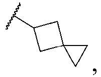 which are optionally substituted with 1-3 groups selected from F, Cl, Br, -CH₃, -CH₂CH₃, -CH₂F, -CHF₂, -CF₃, -CH₂CH₂F, -CH₂CHF₂, or -CH₂CF₃;
alternatively, R is selected from 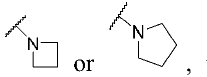 which are optionally substituted with 1-2 groups selected from F, Cl, Br, 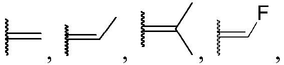 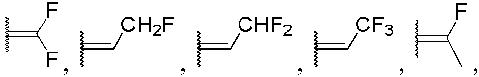 -CH₃, -CH₂CH₃, -CH₂F, -CHF₂, - CF₃, -CH₂CH₂F, -CH₂CHF₂, or -CH₂CF₃, and is substituted with at least one group selected from 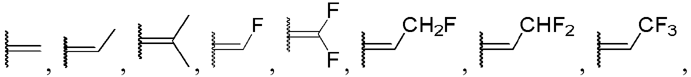 or 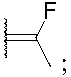
alternatively, R is selected from 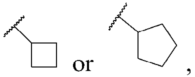 which are optionally substituted with 1-2 groups selected from F, Cl, Br, 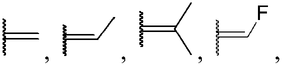 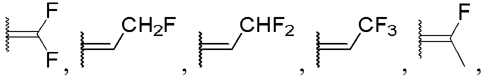 -CH₃, -CH₂CH₃, -CH₂F, -CHF₂, - CF₃, -CH₂CH₂F, -CH₂CHF₂, or -CH₂CF₃, and is substituted with at least one group selected from 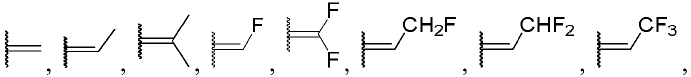 or

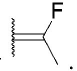

8. The compound, or the stereoisomer, the deuterated substance, or the pharmaceutically acceptable salt thereof according to claim 2, **characterised in that**
P1 is selected from 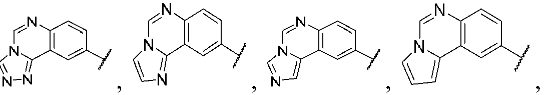 or 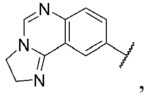
which are optionally substituted with 1-3 groups selected from F, Cl, Br, -CH₃, -CH₂CH₃, -CH₂F, -CHF₂, -CF₃, -CH₂CH₂F, -CH₂CHF₂, or -CH₂CF₃;
P2 is selected from 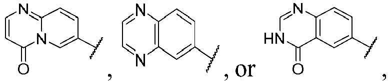
which are optionally substituted with 1-3 groups selected from F, Cl, Br, -CH₃, -CH₂CH₃, -CH₂F, -CHF₂, -CF₃, -CH₂CH₂F, -CH₂CHF₂, -CH₂CF₃, -OCH₃, cyclopropyl, 6-membered heterocycloalkyl containing 1-3 heteroatoms selected from N, S, or O, 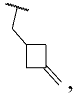 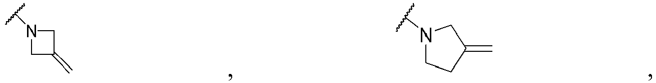 or 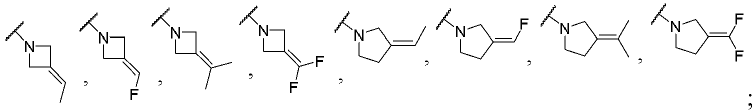
P3 is selected from 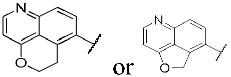 , which are optionally substituted with 1-3 groups selected from F, Cl, Br, =O, -CH₃, -CH₂CH₃, -CH₂F, -CHF₂, -CF₃, -CH₂CH₂F, -CH₂CHF₂, or -CH₂CF₃;
P4 is selected from 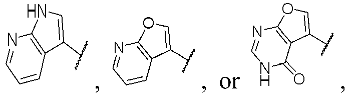 which are optionally substituted with 1-3 groups selected from F, Cl, Br, =O, -CH₃, -CH₂CH₃, -CH₂F, - CHF₂, -CF₃, -CH₂CH₂F, -CH₂CHF₂, -CH₂CF₃, or

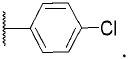

9. The compound, or the stereoisomer, the deuterated substance, or the pharmaceutically acceptable salt thereof according to claim 1, **characterised in that**
Y is O or NH;
M is NH;
W is a bond;
X₄ is S(O)₂;
X₅ is CRₓ₅;
X₆ is CRₓ₆;
X₇ is CRₓ₇;
X₈ is CRₓ₈;
Rₓ₅, Rₓ₆, Rₓ₇, and Rₓ₈ are each independently selected from H, F, Cl, CN, -CH₃, -CH₂CH₃, -CH₂F, -CHF₂, -CF₃, -CH₂CH₂F, -CH₂CHF₂, or -CH₂CF₃;
Cy is selected from 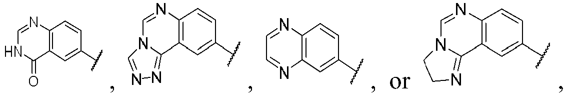 which are optionally substituted with 1-3 groups selected from F, Cl, -CH₃, - CH₂CH₃, -CH₂F, -CHF₂, -CF₃, -CH₂CH₂F, -CH₂CHF₂, -CH₂CF₃, -OCH₃, cyclopropyl, 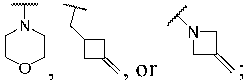
R is selected from 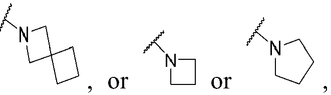 which are optionally substituted with 1-2 groups selected from F, Cl, Br, 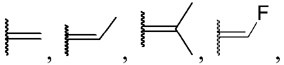 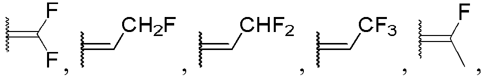 -CH₃, -CH₂CH₃, -CH₂F, -CHF₂, - CF₃, -CH₂CH₂F, -CH₂CHF₂, or -CH₂CF₃, and 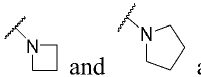 are substituted with at least one group selected from

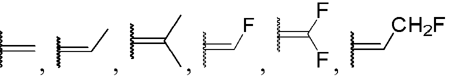

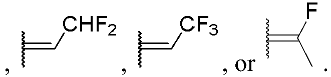

10. The compound, or the stereoisomer, the deuterated substance, or the pharmaceutically acceptable salt thereof according to claim 1, **characterised in that** the compound is selected from one of the structures in Table 1.

11. A pharmaceutical composition or pharmaceutical preparation, **characterised in that** the pharmaceutical composition or pharmaceutical preparation comprises the compound, or the stereoisomer, the deuterated substance, or the pharmaceutically acceptable salt thereof according to any one of claims 1-10, and a pharmaceutically acceptable carrier and/or auxiliary material.

12. The pharmaceutical composition or pharmaceutical preparation according to claim 11, **characterised in that** the pharmaceutical composition or pharmaceutical preparation comprises 1-1500 mg of the compound, or the stereoisomer, the deuterated substance, or the pharmaceutically acceptable salt thereof according to any one of claims 1-10, and a pharmaceutically acceptable carrier and/or auxiliary material.

13. Use of the compound, or the stereoisomer, the deuterated substance, or the pharmaceutically acceptable salt thereof according to any one of claims 1-10, or the pharmaceutical composition or pharmaceutical preparation according to claim 11 in the preparation of a medicament for the treatment/prevention of a BRAFmediated disease.

14. A method for treating a disease in a mammal, **characterized by** comprising administering to a subject a therapeutically effective amount of the compound, or the stereoisomer, the deuterated substance, or the pharmaceutically acceptable salt thereof according to any one of claims 1-10, wherein the therapeutically effective amount is preferably 1-1500 mg, and the disease is preferably a tumour, more preferably brain tumour, melanoma, colorectal cancer, non-small cell lung cancer, glioma, papillary thyroid carcinoma, or skin cancer.
